(19) Europäisches Patentamt / European Patent Office / Office européen des brevets

(11) **EP 4 706 675 A1**

(12) **EUROPEAN PATENT APPLICATION**

(43) Date of publication:
**11.03.2026 Bulletin 2026/11**

(21) Application number: **24199448.2**

(22) Date of filing: **10.09.2024**

(51) International Patent Classification (IPC):
**A61K 40/11** (2025.01)   **A61K 40/31** (2025.01)
**A61K 40/42** (2025.01)   **C07K 16/28** (2006.01)
**C07K 16/30** (2006.01)

(52) Cooperative Patent Classification (CPC):
**A61K 40/11; A61K 40/31; A61K 40/42;**
**A61K 40/4211; A61K 40/4224; A61K 40/4258;**
**C07K 16/2803; C07K 16/3084;** A61K 2239/13;
A61K 2239/48; A61K 2239/49; C07K 2317/622

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Max-Planck-Gesellschaft zur**
**Förderung der**
**Wissenschaften e.V.**
**80539 München (DE)**

(72) Inventors:
• **Moscovitz, Oren**
  **10585 Berlin (DE)**

• **Seeberger, Peter H.**
  **14532 Kleinmachnow (DE)**
• **Lühle, Jost**
  **14482 Potsdam (DE)**
• **Goerdeler, Felix**
  **10715 Berlin (DE)**

(74) Representative: **Wichmann, Hendrik**
**Wuesthoff & Wuesthoff**
**Patentanwälte und Rechtsanwalt PartG mbB**
**Schweigerstraße 2**
**81541 München (DE)**

Remarks:
The complete document including Reference
Table(s) and the Sequence Listing(s) can be
downloaded from the EPO website

(54) **CYSTEINE-ENGINEERED CHIMERIC ANTIGEN RECEPTOR**

(57)    The invention pertains to a chimeric antigen receptor (CAR) that includes an antigen-binding domain. The antigen-binding domain comprises a heavy chain variable region and optionally a light chain variable region. The CAR is capable of binding to an antigen and comprises three complementarity-determining regions in the heavy chain variable region and optionally the light chain variable region. At least one of these regions contains an amino acid residue, other than cysteine, with a solvent-accessible surface area ranging from 0.00 to 0.75 and is located at a distance of 3 to 14 A from the antigen and is substituted to a thiol-containing amino acid. This unique configuration enhances the CAR's antigen-binding and cytotoxicity capabilities.

EP 4 706 675 A1

## Description

## Background of invention

Immunotherapy represents a key frontier in the development of novel therapeutics. Antibodies and antibody fragments play a central role as they allow specific targeting of cancer cells. The treatment of hematological cancers was revolutionized in the last decades by Chimeric Antigen Receptor (CAR-) T cells. In this method, the patient's own T lymphocytes are genetically engineered *ex vivo* with a CAR composed of an extracellular cancer-specific antibody fragment and intracellular signaling domains for triggering T cell activation and cytotoxicity.[1] These CAR-T cells are re-transfused and mediate their anticancer activity *in vivo.* To date, there are six different CAR-T cell therapies in clinical use against relapsed or refractory B cell lymphoma (BCL), acute lymphoblastic leukemia (ALL) and multiple myeloma (MM).[2-7] Except for MM therapies, all approved formats employ CD19, a transmembrane glycoprotein highly expressed by most B cell malignancies. Moreover, the expression of CD19 on healthy cells is restricted to B cells, minimizing on-target/off-tumor effects.[8]

Despite the remarkable achievements of CAR-T cell therapy for hematological cancers, the technology still faces several challenges. These include adverse effects like neurotoxicity or cytokine release syndrome, as well as emerging resistance of the cancer.[9-11] The latter mechanism, also referred to as antigen escape, is one of the main drawbacks of conventional CAR-T cell therapies. Because they target a specific tumor-associated antigen (TAA), selective pressure upon exposure to CAR-T cells can lead to downregulation or complete target loss.[12,13] For CD19-targeted CAR-T cells, antigen-negative relapse rates of up to 25% in ALL and BCL have been reported in the clinic.[14-17] Therefore, there is a high need for additional targets for CAR-T cell therapies. Current attempts focus on alternative targets such as CD20 and bispecific receptors or adapter CAR-T cells to respond dynamically to antigen escape.[18-20] However, these approaches still target specific TAAs, leaving the window open for cancer adaptation.

An alternative strategy for cancer immunotherapy is to co-target the cellular microenvironment, thereby expanding the target scope and minimizing the risk of evasion mechanisms.[21,22] A common feature of several cancer types, including BCL, is a reduced cellular microenvironment, caused by differential expression of disulfide-active enzymes, such as protein disulfide isomerase (PDI) or the thioredoxin system.[23-27] These alterations can contribute substantially to disease progression. For example, reduced thiol groups on breast cancer cells facilitate attachment to the endothelium, thereby promoting metastasis.[26]

Furthermore, overexpression of PDI is associated with cancer growth and drug resistance.[28,29] In addition to these effects to cancer biology, the reduced cellular microenvironment is also an attractive target for therapy. Small molecule PDI inhibitors have been reported to reduce metastasis in breast cancer and prolong the survival of mice transplanted with multiple myeloma.[26,30] On the other hand, increased levels of reduced surface thiols can be exploited for drug delivery, as they can be targeted directly by disulfide-active compounds or antibodies.[25,31]

In the inventor's previous work, a single-domain antibody (nanobody) derived from an alpaca heavy chain-only IgG was developed, which binds several subtypes of BCL.[25] This nanobody, denoted CB2, interacts with altered redox states of the cellular microenvironment via the non-canonical cysteine 105 in the complementarity-determining region (CDR) 3. While thiol-mediated uptake of CB2 enables its use as an intracellular drug carrier, a fraction of the nanobody remains bound to the cell surface.[25]

## Summary of the invention

The invention is defined in the claims. The invention provides a Chimeric Antigen Receptor (CAR) that binds specifically to target cells an additionally in a thiol-dependent manner, enabling effective treatment of cancers, also those undergoing antigen escape. The CAR comprises an antigen-binding domain, a hinge, a transmembrane domain, a co-stimulatory domain, and an activating domain, with at least one Complementarity-Determining Region (CDR) comprising at least one amino acid residue other than cysteine, which is substituted to a thiol group containing amino acid. This invention offers a solution to the limitations of current CARs, such as antigen escape, providing a more effective approach to cancer treatment.

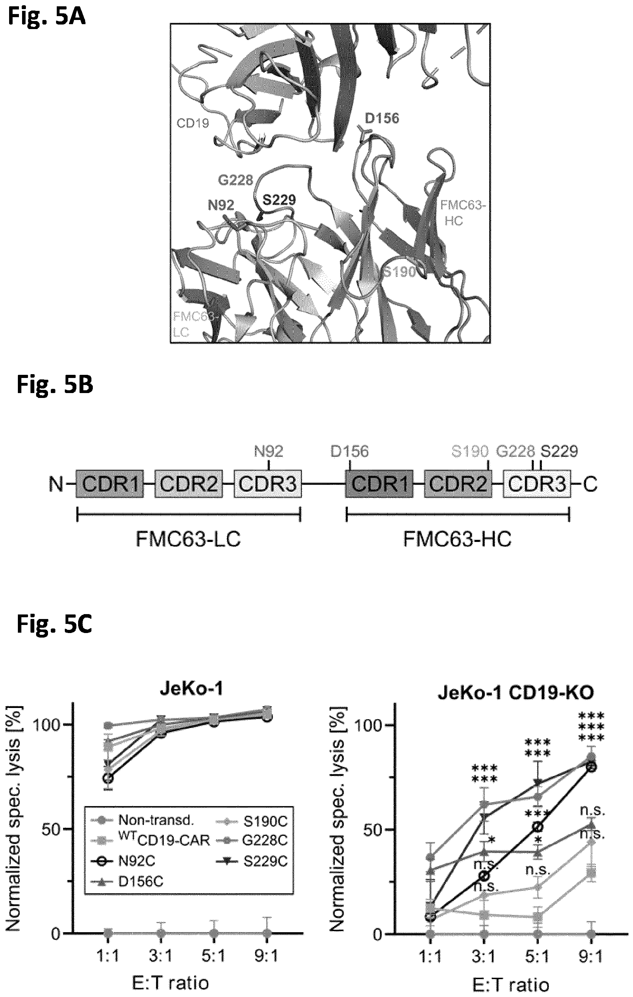

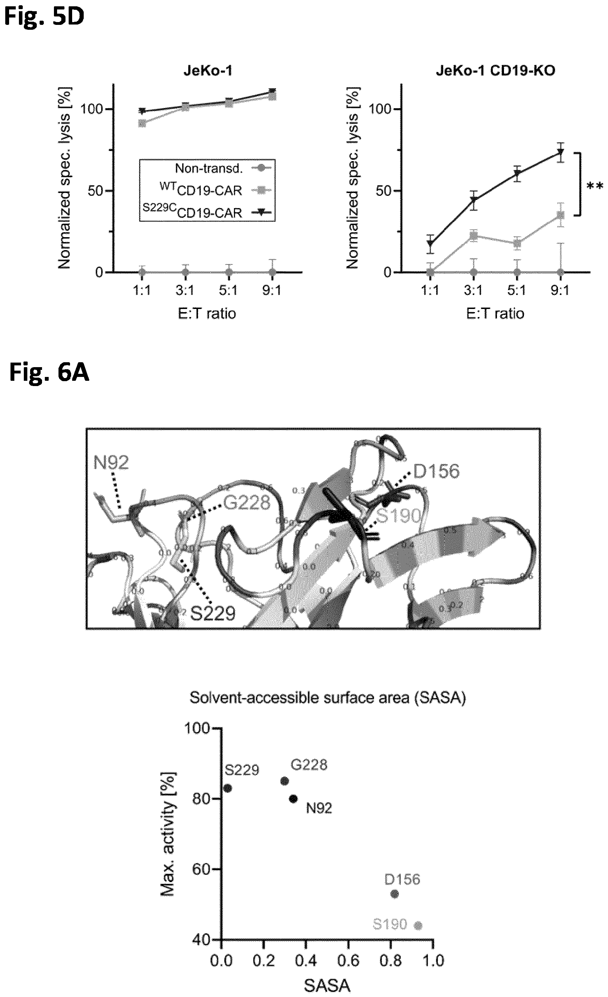

In particular, the development of a novel CAR-T cell therapy with cysteine-engineered antibody fragments is disclosed, thus directly targeting the cellular microenvironment instead of a single antigen. For example, the inventors show that CB2-CAR alone can trigger T cell activation and cytotoxicity against different subtypes of B-cell lymphoma (BCL), while healthy human Peripheral Blood Mononuclear Cells (PBMCs) are not targeted. Furthermore, the inventors show CB2-CAR-T cell activity against antigen escape models that are resistant to conventional CAR-T therapies. In addition, the inventors validate the results by cysteine engineering additional non-cancer-related nanobody-CAR, effectively initiating cancer targeting. Finally, the inventors demonstrate the method's ability to generate T-cells showing an antigen-dependent and a thiol-dependent binding by a single CAR construct on the clinically used CAR-T-cell therapy Kymriah®. Thus, by introducing one or more thiol group containing amino acids, preferably one, at positions on the anti-CD19 scFv FMC63, the inventors evaluated the CAR-T cell antigen-dependent and independent cytotoxicity and showed how cysteine-engineered FMC63-CAR-T cells demonstrate high efficacy against CD19-positive and negative lymphoma, both *in vitro* and in xenograft mouse models (Figures 5, 6 and 7). Additionally, the inventors demonstrate a high cytotoxic efficacy against CD-19 negative cancer cells (Figure 5C).

Hence, the present invention provides a CAR that not only binds specifically to the target but also in a thiol-dependent way. This is particularly advantageous as cancer cells often have an altered redox potential due to the increased expression of disulfide isomerase. The thiol-dependent targeting of the CAR makes it particularly suited for cancers undergoing antigen escape. The CARs according to the present invention efficiently target cancer cells. This targeting and the cytotoxicity of a T-cell comprising such a CAR has been experimentally shown e.g. for an anti-CD19 CAR. Mice treated with such CAR-T cells survived significantly longer and showed a significant cancer reduction. Hence, the invention is experimentally supported by in vivo data demonstrating optimal cytotoxic activity of a CAR as part of a CAR T-cell. Additionally, the inventors provide a clear rationale for the criteria to be met for thiol group containing amino acid substitution based on solvent-accessible surface area and distance to the target. Importantly, when the cysteine is substituted with a serine, the cytotoxic effect in the CAR-T cell is lost, highlighting the critical role of the thiol group containing amino acid in this invention. Example 3 herein provides experimental evidence that the replacement of the cysteine by serine can lead to a complete loss of the T cells cytotoxic activity.

The invention provides a CAR that comprises at least one amino acid residue other than cysteine, which has a solvent-accessible surface area from 0.00 to 0.75 and is located at a distance to the antigen from 3 to 14 Å. This amino acid residue is substituted to a thiol group containing amino acid, enabling the CAR to bind to the target in a thiol-dependent manner.

The invention offers several advantages over the current state of the art. For example, the thiol-dependent targeting of the CAR allows for the effective treatment of cancers undergoing antigen escape, a significant challenge in current cancer treatments. Furthermore, the treatment with these CARs also is generally considered to be safe, as demonstrated with, e.g., the cysteine-engineered anti-CD19 scFv FMC63 construct in mice, which showed no side effects for a period of more than 42 days.

**Description of the Figures**

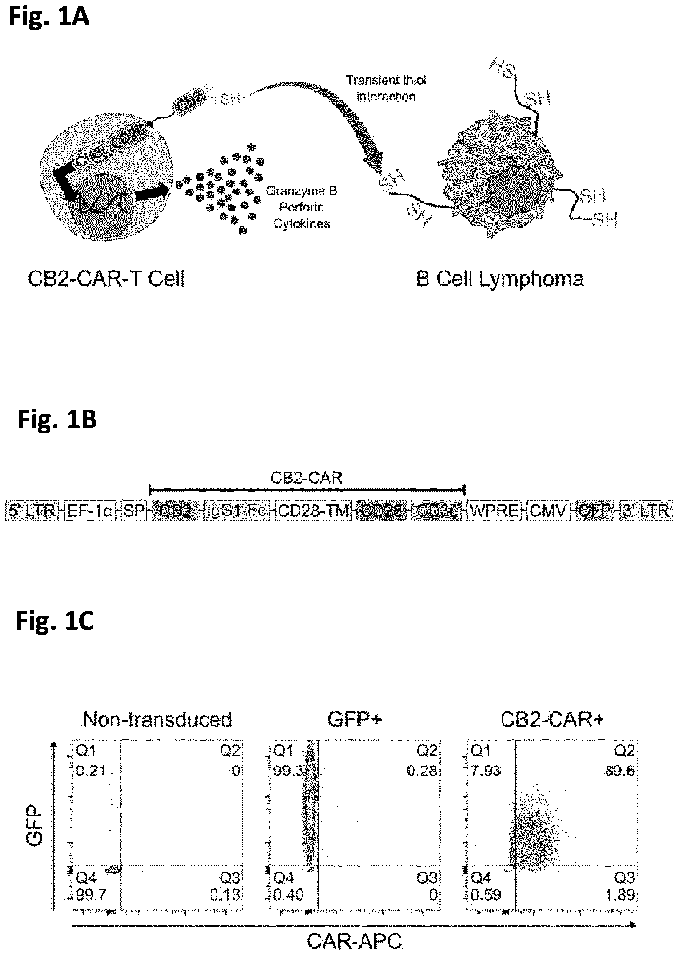

**Fig. 1A** Generation of CB2-CAR-T cells. Schematic representation of thiol-dependent CB2-CAR-T cell activity against BCL.

**Fig. 1B** Domain structure of CB2-CD28-CAR_GFP construct. Abbreviations: LTR- long terminal repeat, EF-1$\alpha$ - human elongation factor-1 alpha promotor, SP - signal peptide, IgG1-Fc - human IgG1-Fc hinge, CD28-TM - CD28 transmembrane domain, CD28 - CD28 costimulatory domain, CD3$\zeta$ - CD3$\zeta$ signaling domain, WPRE - Woodchuck Hepatitis Virus Posttranscriptional Regulatory Element, CMV - human cytomegalovirus enhancer and promotor, GFP - green fluorescent protein.

**Fig. 1C** Determination of transduction efficiencies of GFP control and CB2-CAR-T cells on day 6 after transduction. Representative plots are shown. Cells were stained with MonoRab™ Rabbit Anti-Camelid VHH Cocktail iFluor 647 and measured on a FACSCanto™ II Flow Cytometry System detecting antibody fluorescence (CAR-APC) and GFP fluorescence (GFP). Percentages of the individual populations are indicated in the graphs. (

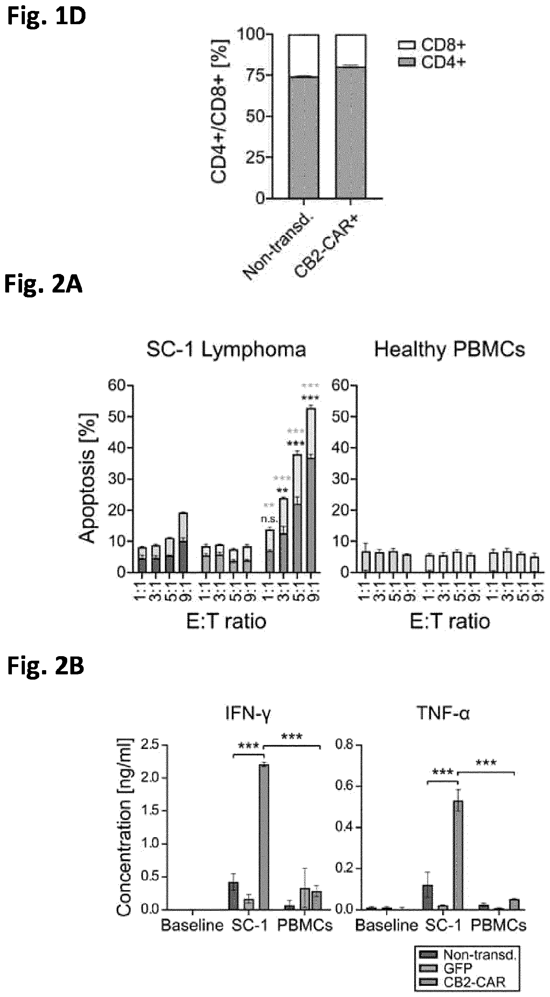

**Fig. 1D** Immunophenotyping of non-transduced and CB2-CART cells. Cells were stained with PE anti-human CD4 Antibody and FITC anti-human CD8 Antibody and measured on a FACSCanto™ II Flow Cytometry System. Grey bars represent percentage of CD4+ and white bars of CD8+ T cells. Mean values $\pm$ SEM of n=3 replicates are shown.

**Fig. 2A** CB2-CAR-T cells mediate cytotoxicity against B cell lymphoma and undergo T cell activation. Flow cytometry-based apoptosis assays. SC-1 lymphoma or healthy human PBMCs were incubated for 72 hours with CB2-CAR or control T cells at different E:T ratios. Apoptosis was assessed by incubating with Annexin V-PE (early apoptosis) and 7-AAD (late apoptosis) and measuring on a FACSCanto™ II Flow Cytometry System. Stacked bars represent the different types of apoptosis (darker bottom part of bars for late apoptosis and lighter top part for early apoptosis). Mean values $\pm$ SEM of n=3 replicates are shown. Significance was assessed via 2-way ANOVA followed by Tukey's post-hoc test. Bottom significance levels refer to differences in late apoptosis and top significance levels to differences in early apoptosis compared to both controls (in the case of different significance levels, the less significant is indicated).

**Fig. 2B** Cytokine secretion assays. Levels of IFN-$\gamma$ and TNF-o in co-culture supernatants after 72 hours were determined via ELISA. T cells were co-incubated with SC-1 cells or PBMCs respectively. Baseline samples represent T cells cultured without target cells under the same conditions. Mean values ± SEM of n=3 replicates are shown, and significance of differences was assessed via 2-way ANOVA followed by Tukey's post-hoc test.

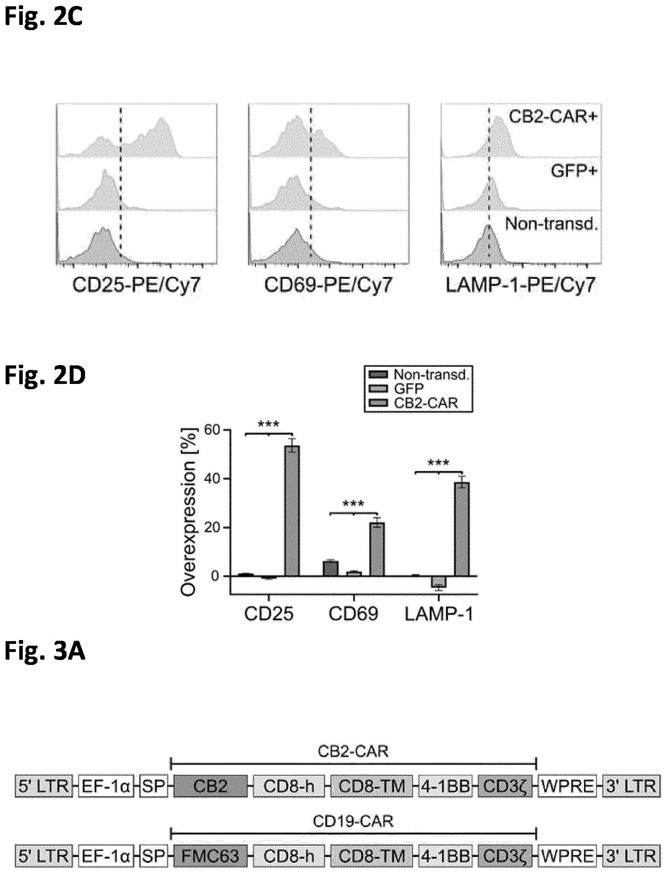

**Fig. 2C** Representative flow cytometry plots for the analysis of T cell activation markers. Different samples of T cells were co-cultured with SC-1 cells for 72 hours and stained with PE/Cyanine7-conjugated antibodies against the markers CD25, CD69, and LAMP-1 respectively and measured on a FACSCanto™ II Flow Cytometry System. Offset histograms of CB2-CAR-T cells and control T cells are shown for each marker. Dashed lines indicate thresholds for positive cells and were set according to T cells cultured without target cells.

**Fig. 2D** Quantification of T cell marker expression. Fractions of positive cells shown in C were quantified by subtracting the baseline expression of each marker (T cells only). Mean values ± SEM of n=3 replicates are shown, and significance of differences was assessed via 1-way ANOVA followed by Tukey's post-hoc test for each marker individually. Significance levels in all subfigures: n.s. - not significant; * - $p<0.05$; ** - $p<0.01$; *** - $p<0.001$.

**Fig. 3A** B cell lymphoma subtypes and antigen escape models are efficiently targeted by CB2-CAR-T cells. Domain structures of CB2-41BB-CAR and CD19-CAR. Abbreviations: LTR - long terminal repeat, EF-1$\alpha$ - human elongation factor-1 alpha promotor, SP - signal peptide, CD8-h - CD8 hinge, CD8-TM - CD8 transmembrane domain, 4-1BB - 4-1BB costimulatory domain, CD3$\zeta$ - CD3$\zeta$ signaling domain, WPRE - Woodchuck Hepatitis Virus Posttranscriptional Regulatory Element.

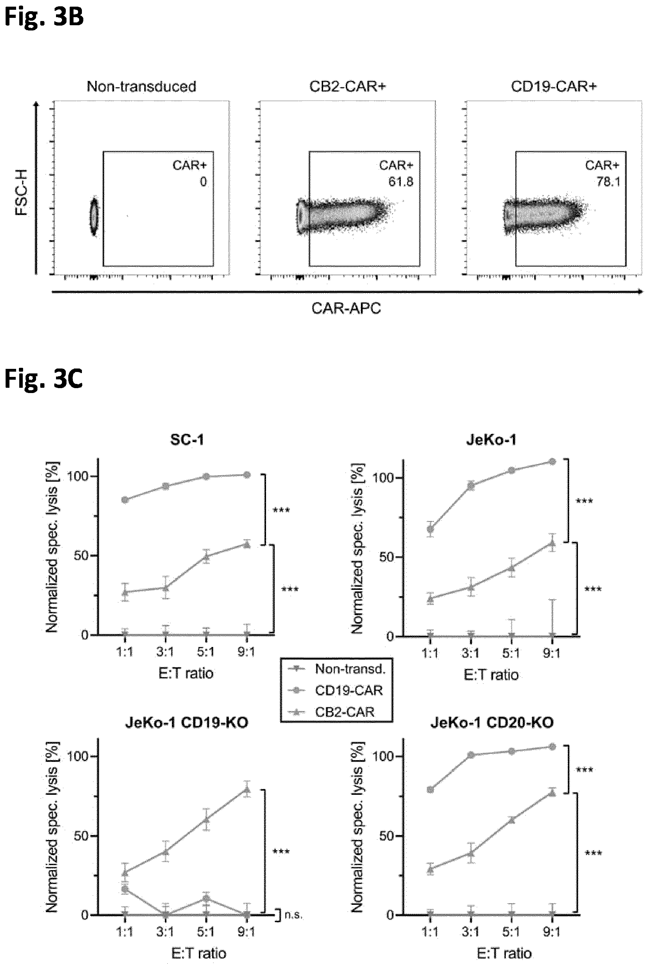

**Fig. 3B** Determination of transduction efficiencies of CB2-41BB-CAR and CD19-CAR on day 6 after transduction. Representative plots are shown. Cells were stained with MonoRab™ Rabbit Anti-Camelid VHH Cocktail iFluor 647 (CB2-41BB-CAR) or Recombinant Human CD19 Fc Chimera Alexa FluorO 647 (CD19-CAR) and measured on a FACSCanto™ II Flow Cytometry System detecting CAR-APC fluorescence. Percentages of CAR+ T cells are indicated in the graphs.

**Fig. 3C** Luciferase-based cytotoxicity assays of different lymphoma cell lines co-cultured with CAR-T cells at different E:T ratios for 24 hours (SC-1) or 48 hours (JeKo-1, JeKo-1 CD19-KO, and JeKo-1 CD20-KO). Cytotoxicity was assessed by adding D-luciferin potassium salt and reading out relative light units with a CLARIOstar Plus plate reader. Data points were normalized to non-transduced T cells at the respective E:T ratio. Grand mean values ± pooled SEM of N=3, n=3 replicates are shown. Tested constructs are non-transduced (▼), CB2-41BB-CAR (▲), and CD19-CAR (●). Significance of differences was assessed via 2-way ANOVA followed by Tukey's post-hoc test (n.s. - not significant; * - $p<0.05$; ** - $p<0.01$; *** - $p<0.001$).

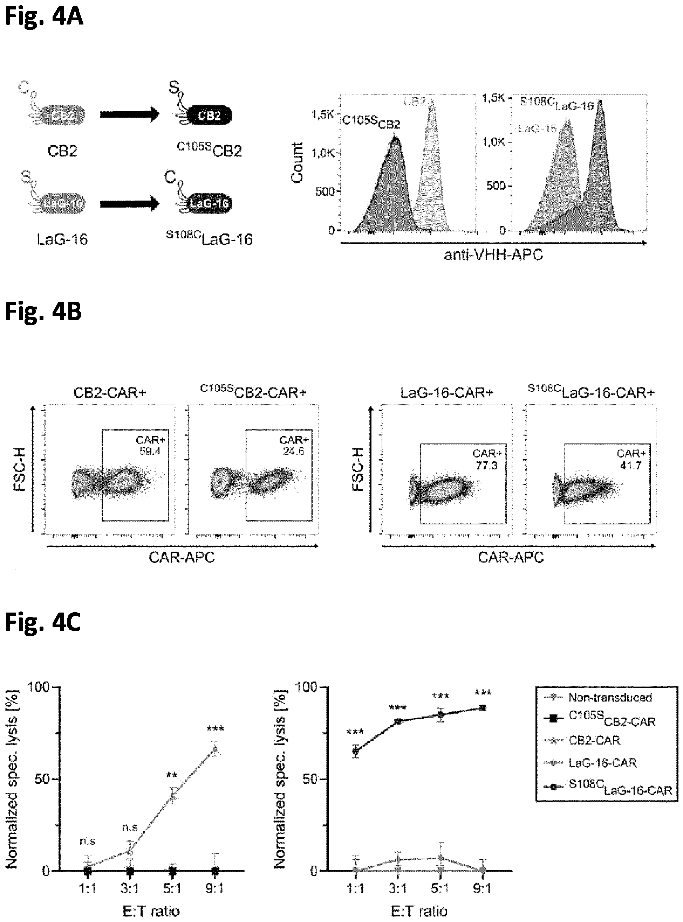

**Fig. 4A** Cysteine-engineered nanobodies redirect CAR-T cells to target B cell lymphoma. Illustration of investigated nanobodies and binding assays to SC-1 lymphoma. SC-1 cells were incubated with recombinant nanobodies and MonoRab™ Rabbit Anti-Camelid VHH Cocktail iFluor 647 and measured on a FACSCanto™ II Flow Cytometry System, detecting the anti-VHH-APC signal. Histograms for each nanobody and its mutant were overlayed to visualize differences in binding (grey histograms in the background represent secondary antibody-only controls). Representative plots of n=3 experiments are shown.

**Fig. 4B** Determination of transduction efficiencies of CB2-CAR- and LaG-16-CAR-T cells on day 6 after transduction. Representative plots are shown. Cells were stained with MonoRab™ Rabbit Anti-Camelid VHH Cocktail iFluor 647 and measured on a FACSCanto™ II Flow Cytometry System detecting CAR-APC fluorescence. Percentages of CAR+ T cells are indicated in the graphs.

**Fig. 4C** Luciferase-based cytotoxicity assays of SC-1 cells co-cultured with CAR-T cells at different E:T ratios for 24 hours. Cytotoxicity was assessed by adding D-luciferin potassium salt and reading out relative light units with a CLARIOstar Plus plate reader. Data points were normalized to non-transduced T cells at the respective E:T ratio. Grand mean values ± pooled SEM of N=3, n=3 replicates are shown. Tested constructs are non-transduced (▼), $^{C105S}$CB2-CAR (■), CB2-CAR (▲), LaG-16-CAR (♦), and $^{S108C}$Lag-16-CAR (⬤). Significance of differences was assessed via 2-way ANOVA followed by Tukey's post-hoc test (n.s. - not significant; * - $p<0.05$; ** - $p<0.01$; *** - $p<0.001$).

**Fig. 5A** Evaluation of Cytotoxic Activity of CD19-CAR Cysteine Mutants. Illustration of the mutated residues N92, D156, S190, G228, and S229 in the three-dimensional structure of FMC63 in complex with CD19 (PDB entry ID: 7URV).

**Fig. 5B** Schematic representation of the individual mutated residues in the linear sequence of FMC63 (LC - light chain, HC - heavy chain).

**Fig. 5C** Luciferase-based cytotoxicity assays of JeKo-1 lymphoma and JeKo-1 CD19-knockout co-cultured with CD19-CAR-T cell mutants at different E:T ratios for 48 hours. Cytotoxicity was assessed by adding D-luciferin potassium salt and reading out relative light units with a CLARIOstar Plus plate reader. Data points were normalized to non-transduced T cells at the respective E:T ratio. Mean values $\pm$ SEM of n=3 replicates are shown.

**Fig. 5D** Luciferase-based cytotoxicity assays of JeKo-1 lymphoma and JeKo-1 CD19-knockout co-cultured with $^{WT}$CD19-CAR- and $^{S229C}$CD19-CAR-T cells from three independent blood donors at different E:T ratios for 48 hours. Grand mean values $\pm$ pooled SEM of N=3, n=3 replicates are shown. Tested constructs are non-transduced (•), $^{WT}$CD19-CAR (■), $^{N92C}$CD19-CAR (○), $^{D156C}$CD19-CAR (▲), $^{S190C}$CD19-CAR (♦), $^{G228C}$CD19-CAR (⬤ ), and $^{S229C}$CD19-CAR (▼). Significance of differences was assessed via 2-way ANOVA followed by Tukey's post-hoc test (n.s. - not significant; * - p<0.05; ** - p<0.01; *** - p<0.001). Significance levels in comparison with $^{WT}$CD19-CAR are shown.

**Fig. 6A** Structural analysis of anti-CD19 scFv FMC63 to determine optimal solvent-accessible surface area (SASA). Structure was retrieved from the protein database PDB (Entry ID 7URV) and edited using PyMOL. SASA values were computed in PyMOL via the command "PyMOL>get_sasa_relative polymer". Coloring of the protein structure refers to SASA values (blue - high, grey - low). SASA of the candidate amino acids were plotted against maximum activity (effector-to-target ratio of 9:1) of respective T cells expressing the mutated scFvs in context of a CAR.

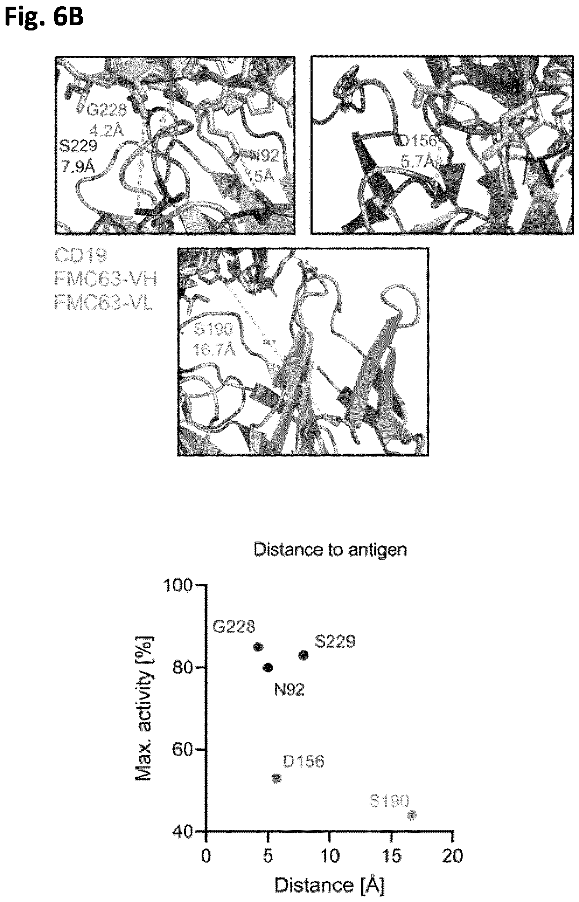

**Fig. 6B** Structural analysis of anti-CD19 scFv FMC63 to determine optimal distance to target antigen. (B) Distance values were computed in PyMOL using the measurement wizard tool. The distance of the respective side chain of the candidate amino acids with the closest atom of CD19 are shown and plotted against maximum activity (effector-to-target ratio of 9:1) of respective T cells expressing the mutated scFvs in context of a CAR.

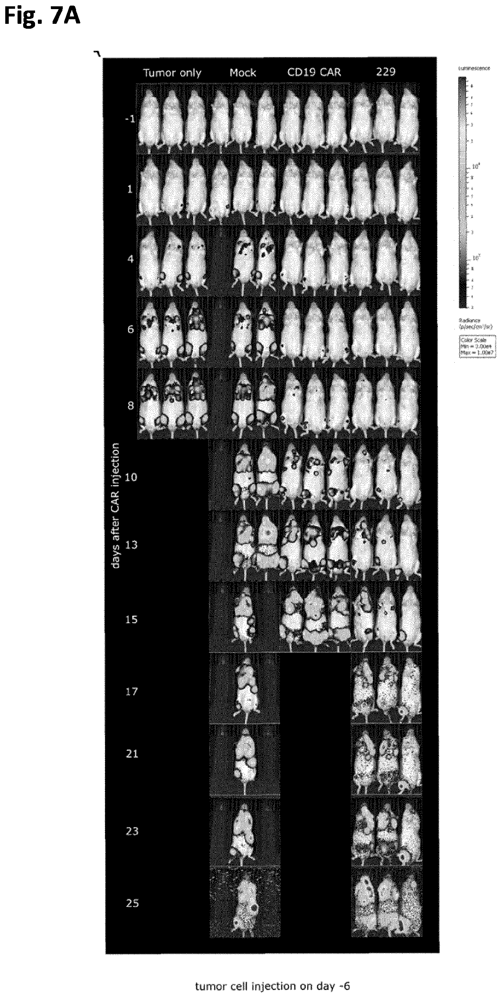

**Fig. 7A** *In vivo* assay with cysteine-engineered CD19-CAR-T cells. Mice were xenografted on day -6 with human B cell lymphoma cell lines JeKo-1 and JeKo-1 CD190-knockout at 1:1 ratio ($0.5 \times 10^6$ cells each; n=3 mice per group). At time points indicated, tumor development was measured via luciferase signal.

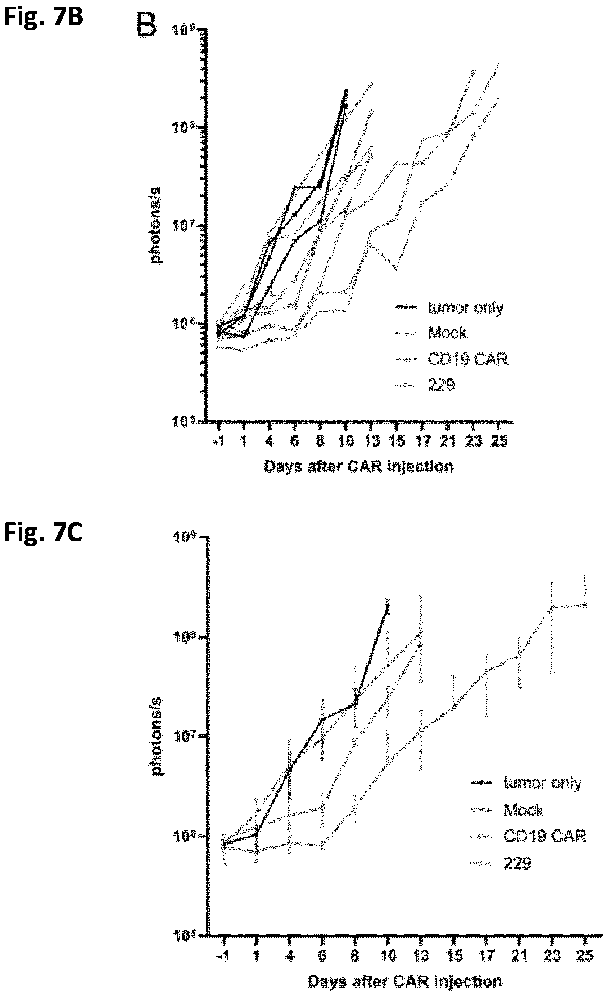

**Fig. 7 B-C** Intensities in photons per second were quantified and plotted as individual curves per mouse (top panel) and average (lower panel). One mouse of the mock group that showed unexpectedly long survival was excluded from the quantification.

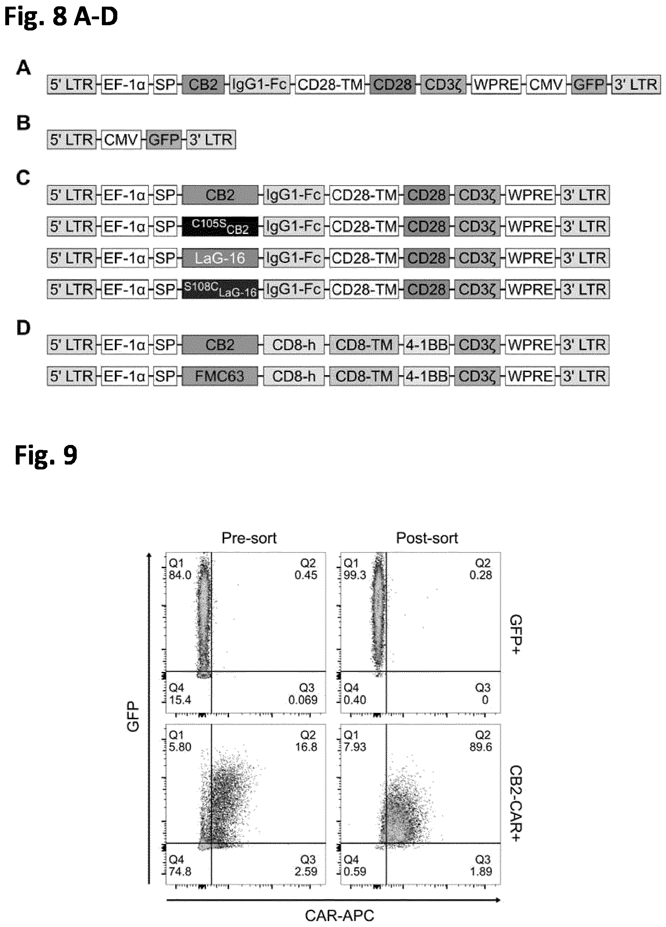

**Fig. 8 A-D** Overview of lentiviral constructs. (A) CB2-CD28-CAR_GFP construct. (B) GFP control construct. (C) CD28-CAR constructs. (D) 41BB-CAR constructs. Abbreviations: LTR - long terminal repeat, EF-1$\alpha$ - human elongation factor-1 alpha promotor, SP - signal peptide, IgG1-Fc - human IgG1-Fc hinge, CD28-TM - CD28 transmembrane domain, CD28 - CD28 costimulatory domain, CD3$\zeta$ - CD3$\zeta$ signaling domain, WPRE - Woodchuck Hepatitis Virus Posttranscriptional Regulatory Element, CMV - human cytomegalovirus enhancer and promotor, GFP - green fluorescent protein, CD8-h - CD8 hinge, CD8-TM - CD8 transmembrane domain, 4-1BB - 4-1BB costimulatory domain.

**Fig. 9** Sorting efficiency of CB2-CD28-CAR_GFP and GFP control T cells. Cells were sorted using a FACSMelody™ Cell Sorter. Pre-sorting (left panels) and post-sorting (right panels) efficiencies were measured by staining with MonoRab™ Rabbit Anti-Camelid VHH Cocktail iFluor 647 and detecting antibody fluorescence (CAR-APC) and GFP fluorescence (GFP).

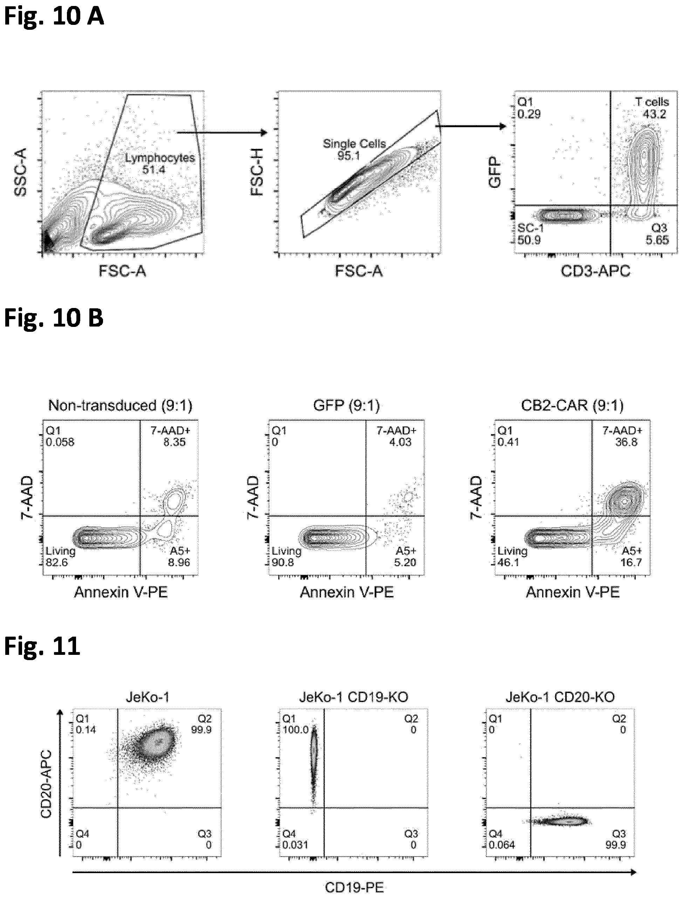

**Fig. 10 A-B** Gating strategy for flow cytometry-based apoptosis assays. Co-cultured cells were stained with anti-CD3-APC antibody, Annexin V-PE, and 7-AAD Viability Staining Solution and measured on a FACSCanto™ II Flow Cytometry System. (A) Gating of single target cells (SC-1). Cells were gated for lymphocytes (left panel), single cells (middle panel), and SC-1 cells (GFP-/CD3-APC-; right panel). (B) Gating of apoptotic target cells. Single SC-1 cells (gated in A) were further gated for Annexin V-PE+/7-AAD- cells (early apoptotic cells) and Annexin V-PE+/7-AAD+ cells (late apoptotic cells). Exemplary plots for effector to target ratio of 9:1 are shown. Abbreviations: FSC - forward scatter, SSC - side scatter.

**Fig. 11** Knockout efficiencies of JeKo-1 cell lines. Cells were stained with anti-CD19-PE and anti-CD20-APC antibodies and measured on a FACSCanto™ II Flow Cytometry System. Gates were adjusted according to non-stained control (not shown). Left panel: JeKo-1, middle panel: JeKo-1 CD19-knockout, right panel: JeKo-1 CD20-knockout.

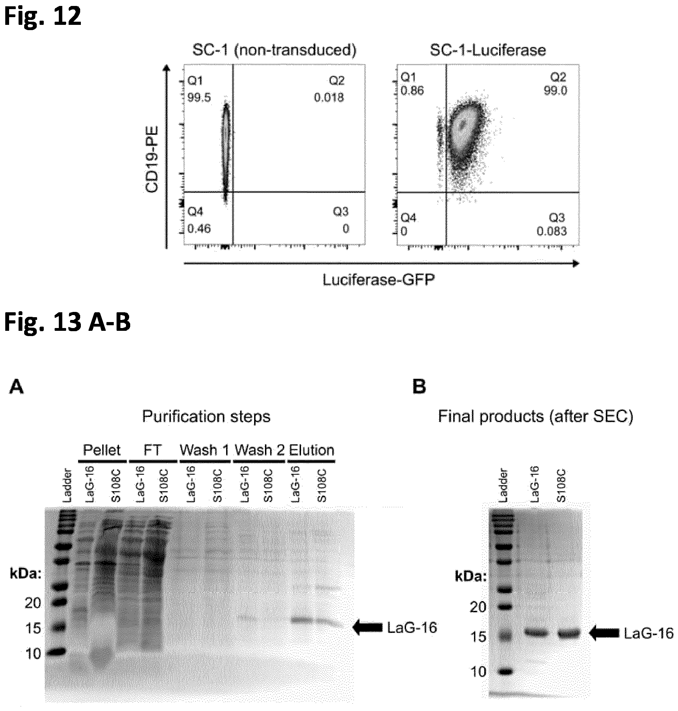

**Fig. 12:** Validation of firefly luciferase-expressing SC-1 cells. SC 1 cell line was transduced with lentivirus carrying firefly luciferase and GFP. Successfully transduced cells were single-cell sorted on a FACSMelody™ Cell Sorter and expanded. The verification of the clone used for subsequent assays is shown. Cells were stained with anti-CD19-PE antibody and measured on a FACSCanto™ II Flow Cytometry System. CD19 and Luciferase-GFP expression was assessed to confirm successful generation of SC-1_Luciferase cells.

**Fig. 13:** SDS-PAGE gels of WT LaG-16 and S108CLaG-16 protein purification. Sodium dodecylsulfate polyacrylamide gel electrophoresis (SDS-PAGE) separation demonstrating protein purity during the affinity purification of WT LaG-16 and S108CLaG-16 nanobodies (A) and the final protein product used for the different experiments after size exclusion chromatography (SEC) separation (B).

## Detailed description

The present invention provides a chimeric antigen receptor (CAR) that includes an antigen-binding domain, a hinge, a transmembrane domain, a co-stimulatory domain, and an activating domain. The CAR is capable of binding to an antigen. The antigen-binding domain comprises a heavy chain variable region, and optionally a light chain variable region. Each VH, and optionally VL, includes three complementarity-determining regions (CDRs). CDRs are the parts of the antigen-binding domain that directly interact with the antigen. They are typically formed by loops of amino acids that extend out from the antigen-binding domain and make contact with the antigen. The specific sequence and structure of the CDRs determine the specificity of the CAR for its target antigen.

In the present invention, at least one of the CDRs includes at least one amino acid residue other than cysteine, which has a solvent-accessible surface area from 0.00 to 0.75 and is located at a distance to the antigen from 3 to 14 Å. This amino acid residue is substituted to a thiol group containing amino acid.

A thiol group containing amino acid is for example cysteine. Cysteine is a unique amino acid that contains a thiol group, which can form a disulfide bond with another cysteine residue. This can result in a more stable binding interaction between the CAR and the antigen, which can enhance the effectiveness of the CAR in recognizing and responding to the antigen.

The term 'thiol group containing amino acid' as used herein refers to natural and unnatural cysteine analogs. In general, the term 'thiol group containing amino acid' as used herein refers to any amino acid, natural or unnatural comprising a thiol group. A thiol group containing amino acid may be any cysteine analog or derivative containing a free thiol. Natural cysteines analogs include, but are not limited to, cysteamine, 3-mercapto propionic acid, cysteine sulfinic acid, and S-sulfocysteine. Unnatural cysteine analogs include, but are not limited to, N-acetylcysteine (NAC), N, N'-Diacetyl-L-cystine, N, N'-Diacetyl-L-cystine Dimethylester (DACDM), S-Propylcysteine, S-Allylcysteine, S-propargyl-cysteine (SPRC), and S-Phenylcysteine. Most preferably, the thiol group containing amino acid is a cysteine. The thiol group containing amino acid is ideally selected from cysteine, cysteamine, 3-mercapto propionic acid, cysteine sulfinic acid, S-sulfocysteine, N-acetylcysteine (NAC), N, N'-Diacetyl-L-cystine, N, N'-Diacetyl-L-cystine Dimethylester (DACDM), S-Propylcysteine, S-Allylcysteine, S-propargyl-cysteine (SPRC), and S-Phenylcysteine. Most preferably, the thiol group containing amino acid is a cysteine.

The hinge, transmembrane domain, co-stimulatory domain, and activating domain of the CAR are also important components that contribute to the function of the CAR. The hinge provides flexibility to the CAR, allowing it to move and orient itself for optimal binding to the antigen. The transmembrane domain anchors the CAR in the cell membrane. The co-stimulatory domain and activating domain transmit signals into the cell upon binding of the CAR to the antigen, triggering an immune response.

The CAR of the present invention can be used in various applications in the field of CAR-T cell therapy for the treatment of cancer. In this therapy, T cells from a patient are genetically modified to express the CAR, and then infused back into the patient. The CAR T cells can then recognize and kill cancer cells that express the target antigen. The CAR T-cells as described herein have the ability to target cells, even when the cells show reduced expression of the antigen or if a subpopulation shows reduced expression or even a lack of expression due to the thiol group containing amino acid substitution in the CDR. This substitution enhances the effectiveness of the CAR T-cell therapy.

The solvent-accessible surface area of an amino acid residue refers to the surface area of the residue that is accessible to a solvent, such as water. Solvent-accessible surface area (SASA) is commonly known to the skilled person and the SASA quantifies the surface area of a biomolecule that is accessible to solvent molecules, typically water. SASA is defined as the surface area of a biomolecule that can be reached by a solvent molecule, which is often modeled as a sphere with a specific radius (commonly 1.4 Å, approximating the radius of a water molecule). The measurement of SASA was first introduced by Lee and Richards in 1971, and it is sometimes referred to as the Lee-Richards molecular surface. SASA is typically calculated using algorithms that simulate the rolling of a probe sphere over the surface of the biomolecule. The most common methods include the Shrake-Rupley Algorithm, the Linear Combination of Pairwise Overlaps (LCPO), or the Power Diagram Method. The skilled person is aware of suitable software to determine the SASA of proteins easily such as dr-sasa or GROMACS.

The distance to the antigen refers to the distance between the amino acid residue in the CDR and the antigen when the CAR is bound to the antigen. The skilled person is aware of software such as PyMol and Chimera to determine the distance based on e.g. a PDB file.

The skilled person is aware that SASA and the distance can be measured according to a PDB filed of the antigen and the antigen-binding domain, which may be part of e.g. an antibody, antibody fragment, or nanobody, as demonstrated experimentally herein. In other words, the PDB file typically does not contain the entire CAR.

These factors are crucial in determining the optimal position for the thiol group containing amino acid substitution, ensuring that the substituted thiol group containing amino acid residue is in a position where it can effectively interact with the antigen and contribute to the stability of the binding interaction.

In conclusion, the thiol group containing amino acid substitution in the CDRs of the CAR is a strategic modification that takes into account the solvent-accessible surface area and the distance to the antigen. This enhances the effectiveness of the CAR in recognizing and responding to the antigen, thereby improving the effectiveness of CAR T-cell therapy for the treatment of cancer.

The chimeric antigen receptor (CAR) as described can have a solvent-accessible surface area of the amino acid residue that is substituted to a thiol group containing amino acid ranging from 0.00 to 0.70. More preferably, the solvent-accessible surface area can range from 0.00 to 0.65. Even more preferably, the solvent-accessible surface area can range from 0.00 to 0.60. Even more preferably, the solvent-accessible surface area can range from 0.00 to 0.55. Most preferably, the solvent-accessible surface area can range from 0.00 to 0.50.

The solvent-accessible surface area (SASA) is a critical factor in determining the optimal position for the thiol group containing amino acid substitution in the CDRs of the CAR. The thiol group containing amino acid substitution can enhance the binding interaction between the CAR and the antigen, thereby improving the effectiveness of the CAR in recognizing and responding to the antigen. Experimental evidence regarding the solvent-accessible surface area is provided in Figure 6A, showing that CARs with a SASA in a range from 0.0 to 0.4 are in the most optimal range.

The chimeric antigen receptor as described can have an amino acid residue that is substituted to thiol group containing amino acid and may be located at a distance to the antigen ranging from 3 to 13 Å. More preferably, the distance to the antigen can range from 3.5 to 12 Å. Even more preferably, the distance to the antigen can range from 4 to 11 Å. Even more preferably, the distance to the antigen can range from 4 to 10 Å. Even more preferably, the distance to the antigen can range from 4 to 9 Å. Most preferably, the distance to the antigen can range from 4 to 8 Å. Experimental evidence fur such distances is provided in Figure 6B showing that distances from 4 to 8 Å, such as 4, 5, 6, 7 or 8 Å, are most preferred.

The distance to the antigen refers to the distance between the amino acid residue in the complementarity-determining regions of the CAR and the antigen when the CAR is bound to the antigen. This distance can be influenced by the three-dimensional structure of the CAR and the antigen, as well as the specific binding interaction between the CAR and the antigen.

The specific ranges of the distance to the antigen as described can provide optimal conditions for the thiol group containing amino acid substitution. These ranges can ensure that the substituted thiol group containing amino acid residue is at an appropriate distance from the antigen to allow for effective binding interaction. This can enhance the effectiveness of the CAR in recognizing and responding to the antigen, which can be beneficial in CART-cell therapy for the treatment of cancer. Experimental evidence regarding the solvent-accessible surface area is provided in Figure 6.

The chimeric antigen receptor as described can have a solvent accessibility and a distance to the antigen that are defined based on a structural model of the antigen and the antigen-binding domain. The structural model can be an X-ray structure, a cryo-EM-based model, or an AI-generated model. Preferably, the structural model is an X-ray structure.

The structural model can provide a detailed three-dimensional representation of the antigen and the antigen-binding domain, which can be used to determine the solvent accessibility and the distance to the antigen. The use of an X-ray structure, a cryo-EM-based model, or an AI-generated model can provide accurate and reliable information about the three-dimensional structure of the antigen and the antigen-binding domain. The skilled person is aware of software to use in order to determine the SASA and the distance such as Pymol or Chimera. Furthermore, the skilled person is aware of the PDB-database where several antigen-binding domains bound to antigens can be downloaded. Furthermore, the skilled person is aware of software modelling said interaction. Additionally, the skilled person is aware of AlphaFold and how to use it.

The chimeric antigen receptor as described can have an antigen-binding domain that binds the antigen with a dissociation constant (Kd) of at least 10 mM, preferably at least 8mM, more preferably at least 6 mM, even more preferably at least 5 mM, even more preferably at least 4 mM, even more preferably at least 3 mM, even more preferably at least 2mM and most preferably at least 1 mM. The Kd is a measure of the affinity of the antigen-binding domain for the antigen. A lower Kd indicates a higher affinity, meaning that the antigen-binding domain binds the antigen more tightly. A Kd of at least 1 mM indicates that the antigen-binding domain has a relatively high affinity for the antigen.

The Kd can be determined by various *in vitro* methods, which the skilled person is aware of. These methods include, for example, surface plasmon resonance (SPR), enzyme-linked immunosorbent assay (ELISA), fluorescence resonance energy transfer (FRET), Isothermal Titration Calorimetry (ITC), or MicroScale Thermophoresis (MST). SPR is preferred. Ideally, the $K_d$ to the antigen is determinable, wherein the antigen-binding domain is part of an antibody, a Fab fragment, a single chain variable fragment (scFV), single domain antibody fragment, antigen binding domain fragment, a diabody or a combination thereof. In other words, the Kd is ideally not determined by using the entire CAR. These methods can provide accurate and reliable measurements of the Kd, which can be used to evaluate the binding interaction between the antigen-binding domain and the antigen.

The ability of the antigen-binding domain to bind the antigen with a high affinity can enhance the effectiveness of the CAR in recognizing and responding to the antigen. This can be beneficial in CAR T-cell therapy for the treatment of cancer.

The chimeric antigen receptor as described can have an antigen-binding domain that is a Fab fragment, a single chain variable fragment, a single domain antibody fragment, an antigen-binding domain fragment, a diabody, or a combination thereof. The Fab fragment is a region on an antibody that binds to antigens. It is composed of one constant and one variable domain of each of the heavy and the light chain of the antibody. The variable domain contains the paratope that binds the antigen, and the constant domain aids in stabilizing the overall structure.

A single chain variable fragment (scFv) is a fusion protein of the variable regions of the heavy (VH) and light chains (VL) of immunoglobulins, connected with a short linker peptide of about 10-25 amino acids. This construct retains the specificity of the original monoclonal antibody and can be easily produced in bacteria or any other recombinant protein expression platform including mammalian, yeast, plant or cell-free platform, which the skilled person is aware of. A single domain antibody fragment, also known as a nanobody, is a type of antibody fragment consisting of a single monomeric variable antibody domain. They are derived from heavy-chain antibodies found in, e.g., camelids and sharks. An antigen-binding domain fragment is a portion of an antibody that includes the antigen-binding site. This fragment can be a part of a larger antibody molecule or can exist independently. A diabody is a bivalent molecule derived from an antibody. It is composed of two antigen-binding sites and is about one third the size of a full-sized antibody.

These different types of antigen-binding domains can provide various advantages in terms of their size, stability, and ability to bind the antigen. The use of these antigen-binding domains can enhance the effectiveness of the CAR in recognizing and responding to the antigen, which can be beneficial in CAR T-cell therapy.

The described chimeric antigen receptor (CAR) can include multiple antigen-binding domains, enhancing its ability to identify and bind to its target antigen. Each domain can be specific for a different or the same epitope. Multiple domains can boost the CAR's overall binding avidity, leading to an improved immune response, ideally to improved cytotoxicity.

In a preferred embodiment, these domains are fused, creating a single protein chain. This fusion enhances the CAR's stability and facilitates its expression in host cells. Multiple antigen-binding domains are especially beneficial in CAR T-cell therapy for cancer treatment.

The chimeric antigen receptor as described can have one, two, three, or four amino acid residues that are substituted to the thiol group containing amino acid. More preferably, the CAR can have one, two, or three amino acid residues that are substituted to the thiol group containing amino acid. Even more preferably, the CAR can have one or two amino acid residues that are substituted to the thiol group containing amino acid. Most preferably, the CAR has one amino acid residue that is substituted to the thiol group containing amino acid. This substitution of an amino acid residue to the thiol group containing amino acid enhances the interaction between the CAR and the target cell due to thiol group containing amino acid's unique ability to form a disulfide bond.

The chimeric antigen receptor (CAR) can have a thiol group containing amino acid substitution in the heavy chain variable region's complementarity-determining region 3 (CDR3). This substitution can enhance the CAR's antigen recognition and binding. The CDR3, known for its variability and extensive antigen contact, is an ideal location for a thiol group containing amino acid substitution. The thiol group containing amino acid substitution can be achieved through standard methods in the art like site-directed mutagenesis or genetic engineering, aiming to enhance binding interaction while preserving the CAR's structure and function.

The chimeric antigen receptor as described can have an antigen-binding domain that binds to a specific antigen. The antigen can be any one selected from CD19, GD2, CD276, CD20, CD22, CD33, CD123, CD38, BMCA, HER2, PMSA, mesothelin, NY-ESO-1, and EGFRvIII.

These antigens are all known targets for CAR T-cell therapy. They are proteins that are overexpressed on the surface of certain cancer cells, making them ideal targets for CAR T cells. CD19, CD20, CD22, CD33, CD123, and CD38 are all markers that are commonly found on the surface of B cells and are targets for CAR T-cell therapy for B-cell malignancies, such as lymphomas. BMCA is a marker for multiple myeloma. HER2 and EGFRvIII are markers for certain types of breast cancer and glioblastoma, respectively. PMSA is a marker for prostate cancer. Mesothelin is a marker for mesothelioma and certain types of ovarian and pancreatic cancer. NY-ESO-1 is a cancer-testis antigen that is expressed in various types of cancer. CD276 shows broad expression among cancers, especially solid tumors.

The supplementary information provided in the Example section offers experimental evidence that the antigen CD19, one of the targets for the chimeric antigen receptor (CAR), can be effectively targeted. This evidence is of significant importance as it substantiates that the CAR can bind to specific antigens, such as CD19, and shows impressive cytotoxicity towards the lymphoma in mice. Furthermore, a cysteine-substituted CAR, such as the exemplary cysteine-substituted anti-CD19 CAR, additionally shows a thiol-dependent binding to the cancer cells thereby enhancing the effectiveness of CAR T-cell therapy. This data is expected to be generalisable for other CARs based on the thiol group containing amino acid substitution according to the claims.

CD19 is a protein that is overexpressed on the surface of B cells, a type of white blood cell that is often implicated in various forms of cancer, including certain types of leukemia and lymphoma. The overexpression of CD19 makes these cancer cells an ideal exemplary target for CAR T-cell therapy. The experimental evidence provided in the Example section demonstrates that the CAR can bind to, e.g., CD19, with a high degree of specificity as well as in a thiol-dependent manner, enabling the CAR T cells to recognize and kill the cancer cells that express the antigen (see *in vivo* data, Figure 7). A further advantage of the CAR according to the invention is that these CARs do not show side effects. For example, the cysteine engineered anti-CD19 scFv FMC63 construct was administered to healthy mice and the mice did not show any side effects more than 42 days.

This experimental evidence is crucial as it not only validates the theoretical underpinnings of the patent claim but also provides practical proof of the efficacy of the CAR in targeting antigen specifically as well as in a thiol-dependent manner. It demonstrates the potential of the CAR to be used in the treatment of cancers, especially cancers that involve B cells, such as certain types of leukemia and lymphoma.

The evidence provided in the Example section also underscores the potential of the CAR to be used in the development of more effective treatments for various cancers. By demonstrating that the CAR can effectively target CD19, it suggests that the CAR could potentially be used to target other antigens that are overexpressed on the surface cancer cells, thereby broadening the scope of its potential applications in cancer treatment.

The binding domain of CD19 is ideally represented by scFV FMC63. The CD19 binding domain could be represented by a sequence that exhibits a minimum of 70% similarity to SEQ ID NO: 1. A defining characteristic of this specific domain of the chimeric antigen receptor according to SEQ ID NO: 1 is the presence of a thiol group containing amino acid substitution. This unique feature significantly enhances the binding interaction between the chimeric antigen receptor (CAR) and CD19. The chimeric antigen receptor as described can have a CD19 antigen-binding domain that has the sequence of SEQ ID NO: 1. In this sequence, the residue at position 229, position 228, or position 92 can be substituted to the thiol group containing amino acid. The substitution of an amino acid residue to the thiol group containing amino acid enhances the binding interaction between the CAR and CD19. The use of a CD19 antigen-binding domain with a thiol group containing amino acid substitution at position 229, position 228, or position 92 enhances the effectiveness of the CAR in recognizing and responding to CD19.

The chimeric antigen receptor as described can have a CD19 antigen-binding domain that has the sequence of SEQ ID NO: 1. In this sequence, the residue at position 229, position 228, or position 92 can be individually substituted to the thiol group containing amino acid.

The chimeric antigen receptor as described can have an antigen-binding domain that is CB2. CB2 is a naturally occurring nanobody discovered by the inventors in previous work, which has a single cysteine residue present in CDR3. More preferably, the CB2 antigen-binding domain comprises, and preferably consists of, a sequence having at least 70% identity to SEQ ID NO:5, which represents the amino acid sequence of the wild-type CB2 receptor. The sequence identity can be at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO:5.

The term "percentage identity" is used to denote the degree of identity that exists between two sequences of amino acids or nucleotides expressed as a percentage. This term is commonly known in the art.

In the antigen-binding domain, at least one amino acid residue in at least one CDR is substituted to the thiol group containing amino acid. The thiol group containing amino acid substitution can enhance the binding interaction between the CAR and CB2. The chimeric antigen receptor as described can have an antigen-binding domain that is LAG-16. LAG-16 is a commercially available nanobody targeting GFP. More preferably, the LAG-16 antigen-binding domain comprises, and preferably consists of, a sequence having at least 70% identity to SEQ ID NO:6, which represents the amino acid sequence of the wild-type LAG-16 gene. The sequence identity can be at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO:6. In the antigen-binding domain, at least one amino acid residue in at least one CDR is substituted to the thiol group containing amino acid. More preferably, an amino acid at position 108 is substituted to the thiol group containing amino acid with respect to SEQ ID NO:6.

The chimeric antigen receptor as described can have a hinge that is selected from a CD8 hinge, an IgG1 Fc hinge, a CD28 hinge, a FcεRIγ hinge, a NKG2D hinge, or a CD4 hinge.

The hinge region of the CAR is a flexible linker that connects the antigen-binding domain to the transmembrane domain. The hinge allows for movement and flexibility of the antigen-binding domain, which can enhance the ability of the CAR to bind to the antigen.

The CD8 hinge, IgG1 Fc hinge, CD28 hinge, FcεRIγ hinge, NKG2D hinge, and CD4 hinge are all known hinge regions that are used in the design of CARs. These hinge regions can provide various advantages in terms of their flexibility, length, and ability to transmit signals.

The CD8 hinge and the CD28 hinge are derived from T cell co-receptors and can provide strong T cell activation signals. The IgG1 Fc hinge and the FcεRIγ hinge are derived from immunoglobulins and can provide strong B cell activation signals. The NKG2D hinge is derived from a natural killer cell receptor and can provide strong natural killer cell activation signals. The CD4 hinge is derived from a T cell co-receptor and can provide strong T cell activation signals.

The chimeric antigen receptor as described can have a hinge that is a CD8 hinge. More preferably, the CD8 hinge comprises, and preferably consists of, a sequence having at least 70% identity to SEQ ID NO:7, which represents the amino acid sequence of the wild-type CD8 hinge. The sequence identity can be at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO:7. The CD8 hinge is derived from the CD8 co-receptor on T cells. It is known for its flexibility and its ability to transmit strong T cell activation signals.

The chimeric antigen receptor as described can have a hinge that is an IgG Fc hinge. More preferably, the IgG Fc hinge comprises, and preferably consists of, a sequence having at least 70% identity to SEQ ID NO:8, which represents the amino acid sequence of the wild-type IgG Fc hinge. The sequence identity can be at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO:8.

The IgG Fc hinge is derived from the Fc region of an IgG antibody. It is known for its flexibility and its ability to transmit strong B cell activation signals.

The chimeric antigen receptor as described can have a transmembrane domain that is selected from a CD28 transmembrane domain, a CD8 transmembrane domain, a CD3ζ transmembrane domain, a CD4 transmembrane domain, a FcεRIγ transmembrane domain, an OX40 transmembrane domain, a NKG2D transmembrane domain, or a 4-1BB transmembrane domain. The transmembrane domain of the CAR is a region of the protein that spans the cell membrane, anchoring the CAR to the cell. The transmembrane domain plays a crucial role in the stability of the CAR and its ability to transmit signals across the cell membrane. The CD28, CD8, CD3ζ, CD4, FcεRIγ, OX40, NKG2D, and 4-1BB transmembrane domains are all known transmembrane domains that are used in the design of CARs. These transmembrane domains can provide various advantages in terms of their stability and signaling capability. The CD28, CD8, CD3ζ, and CD4 transmembrane domains are derived from T cell co-receptors and can provide strong T cell activation signals. The FcεRIγ transmembrane domain is derived from an immunoglobulin receptor and can provide strong B cell activation signals. The OX40 and 4-1BB transmembrane domains are derived from costimulatory molecules and can provide strong costimulatory signals. The NKG2D transmembrane domain is derived from a natural killer cell receptor and can provide strong natural killer cell activation signals.

The chimeric antigen receptor as described can have a transmembrane domain that is a CD8 transmembrane domain. More preferably, the CD8 transmembrane domain comprises, and preferably consists of, a sequence having at least 70% identity to SEQ ID NO:9, which represents the amino acid sequence of the wild-type CD8 transmembrane domain. The sequence identity can be at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO:9.

The CD8 transmembrane domain is derived from the CD8 co-receptor on T cells. It is known for its stability and its ability to transmit strong T cell activation signals.

The chimeric antigen receptor as described can have a transmembrane domain that is a CD28 transmembrane domain. More preferably, the CD28 transmembrane domain comprises, and preferably consists of, a sequence having at least 70% identity to SEQ ID NO: 10, which represents the amino acid sequence of the wild-type CD28 transmembrane domain. The sequence identity can be at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 10.

The CD28 transmembrane domain is derived from the CD28 co-receptor on T cells. It is known for its stability and its ability to transmit strong T cell activation signals. The CD28 domain, as it is incorporated into the CAR, is preferably composed of a sequence that exhibits at least 70% identity to a specific sequence, designated as SEQ ID NO: 10.

In embodiments that are considered to be more preferred, the CD28 domain in the CAR may exhibit an identity to SEQ ID NO: 10 that reaches up to 100%.

In another embodiment, the co-stimulatory domain of the chimeric antigen receptor (CAR) is selected from a CD28 co-stimulatory domain, a 4-1BB co-stimulatory domain, an OX40 co-stimulatory domain, an ICOS domain, a CD27 domain, a CD40L co-stimulatory domain, a GITR domain, or a combination of any of them.

The co-stimulatory domain is a crucial component of the CAR as it provides the necessary signals for T cell activation and proliferation upon antigen recognition. Different co-stimulatory domains can provide different signals and thus influence the behaviour of the CART cells.

The CD28 co-stimulatory domain is derived from the CD28 protein, a co-stimulatory molecule expressed on T cells that plays a crucial role in T cell activation. The 4-1BB co-stimulatory domain is derived from the 4-1BB protein, another co-stimulatory molecule that can enhance T cell survival and proliferation. The OX40 co-stimulatory domain is derived from the OX40 protein, which can promote T cell survival and memory. The ICOS domain is derived from the ICOS protein, which can enhance T cell proliferation and cytokine production. The CD27 domain is derived from the CD27 protein, which can promote T cell survival and memory. The CD40L co-stimulatory domain is derived from the CD40L protein, which can enhance T cell activation and help stimulate the immune response. The GITR domain is derived from the GITR protein, which can enhance T cell activation and survival.

In some embodiments, the CAR may contain a combination of these co-stimulatory domains. This can provide a more robust and diverse set of signals to the T cells, potentially enhancing their anti-tumor activity. The specific choice of co-stimulatory domain or combination of co-stimulatory domains can be tailored to the specific needs of the therapy.

In a specific embodiment, the co-stimulatory domain of the chimeric antigen receptor is the CD28 co-stimulatory domain. The CD28 co-stimulatory domain is derived from the CD28 protein, a co-stimulatory molecule expressed on T cells that plays a crucial role in T cell activation.

The CD28 co-stimulatory domain in the CAR comprises, and preferably consists of, a sequence having at least 70% identity to a specific sequence, referred to as SEQ ID NO: 11. The percentage identity refers to the similarity between two amino acid sequences. In this case, the sequence of the CD28 co-stimulatory domain in the CAR is compared to SEQ ID NO: 11. The sequence of the CD28 co-stimulatory domain in the CAR may have at least 70% identity to SEQ ID NO: 11. In more preferred embodiments, the sequence of the CD28 co-stimulatory domain in the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 11.

In another specific embodiment, the co-stimulatory domain of the chimeric antigen receptor is the 4-1BB co-stimulatory domain. The 4-1BB co-stimulatory domain in the CAR comprises, and preferably consists of, a sequence having at least 70% identity to a specific sequence, referred to as SEQ ID NO: 12.

The sequence of the 4-1BB co-stimulatory domain in the CAR may have at least 70% identity to SEQ ID NO: 12. In more preferred embodiments, the sequence of the 4-1BB co-stimulatory domain in the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 12.

In another embodiment, the activating domain of the chimeric antigen receptor is a CD3ζ or a FcRγ. The activating domain is a crucial component of the CAR as it provides the necessary signals for T cell activation upon antigen recognition. The CD3ζ activating domain is derived from the CD3ζ protein, a component of the T cell receptor complex that plays a crucial role in signal transduction upon antigen recognition. The CD3ζ activating domain can provide a strong activation signal to the T cells, leading to their activation and proliferation. The FcRγ activating domain is derived from the FcRγ protein, a component of the Fc receptor complex that plays a crucial role in signal transduction upon binding of the Fc portion of antibodies. The FcRγ activating domain can provide a strong activation signal to the T cells, leading to their activation and proliferation.

In some embodiments, the activating domain of the chimeric antigen receptor is the CD3ζ activating domain. The CD3ζ activating domain is derived from the CD3ζ protein, a component of the T cell receptor complex that plays a crucial role in signal transduction upon antigen recognition. The CD3ζ activating domain in the CAR comprises, and preferably consists of, a sequence having at least 70% identity to a specific sequence, referred to as SEQ ID NO: 13. In more preferred embodiments, the sequence of the CD3ζ activating domain in the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 13.

In another embodiment, the chimeric antigen receptor additionally comprises a signal peptide. A signal peptide is a short peptide chain that directs the post-translational transport of a protein.

In the context of the chimeric antigen receptor, the signal peptide can guide the CAR to the cell membrane, where it can interact with its target antigen. The inclusion of a signal peptide in the CAR can therefore enhance the surface expression of the CAR on the T cells, potentially improving their ability to recognize and kill target cells. The specific sequence of the signal peptide can be selected based on the desired properties, such as the efficiency of protein targeting and the stability of the CAR.

In some embodiments, the signal peptide of the chimeric antigen receptor is a CD8 signal peptide. The CD8 signal peptide is derived from the CD8 protein, a surface glycoprotein found on certain immune cells, including T cells. The CD8 signal peptide guides the CD8 protein to the cell membrane during protein synthesis. The CD8 signal peptide in the CAR comprises, and preferably consists of, a sequence having at least 70% identity to a specific sequence, referred to as SEQ ID NO: 14. In more preferred embodiments, the sequence of the CD8 signal peptide in the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 14.

In another specific embodiment, the signal peptide of the chimeric antigen receptor is a granulocyte-macrophage colony-stimulating factor (GM-CSF) signal peptide. The GM-CSF signal peptide is derived from the GM-CSF protein, a cytokine that stimulates the production of granulocytes and macrophages in the bone marrow.

The GM-CSF signal peptide in the CAR comprises, and preferably consists of, a sequence having at least 70% identity to a specific sequence, referred to as SEQ ID NO: 15. In more preferred embodiments, the sequence of the GM-CSF signal peptide in the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 15.

In another embodiment, the chimeric antigen receptor (CAR) binds to a target cell in an antigen-dependent manner. This means that the CAR recognizes and binds to a specific antigen expressed on the surface of the target cell. This antigen-dependent binding is a key feature of CARs, as it allows them to specifically target and kill cells expressing the antigen.

In a preferred embodiment, the CAR additionally binds to the target in an antigen-independent manner. This means that the CAR can also bind to target cells that do not express the specific antigen. This antigen-independent binding could potentially enhance the ability of the CAR to target and kill cancer cells, as it would not be limited to cells expressing the specific antigen. This antigen-independent binding is driven by the presence of the thiol group containing amino acid, as defined in the claims. Furthermore, antigen-independent binding and ideally killing of the cells is abolished upon removal of the introduced thiol group containing amino acid. This abolishment of binding and killing shows that the targeting of the CAR is thiol-dependent and that this thiol group containing amino acid dependence provides a key advantage of the invention. In doing so the cellular microenvironment is co-targeted. Example 3 herein provides experimental evidence that the replacement of the thiol group containing amino acid by serine can lead to a complete loss of the T cells cytotoxic activity. Said antigen-independent binding is particularly advantageous when the CAR is used against a cancer undergoing antigen escape and/or shows an abnormal redox state. Said antigen-independent binding is also particularly advantageous for antigens that inherently demonstrates variable expression levels. For example, the antigen GD2 shows a variable expression in breast cancer patients,[38,39] in particular in triple negative breast cancer.[40] Some patients show a very high level of expression while other patient do not express GD2 at a detectable level. The advantage of a CAR showing thiol-dependent binding and cytotoxicity is that, e.g. a GD2 CAR is expected to be an efficient CAR against triple negative breast cancer cells showing high or low expression of GD2 using a GD2 specific CAR according to the claims.

In a more preferred embodiment, the target cell is a cancer cell. The ability of the CAR to bind to and kill cancer cells makes it a promising tool for cancer immunotherapy.

In an even more preferred embodiment, the binding of the antigen-binding domain of the CAR to the target cell is detectable by flow cytometry on antigen positive and antigen negative cells. Flow cytometry is a technique used to detect and measure physical and chemical characteristics of a population of cells. In this case, it can be used to detect the binding of the antigen-binding domain of the CAR to the target cells, regardless of whether they express the specific antigen or not.

This could provide a useful tool for evaluating the effectiveness of the CAR in binding to and killing target cells.

In a further embodiment, the antigen-independent binding of the chimeric antigen receptor to the target cell involves the formation of at least one bond between at least one thiol group containing amino acid residue within at least one complementarity-determining region (CDR) of the CAR and at least one thiol group containing amino acid residue present in at least one surface protein of a cell.

In the context of the CAR, the thiol group containing amino acid residue(s) in the CDR can form a disulfide bond with a cysteine residue in a surface protein of the target cell. This bond formation can lead to the antigen-independent binding of the CAR to the target cell.

This feature of the CAR can potentially enhance its ability to bind to and kill target cells. Even if the target cells do not express the specific antigen recognized by the CAR, the CAR can still bind to the target cells through the formation of disulfide bonds between the thiol group containing amino acid residues in the CAR and the target cells. This could potentially increase the range of target cells that the CAR can bind to and kill, enhancing its effectiveness in cancer immunotherapy.

The invention also encompasses a polynucleotide encoding the chimeric antigen receptor (CAR) as described herein. A polynucleotide is a biopolymer composed of 13 or more nucleotide monomers covalently bound in a chain. The polynucleotide may be DNA or RNA.

The polynucleotide of the invention encodes the specific sequence of amino acids that make up the CAR. This includes the sequences for the antigen-binding domain, the hinge, the transmembrane domain, the co-stimulatory domain, and the activating domain. If the CAR includes a signal peptide, the polynucleotide would also encode this sequence.

The polynucleotide can be introduced into a host cell, such as a T cell, using various genetic engineering techniques. Once inside the cell, the polynucleotide can be transcribed and translated to produce the CAR. The CAR can then be expressed on the surface of the cell, enabling the cell to recognize and bind to the target antigen.

The polynucleotide of the invention can be used in the production of CAR T cells for cancer immunotherapy. By introducing the polynucleotide into a patient's T cells, the T cells can be engineered to express the CAR and target the patient's cancer cells. This can potentially lead to a more effective and targeted immune response against the cancer.

In a further embodiment, the polynucleotide encoding the chimeric antigen receptor may also comprise additional sequences that facilitate its function within the host cell. These may include a genome integration sequence, a transcription regulation sequence, and/or a posttranscriptional regulatory sequence. A genome integration sequence is a specific DNA sequence that allows the polynucleotide to be integrated into the genome of the host cell. This can ensure stable and long-term expression of the CAR in the host cell. The integration sequence can be recognized by certain enzymes, such as integrases or recombinases, which can insert the polynucleotide at specific locations in the host cell's genome.

A transcription regulation sequence can control the expression of the CAR. This can include promoter sequences, which initiate transcription of the CAR gene, and enhancer sequences, which can increase the rate of transcription. By controlling the transcription of the CAR gene, the level of CAR protein produced in the host cell can be regulated.

A posttranscriptional regulatory sequence can influence the processing, stability, and translation of the CAR mRNA after it has been transcribed. This can include sequences that influence splicing, polyadenylation, mRNA stability, and translation efficiency.

The inclusion of these additional sequences in the polynucleotide can enhance the expression and function of the CAR in the host cell. This can potentially improve the effectiveness of CAR based therapies.

In specific embodiments, the genome integration sequences in the polynucleotide encoding the chimeric antigen receptor are 5' LTR (Long Terminal Repeat) and 3' LTR sequences. LTRs are sequences of DNA that repeat at either end of retroviral RNA. They are used in the process of integrating the viral genome into the host cell's DNA. In the context of the polynucleotide, the 5' LTR and 3' LTR sequences can facilitate the integration of the polynucleotide into the genome of the host cell.

The transcription regulation sequences in the polynucleotide are the human elongation factor-1 alpha promoter or the cytomegalovirus promoter. The human elongation factor-1 alpha promoter is a strong, constitutive promoter that can drive high levels of gene expression in a variety of cell types. The cytomegalovirus promoter is another strong, constitutive promoter that is commonly used in gene therapy and genetic engineering to drive high levels of gene expression.

The posttranscriptional regulatory sequence in the polynucleotide is the Woodchuck Hepatitis Virus Posttranscriptional Regulatory Element (WPRE). The WPRE is a sequence derived from the woodchuck hepatitis virus that can enhance the expression of downstream genes. It can increase the stability of the mRNA, enhance its export from the nucleus to the cytoplasm, and increase its translation efficiency.

The inclusion of these specific sequences in the polynucleotide can enhance the integration, expression, and function of the CAR in the host cell. This can improve the effectiveness of CAR T cell therapies.

In another embodiment, the polynucleotide encoding the chimeric antigen receptor is a DNA molecule.

The DNA molecule encoding the CAR can be introduced into a host cell, such as a T cell, using various genetic engineering techniques. Once inside the cell, the DNA can be transcribed into mRNA, which can then be translated to produce the CAR. The CAR can then be expressed on the surface of the cell, enabling the cell to recognize and bind to the target antigen.

The use of DNA as the polynucleotide encoding the CAR has several advantages. DNA is stable (more stable than RNA) and can be easily manipulated using standard molecular biology techniques. Furthermore, DNA can be integrated into the genome of the host cell, allowing for stable and long-term expression of the CAR. This can be particularly beneficial in the context of CAR T cell therapies, where long-term persistence of the CAR T cells can enhance their anti-tumor activity.

In a specific embodiment, the polynucleotide encoding the chimeric antigen receptor comprises at least one sequence having at least 70% identity to a specific sequence, referred to as SEQ ID NO: 16. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 16. SEQ ID NO: 16 is a specific DNA sequence that encodes a single-chain variable fragment (scFv) known as FMC63. This scFv is commonly used in the design of CARs due to its high affinity for the CD19 antigen, a protein commonly expressed on the surface of B cells. SEQ ID NO: 16 comprises at least one nucleotide substitution in at least one CDR encoding region that results in at least one amino acid residue mutated to the thiol group containing amino acid.

In another specific embodiment, the polynucleotide encoding the chimeric antigen receptor comprises at least one sequence having at least 70% identity to a specific sequence, referred to as SEQ ID NO: 17. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 17.

In a specific embodiment, the polynucleotide encoding the chimeric antigen receptor comprises at least one sequence having at least 70% identity to a specific sequence, referred to as SEQ ID NO: 18. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 18.

SEQ ID NO: 18 is a specific DNA sequence that encodes a particular variant of a chimeric antigen receptor. SEQ ID NO: 18 comprises at least one nucleotide substitution in at least one complementarity-determining region encoding region that results in at least one amino acid residue being substituted to the thiol group containing amino acid.

In a specific embodiment, the polynucleotide encoding the chimeric antigen receptor comprises at least one sequence having at least 70% identity to a specific sequence, referred to as SEQ ID NO: 19. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 19. SEQ ID NO: 19 is a specific DNA sequence that encodes a hinge region derived from the CD8 protein. The hinge region of the CAR provides flexibility and allows the antigen-binding domain to move relative to the rest of the CAR.

In a specific embodiment, the polynucleotide encoding the chimeric antigen receptor comprises at least one sequence having at least 70% identity to a specific sequence, referred to as SEQ ID NO: 20. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 20. SEQ ID NO: 20 is a specific DNA sequence that encodes a hinge region derived from the IgG Fc protein. The hinge region of the CAR provides flexibility and allows the antigen-binding domain to move relative to the rest of the CAR.

In a specific embodiment, the polynucleotide encoding the chimeric antigen receptor comprises at least one sequence having at least 70% identity to a specific sequence, referred to as SEQ ID NO: 21. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 21. SEQ ID NO: 21 is a specific DNA sequence that encodes a transmembrane domain derived from the CD8 protein. The transmembrane domain of the CAR anchors the CAR in the cell membrane.

In a specific embodiment, the polynucleotide encoding the chimeric antigen receptor comprises at least one sequence having at least 70% identity to a specific sequence, referred to as SEQ ID NO: 22. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 22. SEQ ID NO: 22 is a specific DNA sequence that encodes a transmembrane domain derived from the CD28 protein.

In a specific embodiment, the polynucleotide encoding the chimeric antigen receptor comprises at least one sequence having at least 70% identity to a specific sequence, referred to as SEQ ID NO: 23. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 23. SEQ ID NO: 23 is a specific DNA sequence that encodes a co-stimulatory domain derived from the CD28 protein.

In a specific embodiment, the polynucleotide encoding the chimeric antigen receptor comprises at least one sequence having at least 70% identity to a specific sequence, referred to as SEQ ID NO: 24. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 24. SEQ ID NO: 24 is a specific DNA sequence that encodes a co-stimulatory domain derived from the 4-1BB protein.

In a specific embodiment, the polynucleotide encoding the chimeric antigen receptor comprises at least one sequence having at least 70% identity to a specific sequence, referred to as SEQ ID NO: 25. T In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 25. SEQ ID NO: 25 is a specific DNA sequence that encodes an activating domain derived from the CD3ζ protein.

In a specific embodiment, the polynucleotide encoding the chimeric antigen receptor comprises at least one sequence having at least 70% identity to a specific sequence, referred to as SEQ ID NO: 26. The percentage identity refers to the similarity between two nucleotide sequences. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 26. SEQ ID NO: 26 is a specific DNA sequence that encodes a signal peptide derived from the CD8 protein.

In a specific embodiment, the polynucleotide encoding the chimeric antigen receptor comprises at least one sequence having at least 70% identity to a specific sequence, referred to as SEQ ID NO: 27. In more preferred embodiments, the sequence of the polynucleotide encoding the CAR may have at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 27. SEQ ID NO: 27 is a specific DNA sequence that encodes a signal peptide derived from the granulocyte-macrophage colony-stimulating factor (GM-CSF).

In another embodiment, the polynucleotide encoding the chimeric antigen receptor is an RNA molecule, preferably an mRNA molecule. The RNA molecule encoding the CAR can be introduced into a host cell, such as a T cell, using various genetic engineering techniques. Once inside the cell, the RNA can be directly translated to produce the CAR and does not need to be transcribed. The CAR can then be expressed on the surface of the cell, enabling the cell to recognize and bind to the target antigen.

The use of RNA as the polynucleotide encoding the CAR has several advantages. RNA can be easily synthesized and manipulated in the laboratory. Furthermore, RNA does not need to be integrated into the genome of the host cell to be expressed, which can reduce the risk of insertional mutagenesis. This can be particularly beneficial in the context of CAR T cell therapies, where safety is a major concern.

The invention also encompasses a polynucleotide encoding the chimeric antigen receptor (CAR). This polynucleotide includes a nucleotide sequence that codes for the antigen-binding domain, the hinge, the transmembrane domain, the co-stimulatory domain, and the activating domain of the CAR.

The antigen-binding domain encoded by the polynucleotide includes a heavy chain variable region, and optionally a light chain variable region. Each of these regions comprises three complementarity-determining regions (CDRs). At least one of the CDRs includes at least one amino acid residue other than cysteine, which has a solvent-accessible surface area from 0.00 to 0.75 and is located at a distance to the antigen from 3 to 14 Å. This amino acid residue is substituted to the thiol group containing amino acid in the encoded CAR.

The polynucleotide can be incorporated into a vector for delivery into cells, such as T cells. The vector can be a viral vector, such as a lentiviral or retroviral vector, or a non-viral vector, such as a plasmid. Once inside the cells, the polynucleotide is transcribed and translated to produce the CAR. The CAR is then expressed on the cell surface, where it can bind to the antigen and activate the cell.

The polynucleotide can be used in CAR T-cell therapy for treating cancer. In this therapy, T cells from a patient are genetically modified with the polynucleotide to express the CAR, and then infused back into the patient. The CAR enables the T cells to recognize and kill cancer cells expressing the antigen. The unique features of the CAR encoded by the polynucleotide can potentially enhance the effectiveness of this therapy.

The invention further provides a polynucleotide that is an RNA molecule, preferably a messenger RNA (mRNA), encoding the chimeric antigen receptor. This RNA molecule includes a nucleotide sequence that codes for the antigen-binding domain, the hinge, the transmembrane domain, the co-stimulatory domain, and the activating domain of the CAR.

The antigen-binding domain encoded by the RNA molecule includes a heavy chain variable region, and optionally a light chain variable region. Each of these regions comprises three CDRs. At least one of the CDRs includes at least one amino acid residue other than cysteine, which has a solvent-accessible surface area from 0.00 to 0.75 and is located at a distance to the antigen from 3 to 14 Å. This amino acid residue is substituted to the thiol group containing amino acid in the encoded CAR.

The use of an RNA molecule, particularly an mRNA, for encoding the CAR offers several advantages. For instance, mRNA does not integrate into the host genome, reducing the risk of insertional mutagenesis. Moreover, mRNA can be rapidly and transiently expressed in cells, allowing for controlled dosing and timing of CAR expression.

The RNA molecule can be delivered into cells, such as T cells, using various methods. For example, the RNA molecule can be encapsulated in lipid nanoparticles for delivery. Once inside the cells, the RNA molecule is translated to produce the CAR. The CAR is then expressed on the cell surface, where it can bind to the antigen and activate the cell.

The invention further provides a polynucleotide that encodes the chimeric antigen receptor and comprises at least one sequence having at least 70% identity to a specified sequence, SEQ ID NO: 28. The level of identity may be preferably at least 75%, more preferably at least 80%, and can increase incrementally as specified, up to most preferably 100% identical to SEQ ID NO: 28. SEQ ID NO: 28 is a specific nucleotide sequence that encodes a single-chain variable fragment (scFv) known as FMC63. This scFv is commonly used in CARs for its ability to bind to the CD19 antigen, a protein found on the surface of B cells, making it useful in CAR T-cell therapies for B cell malignancies. The disclosure also provides a polynucleotide that encodes the chimeric antigen receptor and comprises at least one sequence having at least 70% identity to a specified sequence, SEQ ID NO: 29. The level of identity may be preferably at least 75%, more preferably at least 80%, and can increase incrementally as specified, up to most preferably 100% identical to SEQ ID NO: 29.

The disclosure further provides a polynucleotide that encodes the chimeric antigen receptor and comprises at least one sequence having at least 70% identity to a specified sequence, SEQ ID NO: 30. The level of identity may be preferably at least 75%, more preferably at least 80%, and can increase incrementally as specified, up to most preferably 100% identical to SEQ ID NO: 30, wherein SEQ ID NO: 30 comprises at least one nucleotide substitution in at least one CDR encoding region that results in at least one amino acid residue substituted to cysteine.

The disclosure further provides a polynucleotide that encodes the chimeric antigen receptor and comprises at least one sequence having at least 70% identity to a specified sequence, SEQ ID NO: 31. The level of identity may be preferably at least 75%, more preferably at least 80%, and can increase incrementally as specified, up to most preferably 100% identical to SEQ ID NO: 31.

The disclosure further provides a polynucleotide that encodes the chimeric antigen receptor and comprises at least one sequence having at least 70% identity to a specified sequence, SEQ ID NO: 32. The level of identity may be preferably at least 75%, more preferably at least 80%, and can increase incrementally as specified, up to most preferably 100% identical to SEQ ID NO: 32. SEQ ID NO: 32 is a specific nucleotide sequence that encodes a particular variant of the hinge region of the chimeric antigen receptor.

The disclosure further provides a polynucleotide that encodes the chimeric antigen receptor and comprises at least one sequence having at least 70% identity to SEQ ID NO: 33. The disclosure further provides a polynucleotide that encodes the chimeric antigen receptor and comprises at least one sequence having at least 70% identity to SEQ ID NO: 34. The level of identity may be preferably at least 75%, more preferably at least 80%, and can increase incrementally as specified, up to most preferably 100% identical to SEQ ID NO: 34. The disclosure further provides a polynucleotide that encodes the chimeric antigen receptor and comprises at least one sequence having at least 70% identity to a specified sequence, SEQ ID NO: 35. The level of identity may be preferably at least 75%, more preferably at least 80%, and can increase incrementally as specified, up to most preferably 100% identical to SEQ ID NO: 35. The disclosure further provides a polynucleotide that encodes the chimeric antigen receptor and comprises at least one sequence having at least 70% identity to a specified sequence, SEQ ID NO: 36. The level of identity may be preferably at least 75%, more preferably at least 80%, and can increase incrementally as specified, up to most preferably 100% identical to SEQ ID NO: 36. SEQ ID NO: 36 is a specific nucleotide sequence that encodes a particular variant of the co-stimulatory domain of the chimeric antigen receptor. The disclosure further provides a polynucleotide that encodes the chimeric antigen receptor and comprises at least one sequence having at least 70% identity to a specified sequence, SEQ ID NO: 37. The level of identity may be preferably at least 75%, more preferably at least 80%, and can increase incrementally as specified, up to most preferably 100% identical to SEQ ID NO: 37. SEQ ID NO: 37 is a specific nucleotide sequence that encodes a particular variant of the activating domain of the chimeric antigen receptor. The disclosure further provides a polynucleotide that encodes the chimeric antigen receptor and comprises at least one sequence having at least 70% identity to a specified sequence, SEQ ID NO: 38. The level of identity may be preferably at least 75%, more preferably at least 80%, and can increase incrementally as specified, up to most preferably 100% identical to SEQ ID NO: 38. SEQ ID NO: 38 is a specific nucleotide sequence that encodes a particular variant of the signal peptide of the chimeric antigen receptor.

The disclosure further provides a polynucleotide that encodes the chimeric antigen receptor and comprises at least one sequence having at least 70% identity to a specified sequence, SEQ ID NO: 39. The level of identity may be preferably at least 75%, more preferably at least 80%, and can increase incrementally as specified, up to most preferably 100% identical to SEQ ID NO: 39. SEQ ID NO: 39 is a specific nucleotide sequence that encodes a particular variant of the signal peptide of the chimeric antigen receptor.

The disclosure also encompasses a carrier containing the polynucleotide encoding the chimeric antigen receptor (CAR). The carrier can be any suitable vehicle that can carry and deliver the polynucleotide to a target cell. The carrier can be a vector, such as a plasmid, a virus, or a bacteriophage. The vector can be designed to integrate the polynucleotide into the genome of the target cell or to maintain the polynucleotide as an episome in the target cell.

The carrier containing the polynucleotide encoding the CAR can be used to genetically modify cells, such as immune cells, to express the CAR on their surface. The modified cells can then recognize and bind to the specific antigen, leading to activation of the immune cells and triggering an immune response against cells expressing the antigen. This approach can be used in immunotherapy for cancer, where the antigen is overexpressed or uniquely expressed on the surface of the cancer cells.

The carrier containing the polynucleotide encoding the chimeric antigen receptor can be a nanoparticle. Due to their small size, nanoparticles can easily penetrate biological membranes and deliver their cargo directly to the target cells. This makes them an ideal carrier for delivering polynucleotides to cells.

In a preferred embodiment, the nanoparticle is a lipid nanoparticle. Lipid nanoparticles are composed of lipids and have a core-shell structure, with the core being hydrophobic and the shell being hydrophilic. This structure allows lipid nanoparticles to encapsulate the polynucleotide in their core and to be dispersed in aqueous solutions.

The lipid nanoparticle can encapsulate the polynucleotide encoding the chimeric antigen receptor and protect it from degradation. Upon delivery to the target cell, the lipid nanoparticle can fuse with the cell membrane and release the polynucleotide into the cell. The polynucleotide can then be transcribed and translated to produce the CAR, which can be expressed on the cell surface and bind to the specific antigen.

The use of lipid nanoparticles as a carrier for the polynucleotide encoding the CAR offers several advantages. First, lipid nanoparticles can efficiently deliver the polynucleotide to the target cells. Second, lipid nanoparticles can protect the polynucleotide from degradation, thereby increasing its stability. Third, lipid nanoparticles can be easily prepared and scaled up for large-scale production. Finally, lipid nanoparticles have been shown to be safe and well-tolerated in clinical trials, making them a promising carrier for gene therapy applications.

The disclosure also includes a viral vector containing the polynucleotide encoding the chimeric antigen receptor. Viral vectors are tools commonly used by molecular biologists to deliver genetic material into cells.

Examples of viral vectors that can be used include, but are not limited to, lentiviral vectors, adenoviral vectors, adeno-associated viral vectors, and retroviral vectors. The choice of viral vector can depend on various factors, such as the type of cells to be transduced, the size of the polynucleotide to be delivered, and the desired duration of CAR expression.

In a preferred embodiment, the viral vector containing the polynucleotide encoding the chimeric antigen receptor is a lentiviral vector. Lentiviruses are a subclass of retroviruses, known for their ability to integrate their genetic material into the genome of the host cell. This property makes lentiviral vectors an excellent tool for stable and long-term expression of the chimeric antigen receptor in the transduced cells.

The lentiviral vector can be engineered to contain the polynucleotide encoding the CAR. Upon infection of the target cells, the lentiviral vector can deliver the polynucleotide into the cells. The polynucleotide can then be integrated into the genome of the cells, leading to stable and long-term expression of the CAR on the cell surface.

The use of lentiviral vectors offers several advantages. First, lentiviral vectors can transduce both dividing and non-dividing cells, which broadens their application. Second, lentiviral vectors can deliver large sizes of genetic material, which allows for the inclusion of complex genetic constructs. Third, lentiviral vectors can provide stable and long-term expression of the CAR, which is beneficial for sustained immune response against the target antigen. The lentiviral vector can be produced using standard molecular biology techniques.

The disclosure also encompasses an engineered immune cell that expresses the chimeric antigen receptor on its surface membrane. The immune cell can be any type of immune cell capable of expressing the CAR, such as a T cell, a B cell, a natural killer cell, or a macrophage.

The engineered immune cell can be produced by introducing the polynucleotide encoding the CAR into the immune cell. This can be achieved using various methods, such as electroporation, viral transduction, or nanoparticle-mediated delivery. Once inside the cell, the polynucleotide can be transcribed and translated to produce the CAR, which can then be transported to the cell surface.

The engineered immune cell expressing the CAR can recognize and bind to the specific antigen, leading to activation of the immune cell and triggering an immune response against cells expressing the antigen. This approach can be used in immunotherapy for the treatment of cancer, where the antigen is overexpressed or uniquely expressed on the surface of the cancer cells.

The engineered immune cell expressing the chimeric antigen receptor can be derived from various types of immune cells. In particular, the immune cell can be a T-lymphocyte, a natural killer (NK) cell, a natural killer T (NKT) cell, or a macrophage. T-lymphocytes, or T cells, are a type of white blood cell that play a central role in cell-mediated immunity. They can recognize and kill infected cells or cancer cells, and they can also help other cells of the immune system to respond to infections or tumors. NK cells are a type of cytotoxic lymphocyte that can rapidly respond to viral-infected cells and tumor formation. They are unique among the immune cells in that they have the ability to recognize stressed cells in the absence of antibodies and major histocompatibility complex (MHC), allowing for a much faster immune reaction. NKT cells are a type of lymphocyte that share characteristics of both T cells and NK cells. They are known for their ability to recognize lipid antigens presented by CD1d, a non-polymorphic MHC class I-like molecule, and they can produce large amounts of cytokines upon activation. Macrophages are a type of white blood cell that engulf and digest cellular debris, foreign substances, microbes, and cancer cells in a process called phagocytosis. They also play a crucial role in initiating the immune response by presenting antigens to T cells and releasing cytoki nes.

The choice of immune cell type can depend on various factors, such as the type of disease to be treated, the specific antigen to be targeted, and the desired immune response. For example, T cells can be used for their potent cytotoxic activity against cancer cells, while NK cells can be used for their rapid response against viral infections. NKT cells can be used for their unique lipid antigen recognition capability, and macrophages can be used for their phagocytic activity and antigen presentation capability.

The T-lymphocyte, or T cell, from which the engineered immune cell is derived can be of various subtypes. These subtypes include cytotoxic T-lymphocytes, helper T-lymphocytes, regulatory T-lymphocytes, memory T-lymphocytes, naive T-lymphocytes, or $\gamma\delta$ T-lymphocytes.

Cytotoxic T-lymphocytes, also known as CD8+ T cells, are a type of T cell that kills cancer cells, cells that are infected, or cells that are damaged in other ways. They are a critical component of the immune system's response to disease. Helper T-lymphocytes, also known as CD4+ T cells, assist other white blood cells in immunologic processes, including maturation of B cells into plasma cells and memory B cells, and activation of cytotoxic T cells and macrophages. These cells are also known as CD4+ T cells because they express the CD4 glycoprotein on their surfaces. Regulatory T-lymphocytes, also known as Tregs, are crucial for the maintenance of immunological tolerance. Their major role is to shut down T cell-mediated immunity toward the end of an immune reaction and to suppress auto-reactive T cells that escaped the process of negative selection in the thymus. Memory T-lymphocytes are a subset of infection- as well as potentially cancer-fighting T cells that have previously encountered and responded to their cognate antigen. Thus, the term "memory T cells" reflects the ability of these cells to quickly recognize and respond to a pathogen upon re-infection. Naive T-lymphocytes are T cells that have not yet encountered the antigen they are programmed to respond to. $\gamma\delta$ T-lymphocytes represent a small subset of T cells that possess a distinct T cell receptor (TCR) on their surface. Most T cells have a TCR composed of two glycoprotein chains called $\alpha\beta$ TCR. In contrast, $\gamma\delta$ T cells possess a TCR that is made up of one $\gamma$ chain and one $\delta$ chain. This group of T cells is usually less common than $\alpha\beta$ T cells, but are at their highest abundance in the gut mucosa, within a population of lymphocytes known as intraepithelial lymphocytes.

The choice of T cell subtype can depend on various factors, such as the type of disease to be treated, the specific antigen to be targeted, and the desired immune response. For example, cytotoxic T cells can be used for their potent cytotoxic activity against cancer cells, while helper T cells can be used for their ability to stimulate other immune cells. Regulatory T cells can be used for their immunosuppressive activity, and memory T cells can be used for their ability to provide long-term immunity. Naive T cells can be used for their potential to differentiate into various T cell subtypes, and $\gamma\delta$ T cells can be used for their unique T cell receptor and tissue distribution.

The engineered immune cell expressing the chimeric antigen receptor is particularly useful in the treatment of cancer. The chimeric antigen receptor allows the immune cell to recognize and bind to a specific antigen expressed on the surface of cancer cells. Upon binding to the antigen, the immune cell is activated and triggers an immune response against the cancer cells. This can lead to the destruction of the cancer cells and the inhibition of tumor growth.

The use of the engineered immune cell for treating cancer offers several advantages. First, the treatment can specifically target and eliminate cancer cells that express the specific antigen targeted by the CAR, while sparing normal cells that do not express this antigen. Second, the treatment can induce a potent immune response against the cancer cells, leading to their destruction. Finally, the treatment can be personalized based on the specific antigen targeted by the CAR, allowing for the effective treatment of a wide range of cancers. Using the engineered immune cell according to the invention has the additional advantage that the immune cell binds to the target additionally in a thiol-dependent manner. In particular, the Example section provides ample evidence that, e.g. a CAR-T cell targeting CD19 shows great cytotoxicity against the cancer, wherein the CAR binds antigen specifically and in a thiol-dependent manner. A further advantage of the CAR according to the invention is that these CARs do not show side effects. For example, the cysteine engineered anti-CD19 scFv FMC63 construct was administered to healthy mice and the mice did not show any side effects more than 42 days.

The engineered immune cell can be administered to a patient suffering from cancer as part of a therapeutic regimen. The administration can be performed by various methods, such as intravenous injection, intratumoral injection, or infusion. The engineered immune cell can be administered alone or in combination with other treatments, such as chemotherapy, radiation therapy, surgery, or other immunotherapies.

The use of the engineered immune cell for treating cancer offers several advantages. First, the treatment is highly specific, as the CAR is designed to recognize a specific antigen expressed on the cancer cells. Second, the treatment can induce a potent immune response against the cancer cells, leading to their destruction. Third, the treatment can provide long-term immunity against the cancer, as the engineered immune cells can persist in the body and continue to attack the cancer cells. Finally, the treatment can be personalized, as the CAR can be designed to target antigens that are uniquely expressed or overexpressed on the patient's cancer cells. Specifically, the CAR may be tailored specifically to patients demonstrating altered redox state based on extracted biopsy tissues (e.g. measured by confocal microscopy or FACS with CB2 or the modified scFv used as CAR).

The engineered immune cell expressing the chimeric antigen receptor can be used in a method of treatment that inhibits cancer growth, metastasis formation, and/or prolongs survival. Ideally, the method of treatment also overcomes drug resistance.

The chimeric antigen receptor according to the invention, allows the immune cell to recognize and bind to the cancer cell. This is because the CAR according to the invention binds antigen specifically and ideally in an antigen-independent manner due to the thiol group containing amino acid substitution. This mechanism has the particular advantage of overcoming drug resistance. Upon binding to the target, such as CD19, the immune cell is activated and triggers an immune response against the cancer cells. This immune response can lead to the destruction of the cancer cells and the inhibition of tumor growth.

In addition to inhibiting tumor growth, the treatment can also prevent the formation of metastases. Metastasis is the process by which cancer cells spread from the primary tumor to other parts of the body. By destroying the cancer cells and inhibiting their growth, the treatment can prevent the cancer cells from invading other tissues and forming new tumors.

Furthermore, the treatment can prolong the survival of the patient. By effectively controlling the growth of the cancer and preventing metastasis, the treatment can extend the life expectancy of the patient.

The engineered immune cell expressing the chimeric antigen receptor can also be used in a method of treatment for cancers that comprise a subset of cancer cells characterized by a reduced expression of the CAR antigen. This includes cancers where the cells have undergone an antigen escape. Antigen escape is a phenomenon where cancer cells reduce the expression of the antigen targeted by the immune response, thereby evading detection and destruction by the immune system. This can lead to the survival and proliferation of these antigen-low or antigen-negative cancer cells, and can contribute to the recurrence or progression of the cancer.

The chimeric antigen receptor allows the immune cell to recognize and bind to the antigen, even if its expression is reduced on the cancer cells. Upon binding to the target, antigen dependently and ideally additionally in a thiol-dependent manner, the immune cell is activated and triggers an immune response against the cancer cells. This immune response can lead to the destruction of the cancer cells, including those with reduced antigen expression, and the inhibition of tumor growth. In other words, the cellular microenvironment is co-targeted via the thiol group containing amino acid.

The use of the engineered immune cell for treating cancers with reduced antigen expression offers several advantages. First, the treatment can target and even eliminate antigen-low or antigen-negative cancer cells, which are often resistant to conventional treatments. Second, the treatment can prevent or delay the recurrence or progression of the cancer caused by antigen escape.

The engineered immune cell expressing the chimeric antigen receptor can also be used in a method of treatment for cancers that comprise a subset of cancer cells that do not express the antigen targeted by the CAR. Furthermore, the engineered immune cell expressing the chimeric antigen receptor can also be used in a method of treatment for cancers that has the risk comprise a subset of cancer cells that do not express the antigen targeted by the CAR but so far does not yet exhibit such a subset. Antigen escape often occurs after the CAR treatment starts in order to escape the treatment. This includes cancers where a subset of the cancer cells, constituting at least 0.001% of the total cancer cell population, do not express the antigen targeted by the CAR, preferably at least 0.005%, more preferably at least 0.01%, even more preferably at least 0.05%, even more preferably at least 0.1%, even more preferably at least 0.5%. Ideally, the cancer cells, constituting at most 50% of the total cancer cell population, do not express the antigen targeted by the CAR. Furthermore, the cancer can have the risk of developing a subset of cancer cells, wherein said subset does not express the antigen targeted by the CAR. The advantage in reducing antigen escape is therefore also present for cancers with 0% or a not detectable but present amount of antigen negative cells.

The presence or absence of the targeted antigen on the cancer cells can be detected by various methods. These methods include fluorescence-activated cell sorting (FACS), Western blot, RNA sequencing (RNA-seq), or mass spectrometry. FACS is a specialized type of flow cytometry that uses fluorescent probes to detect and measure the properties of individual cells within a heterogeneous population. Western blot is a method used to detect specific proteins in a sample. RNA-seq is a technique that uses next-generation sequencing to reveal the presence and quantity of RNA in a biological sample. Mass spectrometry is an analytical technique that measures the mass-to-charge ratio of ions to identify and quantify molecules in simple and complex mixtures. Additional suitable methods include immunohistochemistry (IHC) staining and microscopy techniques on patient-derived tumor tissue sections, spheroids or organoids to determine the presence of specific tumor-associated antigens, all of which the skilled person is aware of.

The use of the engineered immune cell for treating cancers with a subset of antigen-negative cells offers several advantages. First, the treatment can target and eliminate antigen-negative cancer cells, which are often resistant to conventional treatments. Second, the treatment can prevent or delay the recurrence or progression of the cancer caused by antigen escape. Finally, the treatment can be personalized based on the specific antigen targeted by the CAR, allowing for the effective treatment of a wide range of cancers.

The engineered immune cell expressing the chimeric antigen receptor can also be used in a method of treatment for cancers where the targeted cancer cell exhibits at least a 1.5-fold increase in the expression level of at least one enzyme that catalyses the formation and/or breakage of disulfide bonds between cysteine residues, compared to the expression level of the same enzyme in a non-cancerous cell of the same cell type. It is preferred, the targeted cancer cell exhibits at least a 1.6-fold, more preferably at least 1.7-fold, more preferably 1.8-fold, more preferably 1.9-fold, and even more preferably 2-fold, increase in the expression level of at least one enzyme that catalyses the formation and/or breakage of disulfide bonds between cysteine residues.

The expression level of the enzyme can be detected by various methods, including mass spectrometry, RNA sequencing, or immunodetection methods, preferably mass spectrometry. Immunodetection methods can include techniques such as Western blot, which uses antibodies to detect specific proteins in a sample. The enzyme that catalyzes the oxidation of cysteine residues is preferably selected from protein disulfide isomerase or thioredoxin protein. Protein disulfide isomerase is an enzyme that catalyzes the formation and breakage of disulfide bonds between cysteine residues within proteins as they fold. This activity helps to stabilize the three-dimensional structures of proteins. Thioredoxin is a class of small redox proteins known to reduce disulfide bonds and can therefore regulate the redox state of proteins.

The use of the engineered immune cell for treating cancers with increased expression of enzymes that catalyze the oxidation of cysteine residues offers several advantages. First, the treatment can target and eliminate cancer cells with altered redox homeostasis, which are often resistant to conventional treatments. Second, the treatment can prevent or delay the recurrence or progression of the cancer caused by redox imbalance.

The engineered immune cell expressing the chimeric antigen receptor can also be used in a method of treatment for cancers where the targeted cancer cell expresses specific antigens on its surface, such as CD19, GD2, or CD276.

CD19 is a protein that is expressed on the surface of B cells, a type of white blood cell. It is a common target for CAR T cell therapy, particularly for the treatment of B cell malignancies such as acute lymphoblastic leukemia and non-Hodgkin lymphoma. GD2 is a disialoganglioside that is overexpressed on the surface of several types of cancer cells, including triple neuroblastoma, melanoma, osteosarcoma, breast cancer (including the triple negative form), retinoblastoma, brain tumors, pediatric rhabdomyosarcoma, and SCLC. It is a promising target for CAR T cell therapy due to its limited expression on normal tissues and its high expression on certain cancer cells. CD276, also known as B7-H3, is a cell surface protein that belongs to the B7 family of immune checkpoint molecules. It is overexpressed in various types of cancers, including breast, lung, prostate, sarcoma, and brain tumors, and is associated with tumor progression, poor prognosis, and resistance to therapy.

The use of the engineered immune cell for treating cancers that express, e.g., CD19, GD2, or CD276 offers several advantages. First, the treatment can specifically target and eliminate cancer cells that express these antigens, while sparing normal cells that do not express these antigens. Second, the treatment can induce a potent immune response against the cancer cells, leading to their destruction.

The engineered immune cell expressing the chimeric antigen receptor can be used in a method of treatment for various types of cancer, including hematological cancers and solid tumors.

Hematological cancers are cancers that begin in the cells of blood-forming tissue, such as the bone marrow, or in the cells of the immune system. Examples of hematological cancers include leukemia, lymphoma, and multiple myeloma. Solid tumors are an abnormal mass of tissue that usually does not contain cysts or liquid areas. Solid tumors can be benign or malignant. They can occur in several places, such as the breast, lung, colon, and prostate. Solid tumors are named for the type of cells that form them, such as a sarcoma, carcinoma, or lymphoma. Specific types of solid tumors that can be treated with the engineered immune cell include, but are not limited to, breast cancer, colon cancer, lung cancer, skin cancer, brain cancer, head and neck cancer, soft tissue cancer, gastrointestinal cancer, genitourinary and gynecologic cancer, musculoskeletal cancer, and endocrine cancer.

The engineered immune cell expressing the chimeric antigen receptor can also be used in a method of treatment for specific types of hematological cancers, such as lymphoma or leukemia.

The skilled person is aware that there are several types of lymphoma, including Hodgkin's lymphoma and non-Hodgkin's lymphoma. The skilled person is aware that there are several types of leukemia, including acute lymphoblastic leukemia, chronic lymphocytic leukemia, acute myeloid leukemia, and chronic myeloid leukemia.

The engineered immune cell expressing the chimeric antigen receptor can also be used in a method of treatment for specific types of lymphoma, such as Non-Hodgkin Lymphoma, Hodgkin lymphoma, Burkitt Lymphoma, multiple myeloma, or follicular lymphoma. The skilled person is aware of these types of lymphoma and also knows how to diagnose them.

The engineered immune cell expressing the chimeric antigen receptor can also be used in a method of treatment for specific types of leukemia, such as Lymphoblastic Leukemia, Acute Myeloid Leukemia, Chronic Lymphocytic Leukemia, Chronic Myeloid Leukemia, Chronic Myelomonocytic Leukemia, Hairy Cell Leukemia, Prolymphocytic Leukemia, Myelodysplastic Syndromes, or Mixed-Phenotype Acute Leukemia. The skilled person is aware of these types of leukaemias and how to diagnose them.

The engineered immune cell expressing the chimeric antigen receptor can also be used in a method of treatment for various types of cancer. In a specific embodiment, the CAR comprises an antigen-binding domain, a hinge, a transmembrane domain, a co-stimulatory domain, and an activating domain.

The antigen-binding domain of the CAR comprises a specific sequence, which can be the sequence represented by SEQ ID NO: 2, 3 or 4. This domain is responsible for recognizing and binding to the specific antigen expressed on the surface of the cancer cells.

The hinge of the CAR comprises the sequence represented by SEQ ID NO: 7. The hinge serves as a flexible linker that connects the antigen-binding domain to the transmembrane domain, allowing for the movement and flexibility of the CAR.

The transmembrane domain of the CAR may comprise the sequence represented by SEQ ID NO: 9. This domain anchors the CAR to the cell membrane, ensuring its proper localization on the cell surface.

The co-stimulatory domain of the CAR may comprise the sequence represented by SEQ ID NO: 12. This domain plays a crucial role in enhancing the activation and function of the immune cell upon binding of the CAR to the antigen.

The activating domain of the CAR may comprise the sequence represented by SEQ ID NO: 13. This domain is responsible for transmitting signals to the immune cell upon binding of the CAR to the antigen, leading to the activation of the immune cell and triggering an immune response against the cancer cells.

The engineered immune cell expressing the chimeric antigen receptor can also be used in a method of treatment for specific types of breast cancer, such as triple-negative breast cancer, HER2-positive breast cancer, or hormone receptor-positive breast cancer. The skilled person is aware of these types of breast cancer and also knows how to diagnose these types.

The engineered immune cell expressing the chimeric antigen receptor can also be used in a method of treatment for specific types of solid tumors, such as melanoma, sarcoma, osteosarcoma, retinoblastoma, pediatric rhabdomyosarcoma, or small cell lung cancer. The skilled person is aware of these solid tumours and how to diagnose them.

The engineered immune cell expressing the chimeric antigen receptor can also be used in a method of treatment for specific types of brain cancer, such as glioblastoma or neuroblastoma. The skilled person is aware of these brain cancers and how to diagnose them.

The engineered immune cell expressing the chimeric antigen receptor can also be used in an immunomodulating therapy. Immunomodulating therapies are treatments that harness the power of the body's own immune system to fight diseases, including cancer.

In the context of cancer treatment, the engineered immune cell can be used to enhance the body's immune response against cancer cells. The chimeric antigen receptor allows the immune cell to recognize and bind to a specific antigen expressed on the surface of the cancer cells. Upon binding to the antigen, the immune cell is activated and triggers an immune response against the cancer cells. This immune response can lead to the destruction of the cancer cells and the inhibition of tumor growth.

The invention also provides a method for generating a chimeric antigen receptor (CAR). The CAR comprises an antigen-binding domain, a hinge, a transmembrane domain, a co-stimulatory domain, and an activating domain. The antigen-binding domain includes a heavy chain variable region and, optionally, a light chain variable region. Each of these variable regions comprises three complementarity-determining regions (CDRs), which are responsible for the specific recognition and binding to the antigen.

The method for generating the CAR involves several steps. First, at least one amino acid residue suitable for thiol group containing amino acid substitution is selected. This amino acid residue is located within a CDR of the heavy chain variable region or the light chain variable region. The selected amino acid residue has a solvent-accessible surface area from 0.00 to 0.75 and is located at a distance to the antigen from 3 to 14 Å. The solvent-accessible surface area and the distance to the antigen are determined based on a structural model of the antigen and the antigen-binding domain. Second, the selected amino acid residue is mutated to the thiol group containing amino acid. This mutation can be performed using various techniques, such as site-directed mutagenesis, PCR-based mutagenesis, or CRISPR-Cas9-mediated genome editing. Third, the binding of the thiol group containing amino acid-engineered antigen-binding domain to the antigen is tested. This can be done using various methods, such surface plasmon resonance (SPR), enzyme-linked immunosorbent assay (ELISA), fluorescence resonance energy transfer (FRET), Isothermal Titration Calorimetry (ITC), MicroScale Thermophoresis (MST), size exclusion chromatography (SEC) or flow cytometry. The binding data can provide information about the affinity and specificity of the cysteine-engineered antigen-binding domain for the antigen, which can be used to evaluate the performance of the CAR.

The method for generating the CAR offers several advantages. First, it allows for the rational design of CARs with enhanced binding to the antigen. Second, it provides a systematic approach for improving the performance of CARs. Finally, it can be used to generate CARs for a wide range of antigens, allowing for the development of CAR-based therapies for various cancers.

The solvent-accessible surface area of the amino acid residue can influence the interaction of the chimeric antigen receptor with the antigen. By selecting an amino acid residue with a suitable solvent-accessible surface area for thiol group containing amino acid substitution, the binding of the chimeric antigen receptor to the antigen can be enhanced. This can improve the performance of the chimeric antigen receptor and increase the effectiveness of the immunotherapy.

In the method for generating the chimeric antigen receptor, the selection of the amino acid residue suitable for thiol group containing amino acid substitution is also based on its distance to the antigen. The distance of the amino acid residue to the antigen is an important factor that can affect the binding of the chimeric antigen receptor to the antigen.

The invention also provides a method for engineering an immune cell to express the chimeric antigen receptor on its surface membrane. The method involves several steps. First, an immune cell is provided. The immune cell can be any type of immune cell capable of expressing the CAR, such as a T cell, a B cell, a natural killer cell, or a macrophage. In a preferred embodiment, the immune cell is a T-lymphocyte. Next, at least one polynucleotide encoding the CAR is introduced into the immune cell. This can be achieved using various methods, such as electroporation, viral transduction, or nanoparticle-mediated delivery. Third, the polynucleotide is expressed in the immune cell, leading to the expression of the CAR on the cell surface. The expression of the CAR allows the immune cell to recognize and bind to the specific antigen, leading to activation of the immune cell and triggering an immune response against cells expressing the antigen.

The method for engineering the immune cell offers several advantages. First, it allows for the generation of immune cells that can specifically recognize and target cells expressing the specific antigen. Second, it provides a systematic approach for producing engineered immune cells for immunotherapy. Finally, it can be used to generate engineered immune cells for a wide range of antigens, allowing for the development of CAR-based therapies for cancer.

In a further embodiment, the method for engineering the immune cell to express the chimeric antigen receptor also includes a step of testing the cytotoxicity of the generated immune cell. This step is performed in parallel using two different cell lines: one that expresses the antigen and one that does not express the antigen. In particular, the latter may be a cell line, wherein the antigen has been knocked out. Alternatively, the latter may be a cell line, which inherently does not express the antigen.

The cytotoxicity of the engineered immune cell is measurable using luciferase-based cytotoxicity assays. Luciferase is an enzyme that facilitates the conversion of luciferin to oxyluciferin in the presence of ATP, 02, and Mg2+ to produce light. These assays are based on the principle that when a target cell is alive, light is produced after addition of the substrate luciferin. The amount of light produced is inversely proportional to the number of cells killed, providing a measure of the cytotoxicity of the engineered immune cell.

In this method according to the disclosure, the cytotoxicity of the engineered immune cell is greater than 15% at an effector-to-target ratio of 9:1 in both the antigen-expressing cell line and the antigen knockout cell line, preferably greater than 20%, more preferably greater than 25 %, even more preferably greater than 30%, even more preferably greater than 35%, even more preferably greater than 40%, even more preferably greater than 45%, and most preferably greater than 50%. Ideally, the cytotoxicity of the engineered immune cell is not greater than 99% at an effector-to-target ratio of 9:1 in both the antigen-expressing cell line and the antigen knockout cell line. This indicates that the engineered immune cell is capable of killing a significant proportion of the target cells, regardless of whether the target cells express the antigen or not. In such a case, engineered immune cell is capable of killing due to the thiol-dependent targeting activity.

This additional step of testing the cytotoxicity of the engineered immune cell provides a valuable measure of the effectiveness of the CAR in inducing an immune response against the target cells. It also provides a means of comparing the effectiveness of different CAR designs or different methods of engineering the immune cells.

In a specific embodiment of the method for engineering the immune cell, the generated immune cell expresses an anti-CD19 CAR on its surface. It is a common target for CART cell therapy, particularly for the treatment of B cell malignancies such as acute lymphoblastic leukemia and non-Hodgkin lymphoma. The Example section herein demonstrates that mice treated with an anti-CD19 CAR survived significantly longer as the CAR T-cell showed effective cytotoxicity.

The method further includes a step of testing the cytotoxicity of the generated immune cell. This step may be performed in parallel using two different cell lines: one that expresses CD19 (such as the JeKo-1 cell line) and one in which CD19 has been knocked out (such as the JeKo-1 CD19-KO cell line).

The cytotoxicity of the engineered immune cell may be measurable using luciferase-based cytotoxicity assays. In this method, the cytotoxicity of the engineered immune cell is greater than 50% at an effector-to-target ratio of 9:1 in both the CD19-expressing cell line and the CD19 knockout cell line. This indicates that the engineered immune cell is capable of killing a significant proportion of the target cells, regardless of whether they express CD19 or not. Hence, the cytotoxicity is based on the thiol-dependent targeting of the engineered immune cell comprising the CAR.

This additional step of testing the cytotoxicity of the engineered immune cell provides a valuable measure of the effectiveness of the anti-CD19 CAR in inducing an immune response against the target cells. It also provides a means of comparing the effectiveness of different CAR designs or different methods of engineering the immune cells.

The invention is further described in the following embodiments:

1. A chimeric antigen receptor (CAR) comprising

- an antigen-binding domain comprising a heavy chain variable region (VH), and optionally a light chain variable region (VL);

- a hinge;
- a transmembrane domain;
- a co-stimulatory domain; and
- an activating domain;

wherein the CAR is capable of binding to an antigen,
characterised in that the VH, and optionally the VL, comprise three complementarity-determining regions (CDRs), wherein at least one CDR comprises at least one amino acid residue other than cysteine, which has a solvent-accessible surface area from 0.00 to 0.75 and is located at a distance to the antigen from 3 to 14 Å, and wherein said at least one amino acid residue is substituted to a thiol group containing amino acid.

2. The CAR of embodiment 1, wherein the solvent-accessible surface area is from 0.00 to 0.70, more preferably area is from 0.00 to 0.65, even more preferably is from 0.00 to 0.60, even more preferably is from 0.00 to 0.55, and most preferably is from 0.00 to 0.50.

3. The CAR of any one of the preceding embodiments, wherein the residue for thiol group containing amino acid substitution is located at the distance to the antigen of 3-13 Å, more preferably of 3.5-12 Å, even more preferably of 4-11 Å, even more preferably of 4-10 Å, even more preferably of 4-9 Å, and most preferably of 4-8 Å.

4. The CAR of any one of the preceding embodiments, wherein the solvent accessibility and the distance to the antigen are defined based on a structural model of the antigen and the antigen binding domain, wherein the structural model is an X-ray structure, a cryo-EM-based model, or an AI-generated model, preferably an X-ray structure.

5. The CAR of any one of the preceding embodiments, wherein the antigen-binding domain of the CAR binds the antigen with at least 1 mM $K_d$, preferably wherein the $K_d$ is determinable by surface plasmon resonance (SPR), enzyme-linked immunosorbent assay (ELISA), Isothermal Titration Calorimetry (ITC), or MicroScale Thermophoresis (MST).

6. The CAR of any one of the preceding embodiments, wherein the antigen binding domain is a Fab fragment, a single chain variable fragment (scFV), single domain antibody fragment, antigen binding domain fragment, a diabody or a combination thereof.

7. The CAR of any one of the preceding embodiments comprising more than one antigen-binding domain, preferably wherein several domains are fused to each other.

8. The CAR of any one of the preceding embodiments, wherein one, two, three or four amino acid residue(s) are substituted to the thiol group containing amino acid, preferably one, two or three, more preferably one or two, and even more preferably wherein one amino acid residue is substituted to the thiol group containing amino acid.

9. The CAR of any one of the preceding embodiments, wherein the thiol group containing amino acid substitution is in the CDR3 of the VH of the antigen-binding domain.

10. The CAR of any one of the preceding embodiments, wherein the antigen-binding domain binds any one antigen selected from CD19, GD2, CD276, CD20, CD22, CD33, CD123, CD38, BMCA, HER2, PMSA, mesothelin, NY-ESO-1 and EGFRvIII.

11. The CAR of the embodiment 10, wherein the antigen-binding domain binds CD19, more preferably wherein the CD19 antigen-binding domain is scFV FMC63 or wherein the CD19 antigen-binding domain comprises and preferably consists of a sequence having at least 70% identity to SEQ ID NO: 1 [scFV FMC63], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 1; wherein at least one amino acid residue in at least one CDR is substituted to the thiol group containing amino acid.

12. The CAR of the embodiment 10, wherein the CD19 antigen-binding domain has the sequence of SEQ ID NO:1 and wherein the residue at the position 229 of SEQ ID NO:1 is substituted to the thiol group containing amino acid; and/or

wherein the CD19 antigen-binding domain has the sequence of SEQ ID NO:1 and wherein the residue at the position 228 of SEQ ID NO:1 is substituted to the thiol group containing amino acid;
and/or wherein the CD19 antigen-binding domain has the sequence of SEQ ID NO:1 and wherein the residue at the position 92 of SEQ ID NO:1 is substituted to the thiol group containing amino acid.

13. The CAR of the embodiment 10, wherein the CD19 antigen-binding domain has the sequence of SEQ ID NO:1 and wherein the residue at the position 229 of SEQ ID NO:1 is substituted to the thiol group containing amino acid; or

wherein the CD19 antigen-binding domain has the sequence of SEQ ID NO:1 and wherein the residue at the position 228 of SEQ ID NO:1 is substituted to the thiol group containing amino acid ;
or wherein the CD19 antigen-binding domain has the sequence of SEQ ID NO:1 and wherein the residue at the position 92 of SEQ ID NO:1 is substituted to the thiol group containing amino acid .

14. The CAR of any one of the embodiments 1-8, wherein the antigen-binding domain is CB2, more preferably wherein the antigen-binding comprises and preferably consists of a sequence having at least 70% identity to SEQ ID NO: 5 [CB2 WT], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 5.

15. The CAR of any one of the embodiments 1-8, wherein the antigen-binding domain is LAG-16, more preferably wherein the antigen-binding comprises and preferably consists of a sequence having at least 70% identity to SEQ ID NO: 6 [LAG16 WT], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 16; and wherein at least one amino acid residue in at least one CDR is substituted to the thiol group containing amino acid , preferably wherein an amino acid at the position 108 substituted to the thiol group containing amino acid with respect to SEQ ID NO:6.

16. The CAR of any one of the preceding embodiments, wherein the hinge is selected from a CD8 hinge, a IgG1 Fc hinge, a CD28 hinge, a FcεRIγ hinge, a NKG2D hinge or a CD4 hinge.

17. The CAR of the embodiment 16, wherein the hinge is the CD8 hinge, wherein the CD8 hinge comprises and preferably consists of a sequence having at least 70% identity to SEQ ID NO: 7 [CD8 hinge], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 7.

18. The CAR of the embodiment 16, wherein the hinge is IgG Fc hinge, wherein IgG Fc hinge comprises and preferably consists of a sequence having at least 70% identity to SEQ ID NO: 8 [IgG Fc hinge], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 8.

19. The CAR of any one of the preceding embodiments, wherein the transmembrane domain is selected from a CD28 transmembrane domain, a CD8 transmembrane domain, a CD3ζ transmembrane domain, a CD4 transmembrane domain, a FcεRIγ transmembrane domain, a OX40 transmembrane domain, a NKG2D transmembrane domain or a 4-1BB transmembrane domain.

20. The CAR of the embodiment 19, wherein the transmembrane domain is the CD8 transmembrane domain, wherein the CD8 transmembrane domain comprises and preferably consists of a sequence having at least 70% identity to SEQ ID NO: 9 [CD8 TM], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 9.

21. The CAR of the embodiment 19, wherein the transmembrane domain is the CD28 transmembrane domain, wherein the CD28 transmembrane domain comprises and preferably consists of a sequence having at least 70% identity to SEQ ID NO: 10 [CD28 TM], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 10.

22. The CAR of any one of the preceding embodiments, wherein the co-stimulatory domain is selected from a CD28 co-stimulatory domain, a 4-1BB co-stimulatory domain, a OX40 co-stimulatory domain, a ICOS domain, a CD27 domain, a CD40L co-stimulatory domain, a GITR domain, or combination of any of them.

23. The CAR of the embodiment 22, wherein the co-stimulatory domain is the CD28 co-stimulatory domain, wherein the CD28 co-stimulatory domain comprises and preferably consists of a sequence having at least 70% identity to SEQ ID NO: 11 [CD28 CD], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 11.

24. The CAR of the embodiment 22, wherein the co-stimulatory domain is he 4-1BB co-stimulatory domain, wherein the 4-1BB co-stimulatory domain comprises and preferably consists of a sequence having at least is at least 70% identity to SEQ ID NO: 12 [4-1BB CD], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 12.

25. The CAR of any one of the preceding embodiments, wherein the activating domain is a CD3ζ or a FcRγ.

26. The CAR of any one of the preceding embodiments, wherein the activating domain is the CD3ζ activating domain, wherein the CD3ζ, activating domain comprises and preferably consists of a sequence having at least 70% identity to SEQ ID NO: 13 [CD3 ζ SD], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 13.

27. The CAR of any one of the preceding embodiments, additionally comprising a signal peptide.

28. The CAR of the embodiment 27, wherein the signalling peptide is a CD8 signal peptide, wherein the CD8 signal peptide comprises and preferably consists of a sequence having at least 70% identity to SEQ ID NO: 14 [CD8 SP], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 14.

29. The CAR of the embodiment 27, wherein the signalling peptide is granulocyte-macrophage colony-stimulating factor signalling peptide (GM-CSF), wherein the GM-CSF signalling peptide comprises and preferably consists of a sequence having at least 70% identity to SEQ ID NO: 15 [GM-CSF], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 15.

30. The CAR of any one of the preceding embodiments, wherein the CAR binds to a target cell in an antigen-dependent manner, preferably wherein the CAR additionally binds to the target in an antigen-independent manner, more preferably wherein the target cell is a cancer cell, even more preferably wherein the binding is detectable by flow cytometry on antigen positive and antigen negative cells.

31. The CAR of embodiment 30, wherein the antigen-independent binding comprises formation of at least one bond between at least one thiol group containing amino acid residue within at least one CDR of the CAR and at least one cysteine residue present in at least one surface protein of a cell.

32. A polynucleotide encoding the CAR of any one of the preceding embodiments.

33. The polynucleotide of the embodiment 32, wherein the polynucleotide sequence comprises a genome integration sequence and/or a transcription regulation sequence, and/or a posttranscriptional regulatory sequence.

34. The polynucleotide of the embodiment 33, wherein the genome integration sequences are 5' LTR and 3' LTR sequences, the transcription regulation sequences are human elongation factor-1 alpha promotor or cytomegalovirus promoter and the posttranscriptional regulatory sequence is Woodchuck Hepatitis Virus Posttranscriptional Regulatory Element.

35. The polynucleotide of any one of the embodiments 32-34, wherein the polynucleotide is a DNA.

36. The polynucleotide of any one of the embodiments 32-35 wherein the polynucleotide comprises at least one sequence having at least 70% identity to SEQ ID NO: 16 [scFv FMC63 DNA] preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 16, wherein SEQ ID NO: 16 comprises at least one nucleotide substitution in at least one CDR encoding region that results in at least one amino acid residue mutated to the thiol group containing amino acid .

37. The polynucleotide of any one of the embodiments 32-35 wherein the polynucleotide comprises at least one sequence having at least 70% identity to SEQ ID NO: 17 [CB2 DNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 17.

38. The polynucleotide of any one of the embodiments 32-35 wherein the polynucleotide comprises at least one sequence having at least 70% identity to SEQ ID NO: 18 [LAG-16 WT DNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 18, wherein SEQ ID NO: 18 comprises at least one nucleotide substitution in at least one CDR encoding region that results in at least one amino acid residue substituted to the thiol group containing amino acid .

39. The polynucleotide of any one of the embodiments 32-38 wherein the polynucleotide comprises at least one sequence having at least 70% identity to SEQ ID NO: 19 [CD8 hinge], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 19.

40. The polynucleotide of any one of the embodiments 32-38 wherein the polynucleotide comprises at least one sequence having at least 70% identity to SEQ ID NO: 20 [IgG Fc hinge hinge], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 20.

41. The polynucleotide of any one of the embodiments 32-40 wherein the polynucleotide comprises at least one sequence having at least 70% identity to SEQ ID NO: 21 [CD8 TM], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 21.

42. The polynucleotide of any of the embodiments 32-40 wherein the polynucleotide comprises at least one sequence having at least 70% identity to SEQ ID NO: 22 [CD28 TM], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 22.

43. The polynucleotide of any of the embodiments 32-42 wherein the polynucleotide comprises at least one sequence having at least 70% identity to SEQ ID NO: 23 [CD28 CD], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 23.

44. The polynucleotide of any of the embodiments 32-43 wherein the polynucleotide comprises at least one sequence having at least 70% identity to SEQ ID NO: 24 [4-1BB CD], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 24.

45. The polynucleotide of any of the embodiments 32-44 wherein polynucleotide comprises at least one sequence having at least 70% identity to SEQ ID NO: 25 [CD3 $\zeta$ AD], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 25.

46. The polynucleotide of any of the embodiments 32-45 wherein polynucleotide comprises at least one sequence having at least 70% identity to SEQ ID NO: 26 [CD8 SP], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 26.

47. The polynucleotide of any of the embodiments 32-45 wherein polynucleotide comprises at least one sequence having at least 70% identity to SEQ ID NO: 27 [GM-CSF SP], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 27.

48. The polynucleotide of any one of the embodiments 32-34 wherein the polynucleotide is an RNA, preferably an mRNA.

49. The polynucleotide of any of the embodiments 32-34 or 48, wherein the polynucleotide comprises at least one sequence having at least 70% identity to SEQ ID NO: 28 [scFv FMC63 WT RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 28, wherein SEQ ID NO:28 comprises at least one nucleotide substitution in at least one CDR encoding region that results in at least one amino acid residue substituted to the thiol group containing amino acid .

50. The polynucleotide of any of the embodiments 32-34 or 48, wherein the polynucleotide comprises at least one sequence having at least 70% identity to SEQ ID NO: 29 [CB2 RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 29.

51. The polynucleotide of any of the embodiments 32-34 or 48, wherein the polynucleotide comprises at least one sequence having at least 70% identity to SEQ ID NO: 30 [LAG16 WT RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 30, wherein SEQ ID NO:30 comprises at least one nucleotide substitution in at least one CDR encoding region that results in at least one amino acid residue substituted to the thiol group containing amino acid .

52. The polynucleotide of any of the embodiments 32-34 or 48-51 wherein the polynucleotide comprises at least one sequence having at least 70% identity to SEQ ID NO: 31 [CD8 hinge], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 31.

53. The polynucleotide of any of the embodiments 32-34 or 48-51 wherein the polynucleotide comprises at least one sequence having at least 70% identity to SEQ ID NO: 32 [IgG Fc hinge RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 32.

54. The polynucleotide of any of the embodiments 32-34 or 48-53 wherein polynucleotide comprises at least one sequence having at least 70% identity to SEQ ID NO: 33 [CD8 TM RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 33.

55. The polynucleotide of any of the embodiments 32-34 or 48-53 wherein polynucleotide comprises at least one sequence having at least 70% identity to SEQ ID NO: 34 [CD28 TM RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 34.

56. The polynucleotide of any of the embodiments 32-34 or 48-55 wherein the polynucleotide comprises at least one sequence having at least 70% identity to SEQ ID NO: 35 [CD28 CD], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 35.

57. The polynucleotide of any of the embodiments 32-34 or 48-56 wherein the polynucleotide comprises at least one sequence having at least 70% identity to SEQ ID NO: 36 [4-1BB CD], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 36.

58. The polynucleotide of any of the embodiments 32-34 or 48-57 wherein the polynucleotide comprises at least one sequence having at least 70% identity to SEQ ID NO: 37 [CD3 ζ SD RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 37.

59. The polynucleotide of any of the embodiments 32-34 or 48-58 wherein the polynucleotide comprises at least one sequence having at least 70% identity to SEQ ID NO: 38 [CD8 SP RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 38.

60. The polynucleotide of any of the embodiments 32-34 or 48-58 wherein the polynucleotide comprises at least one sequence having at least 70% identity to SEQ ID NO: 39 [GM-CSF SP RNA], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 39.

61. A carrier containing the polynucleotide encoding the CAR of any one of the embodiments 1-31.

62. The carrier of the embodiment 61, wherein the carrier is a nanoparticle, more preferably a lipid nanoparticle.

63. A viral vector containing the polynucleotide encoding the CAR according to any one of the embodiments 1-31.

64. The viral vector of the embodiment 63, wherein the viral vector is a lentiviral vector.

65. An engineered immune cell expressing at the surface membrane the CAR according to any one of the embodiments 1 to 31.

66. The engineered immune cell according to the embodiment 65, wherein the engineered immune cell is derived from a T-lymphocyte, a NK cell, a NKT cell, or a macrophage.

67. The engineered immune cell according to the embodiment 66, wherein the T-lymphocyte is selected from cytotoxic T-lymphocyte, helper T-lymphocyte, regulatory T-lymphocyte, memory T-lymphocyte, naive T-lymphocyte or γδ T-lymphocyte.

68. The engineered immune cell according to any of embodiments 65-67 **for use** in a method of treating a cancer.

69. The engineered immune cell as defined in any one of the embodiments 65-67 for use in the method of treatment according to the embodiment 68, wherein the method of treatment delays cancer growth, metastasis formation and/or prolongs survival.

70. The engineered immune cell as defined in any one of the embodiments 65-67 for use in the method of treatment according to any of the embodiments 68-69, wherein the cancer comprises a subset of cancer cells that is

characterised in a reduced or no expression of the CAR antigen, in particular wherein the cancer cells have undergone an antigen escape.

71. The engineered immune cell according to any one of embodiments 65-67, for use in the method of treatment as defined in the embodiment 70, wherein the cancer comprises a subset of cancer cells that do not express the antigen targeted by the CAR, and wherein:

said antigen-negative subset is at least 0.5% of the total cancer cell population as determinable by FACS; preferably the presence or absence of the targeted antigen is detectable, preferably by FACS, Western blot, RNA-seq or mass spectrometry.

72. The engineered immune cell according to any one of embodiments 65-67, for use in the method of treatment according to any one of the embodiments 68-71, wherein:

the cancer cell targeted in said method of treatment has at least a 2-fold increase in the expression level of at least one enzyme that catalyzes oxidation of cysteine residues, compared to the expression level of said enzyme in a non-cancerous cell of the same cell type;
and preferably wherein the expression level of the enzyme is detectable by using mass spectrometry, RNA-seq or immunodetection methods, preferably a Western blot; and the enzyme is preferably selected from protein disulfide isomerase (PDI) or thioredoxin protein.

73. The engineered immune cell as defined in any one of the embodiments 65-67 for use in the method of treatment according to any one of the embodiments 68-69 or 72 wherein the cancer cell targeted in said method of treatment expresses CD19, GD2, or CD276 on its surface.

74. The engineered immune cell as defined in any one of the embodiments 65-67 for use in the method of treatment according to any one of the embodiments 68-73, wherein the cancer is a haematological cancer or a solid tumour, preferably wherein the solid tumor is breast cancer, colon cancer, lung cancer, skin cancer, brain cancer, head and neck cancer, soft tissue cancer, gastrointestinal cancer, genitourinary and gynecologic cancer, musculoskeletal cancer, sarcoma or endocrine cancer.

75. The engineered immune cell as defined in any one of the embodiments 65-67 for use in the method of treatment according to the embodiment 74, wherein the haematological cancer is a lymphoma or a leukemia.

76. The engineered immune cell as defined in any one of the embodiments 65-67 for use in the method of treatment according to the embodiment 74, wherein the lymphoma is Non-Hodgkin Lymphoma, Hodgkin lymphoma, Burkitt Lymphoma, multiple myeloma or follicular lymphoma.

77. The engineered immune cell as defined in any one of the embodiments 65-67 for use in the method of treatment according to the embodiment 74, wherein the leukemia is Lymphoblastic Leukemia (ALL), Acute Myeloid Leukemia (AML), Chronic Lymphocytic Leukemia (CLL), Chronic Myeloid Leukemia (CML), Chronic Myelomonocytic Leukemia (CMML), Hairy Cell Leukemia (HCL), Prolymphocytic Leukemia (PLL), Myelodysplastic Syndromes (MDS), or Mixed-Phenotype Acute Leukemia (MPAL).

78. The engineered immune cell as defined in any one of the embodiments 65-67 for use in the method of treatment according to the embodiment 74, wherein said CAR comprises an antigen-binding domain, comprising the sequence SEQ ID NO: 2, 3 or 4;

a hinge, comprising the sequence SEQ ID NO: 7;
a transmembrane domain, comprising the sequence SEQ ID NO: 9;
a co-stimulatory domain, comprising the sequence SEQ ID NO: 12;
an activating domain, comprising the sequence SEQ ID NO: 13.

79. The engineered immune cell as defined in any one of the embodiments 65-67 for use in the method of treatment according to the embodiment 74, wherein the breast cancer is triple-negative breast cancer, HER2-positive breast cancer or hormone receptor-positive breast cancer.

80. The engineered immune cell as defined in any one of the embodiments 65-67 for use in the method of treatment according to the embodiment 74, wherein the solid tumour is melanoma, sarcoma, osteosarcoma, retinoblastoma, pediatric rhabdomyosarcoma or small cell lung cancer.

81. The engineered immune cell as defined in any one of the embodiments 65-67 for use in the method of treatment according to the embodiment 74, wherein the brain cancer is a glioblastoma or neuroblastoma.

82. A method of generating a CAR, wherein the CAR comprises

- an antigen-binding domain comprising a heavy chain variable region (VH) and, optionally, also a light chain variable region (VL);
- a hinge;
- a transmembrane domain;
- a co-stimulatory domain; and
- an activating domain;

and wherein the method comprises the steps of:

a) selecting at least one amino acid residue suitable for thiol group containing amino acid substitution, wherein said at least one amino acid residue is comprised within a CDR of VH or VL, has a solvent-accessible surface area from 0.00 to 0.75 and is located at a distance to the antigen from 3 to 14 Å, as defined based on a structural model of an antigen and antigen binding domain and
b) mutating selected residue to cysteine.

83. The method of embodiment 82, wherein at least one amino acid residue suitable for thiol group containing amino acid substitution has a solvent-accessible surface area from 0.00 to 0.70, more preferably from 0.00 to 0.65, even more preferably from 0.00 to 0.60, even more preferably from 0.00 to 0.55, and most preferably from 0.00 to 0.50.
84. The method of the embodiment 82 or 83, wherein at least one amino acid residue suitable for thiol group containing amino acid substitution is located at the distance to the antigen of 3.5-13 Å, even more preferably of 4-12 Å, even more preferably of 4-11 Å, even more preferably of 4-9 Å, and most preferably of 4-8 Å.
85. The method of embodiments 82-84, wherein the method additionally comprises step c) of testing the binding of the thiol group containing amino acid engineered antigen-binding domain to the antigen.
86. A method of engineering an immune cell comprising the steps of:

a) providing the immune cell; preferably wherein the immune cell is a T-lymphocyte;
b) introducing to said cell at least one polynucleotide as defined in any one of the embodiments 32-60;
c) expressing said polynucleotide in said cell thus expressing a CAR according to any of the embodiments 1-31 at the surface membrane of said immune cell.

87. The method of engineering the immune cell of the embodiment 87, wherein the method additionally comprises the step of:
d) testing the cytotoxicity of the generated immune cell expressing the CAR on its surface in parallel using:

i) a cell line expressing the antigen, and
ii) a cell line that that does not express the antigen,

wherein the cytotoxicity is measurable using luciferase-based cytotoxicity assays, and wherein the cytotoxicity is greater than 50% at an effector-to-target (E:T) ratio of 9:1 in both the antigen-expressing cell line and the antigen KO cell line.
preferably wherein ii) the cell line that that does not express the antigen is either a cell line wherein the antigen is knocked out (KO) or wherein the antigen which inherently does not express the antigen.

88. The method of engineering the immune cell of embodiment 87, wherein the immune cell generated in step c) expresses an anti-CD19 CAR on its surface; wherein the method additionally comprises the step of
d) testing the cytotoxicity of the generated immune cell expressing the CAR in parallel using:

i) a cell line expressing CD19, and
ii) a cell line that that does not express the antigen,

wherein the cytotoxicity is measurable using luciferase-based cytotoxicity assays, and wherein the cytotoxicity is greater than 50% at an effector-to-target (E:T) ratio of 9:1 in both the antigen-expressing cell line and the antigen KO cell line, preferably wherein the cell lines are i) is JeKo-1 cell and ii) JeKo-1 CD19-KO
preferably wherein ii) the cell line that that does not express the antigen is either a cell line wherein the antigen is knocked out (KO) or wherein the antigen which inherently does not express the antigen.

**Examples**

**Example 1.**

**CB2-CAR-T cells specifically target SC-1 lymphoma but not healthy PBMCs**

In the previous study by the inventors, it was demonstrated how CB2 nanobodies specifically target BCL cells via thiol interactions and exploit this mechanism for drug delivery.[25]Therefore, the first objective of this invention was to investigate whether CB2 is also suitable to guide CAR-T cells for thiol-dependent targeting of BCL (Figure 1A). For that, the inventors designed a second-generation CAR construct with CB2 as antigen fragment, human IgG1-Fc as hinge, and CD28 as transmembrane and costimulatory domains. GFP was part of the same vector and served as a reporter protein (CB2-CD28-CAR_GFP; Figure 1B).

Primary human T cells were successfully activated and transduced with lentivirus carrying the CB2-CD28-CAR_GFP or GFP control construct. For the CAR, a relatively low transduction efficiency of approximately 15% was achieved but the CAR-T cells were successfully enriched via FACS (Figure 1C; Figure 9). Immunophenotyping of CAR-T cells revealed that the CD4/CD8 ratio was comparable to non-transduced T cells (Figure 1D).

To test the CAR-T cell activity, co-cultures with SC-1 cells (follicular lymphoma) were performed at different Effector to Target (E:T) ratios and apoptosis of the target cells was measured via flow cytometry. CB2-CAR-T cells exhibited significant concentration-dependent cytotoxicity compared with non-transduced and GFP control T cells (>50% at 9:1 ratio). Furthermore, co-cultures with healthy human PBMCs showed no cytotoxic activity (Figure 2A). To further profile CAR-T cell activation, the inventors performed ELISAs to test the secretion of pro-inflammatory cytokines IFN-$\gamma$ and TNF-$\alpha$ upon co-incubation with SC-1 cells. In agreement with the apoptosis assay results, CB2-CAR-T cells secreted approximately 4-fold higher amounts of both cytokines than control T cells, while healthy PBMCs did not trigger cytokine secretion (Figure 2B). To confirm activity on the effector cell level, overexpression of T cell activation markers, namely CD25 and CD69, and the degranulation marker LAMP 1 was assessed via flow cytometry. All three markers were significantly overexpressed in the case of CB2-CAR-T cells co-incubated with SC-1 (54%, 22%, and 39% for CD25, CD69, and LAMP-1, respectively), confirming T cell activation and degranulation (Figure 2C-D).

These results demonstrate the CB2-CAR-T cell activation upon co-culture with SC-1 lymphoma. More precisely, CAR-T cells exhibit cytotoxicity, secrete pro-inflammatory cytokines, and overexpress T cell activation markers. Based on these findings, the inventors were intrigued to inspect the therapeutic scope of CB2-CAR-T cells against other BCL subtypes and antigen escape models.

**Example 2.**

**CB2-CAR-T cells are effective against BCL subtypes and antigen escape models**

To compare the activity of CB2-CAR-T cells with a standard CD19-CAR-T therapy, the inventors changed the receptor configuration to match the clinically used Kymriah® format (CD19-CAR; Figure 3A), which was used as a positive control. For this, the inventors introduced CD8-derived hinge and transmembrane domains and the 4-1BB-derived costimulatory domain (CB2-41BB-CAR; Figure 3A) and generated CB2-41BB-CAR-T and CD19-CAR-T cells by lentiviral transduction with high efficiencies (>60%; Figure 3B).

To evaluate cytotoxicity, CAR-T cells were co-cultured with luciferase-expressing SC-1 cells, JeKo-1 cells (mantle cell lymphoma), and JeKo-1 cells with knocked-out CD19 or CD20 to model antigen escape (Figure 11). To demonstrate clinical relevance, the inventors pooled data from three independent experiments representing different blood donors for CAR-T cells (N=3, n=3). CB2-CAR-T cells exhibited significant cytotoxicity in a concentration-dependent manner against all tested BCL subtypes (Figure 3C). JeKo-1 CD19-knockout cells were targeted with high activity (≈80% specific lysis at 9:1 ratio), while they were resistant to CD19-CAR-T cells. For other BCL lines, the cytotoxicity of CB2-CAR-T cells was lower compared with CD19-CAR-T cells but consistently reached values above 50% (Figure 3C).

In conclusion, CB2-CAR-T cells are active against different BCL subtypes as well as antigen escape models. Moreover, a BCL line resistant to conventional CD19-directed CAR-T cells can be targeted efficiently. Therefore, the inventors aimed to investigate whether cysteine engineering could be applied to other nanobody-based CAR-T cells.

**Example 3.**

**Cysteine-engineered nanobodies can redirect CAR-T cells to target BCL**

As a proof-of-concept, the inventors chose to engineer the non-cancer-related anti-GFP nanobody LaG-16 with a cysteine in CDR3. As described in the previous publication by the inventors, the cysteine-to-serine mutant C105SCB2 exhibits no binding to SC-1 cells in flow cytometry.[25] Therefore, the inventors chose serine 108 in the CDR3 sequence of LaG-16 and introduced a reverse mutation, leading to S108CLaG-16. To assess whether this cysteine-mutant of LaG-16 could bind to SC-1 cells, the inventors performed flow cytometry binding assays. Indeed, S108CLaG-16 showed the same binding pattern as the wild type of CB2, whereas the wild type of LaG-16, like C105SCB2, did not bind (Figure 4A). Based on this result, it was hypothesized that the activity of CB2-CAR-T cells depends exclusively on the exposed cysteine in CDR3, and that nanobody-CARs of different specificity can be redirected towards BCL when endowed with such a cysteine.

To test this hypothesis, the inventors generated CAR-T cells expressing wild type CB2, C105SCB2, wild type LaG-16, and S108CLaG-16 (Figure 8). Transduction efficiencies differed repeatedly between constructs (Figure 4B), but the cell numbers in all assays were adjusted to reach the same amounts of effector cells. The inventors performed luciferase-based cytotoxicity assays with SC-1 cells to compare the activity of the different constructs. In accordance with the cell binding assays, both constructs comprising a cysteine in CDR3 (CB2-CAR and S108CLaG-16-CAR) showed significant cytotoxicity (>50% at 9:1 ratio), while the serine comprising counterparts (C105SCB2-CAR and LaG-16-CAR) did not show any activity. Moreover, activity of S108CLaG-16-CAR-T cells was higher at each E:T ratio compared to CB2-CAR-T cells (Figure 4C).

These results demonstrated how a non-cancer-related nanobody-based CAR-T cells can be redirected to target BCL by a single cysteine substitution on their CDR. Furthermore, replacement of this cysteine by serine leads to a complete loss of the T cells cytotoxic activity.

**Example 4.**

**Cysteine-engineering is applicable to scFv-based CD19-CAR and enables targeting of both CD19+ and CD19- lymphoma**

Encouraged by these results, the inventors turned to assess whether cysteine engineering can be applied to the clinically approved CD19-specific FMC63 scFv-based CAR. Thus, the inventors tested several cysteine-substituted CAR constructs. The cysteine mutations were chosen on the heavy (HC) and light (LC) chains of the scFv based on their locations in the CDR sequences of FMC63 and their surface exposure to ensure accessibility of the cysteine residue. In specific, N92- LC-CDR3, D156-HC-CDR1, S190-HC-CDR2, and G228 and S229-HC-CDR3 (Figure 5A-B). The two adjacent residues in HC-CDR3 were chosen to test whether the specific location in the CDR, as well as the identity of the mutated residue, influences activity. CAR-T cells were generated and co-cultured with JeKo-1 lymphoma to assess cytotoxicity. As anticipated, all constructs efficiently targeted CD19-expressing JeKo-1 cells, indicating that none of the mutations significantly interferes with CD19 binding (Figure 5C upper panel).

For the JeKo-1 CD19-knockout, mutations located in HC-CDR1 and HC-CDR2 showed only a slight increase in cytotoxicity compared to the WTCD19-CAR, indicating an impaired suitability of these CDRs for cysteine engineering. However, the mutations in both LC- and HC-CDR3 exhibited significantly higher cytotoxicity compared to WTCD19-CAR (Figure 5C lower panel). Therefore, S229CCD19-CAR was chosen as the candidate mutant for further in vivo inspections.

To confirm optimal cytotoxic activity and demonstrate clinical relevance, co-culture experiments were repeated three times with WTCD19-CAR- and S229CCD19-CAR-T cells generated from three independent blood donors. The significant cytotoxic effect of the S229C mutant against CD19-knockout lymphoma was confirmed (Figure 5D right panel), while CD19-expressing JeKo-1 cells were efficiently targeted by both constructs (Figure 5D left panel).

**Example 5.**

**S229CCD19-CAR-T cells prolong the survival of lymphoma-xenografted mice.**

For translation towards in vivo experiments, the inventors examined the effect of S229CCD19-CAR T cells in a mouse antigen escape model. Initially, the safety and off-target damage of the engineered S229CCD19-CAR T cells were examined by injecting them into healthy mice. All mice survived for over 42 days post-treatment. Therefore, the inventors injected xenografted mice with a 1:1 ratio of CD19-positive and CD19-negative lymphoma and subsequently treated those mice with CAR-T cells. Interestingly, in corroboration with the previous *in vitro* results, mice treated with S229CCD19-CAR-T cells show a delay in tumor formation as well as a prolonged survival compared to WTCD19-CAR-T cells (Figure 7). As shown in Figure 7A, mice not receiving a CAR (Tumor only) only survived at most 8 days. Mice treated with a WTCD19-CAR-T cells survived at most 15 days post CAR injection. Only mice treated with S229CCD19-CAR-T survived at least 25 days. Figures 7B and 7C depict the luciferase signal over time and it is immediately clear that only the S229CCD19-CAR-T cell treated mice showed less luciferase signal over time, a decrease in tumor growth, and thus also lived significantly longer. These results indicate that thiol-dependent targeting of antigen-escaped lymphoma is effective *in vivo*.

**Example 6.**

**Cysteine-engineering of scFv-based GD2-CAR for targeting of GD2+ and GD2-Triple-Negative Breast Cancer (TNBC) cells**

Encouraged by the CD19 results in mice, the inventors will assess whether cysteine engineering can be applied to the clinically tested GD2-specific 14G2a-scFv-based CAR against Triple-Negative Breast Cancer cells (TNBC). GD2 is a glycolipid recently demonstrated as a valid target in the highly lethal TNBC, but with varying expression levels between patients, from high expression to undetected GD2 levels. Importantly, the authors already demonstrated that abnormal cell membrane redox state on several breast cancer cell lines, including the MDA-MB-231 TNBC cell line.[25] Thus, the inventors aim to test three cysteine-substituted CAR constructs. The cysteine mutations are chosen on the heavy (HC) and light (LC) chains of the scFv based on previous experience from the CD19-CAR construct and available GD2-scFv crystal structures. Their chosen location in the CDR sequences of HC-CDR3 and LC-CDR3 aimed to ensure optimal surface exposure and accessibility of the cysteine residue. Tested mutants are M100C (HC-CDR3; SASA 0.07; distance 4.1 Å), H226C (LC-CDR3; SASA 0.33; distance 6.1 Å), and V227C (LC-CDR3; SASA 0.46; distance 3.6 Å). CAR-T cells will be generated and co-cultured with WT MDA-MB-231 TNBC cells not expressing GD2 to assess their cytotoxicity against antigen-negative target cells. It is expected that all mutant constructs will enable target cell killing, while WT GD2-CAR should be inactive against MDA-MB-231 cells. Next, we will confirm cytotoxicity of all GD2-CAR constructs, including WT GD2-CAR, against the GD2-positive TNBC cell line Hs 578T. Co-culture experiments are planned to be repeated three times with WT GD2-CAR- and mutant GD2-CAR-T cells, generated from three independent blood donors.

**Example 7.**

**Cysteine-engineering of scFv-based CD276-CAR for targeting of CD276+ and CD276- sarcoma cells**

The inventors will additionally assess the suitability of a scFv-based CAR against the tumor-associated antigen CD276 for cysteine engineering. First, the CD276-expressing sarcoma cell line Rh-30 will be investigated for altered redox states on the cell surface, enabling thiol-dependent targeting. Second, a CD276 knockout of Rh-30 will be generated through shRNA silencing. Next, cysteine engineered CD276-CAR-T cells will be generated and tested in cytotoxicity assays against CD276-positive and -negative Rh-30 cells as described above.

**Materials and Methods**

Design of CAR constructs

The second-generation CAR construct comprising human elongation factor-1 alpha promotor (EF-1$\alpha$) $\rightarrow$ CD8 signal peptide (SP) (SEQ ID NO: 25) $\rightarrow$ CB2 domain (SEQ ID NO: 17) $\rightarrow$ human IgG1-Fc hinge (SEQ ID NO: 20) $\rightarrow$ CD28 transmembrane domain (TM) (SEQ ID NO: 22) $\rightarrow$ CD28 costimulatory domain (CD) (SEQ ID NO: 23) $\rightarrow$ CD3$\zeta$ activating domain (AD) (SEQ ID NO: 25) $\rightarrow$ Woodchuck Hepatitis Virus Posttranscriptional Regulatory Element (WPRE) $\rightarrow$ human cytomegalovirus enhancer and promotor (CMV) $\rightarrow$ green fluorescent protein (GFP), abbreviated "CB2-CD28-CAR_GFP", was ordered as synthetic gene in the lentiviral transfer plasmid pLV from VectorBuilder Inc. (Chicago, IL). GFP control construct ("GFP") was cloned in-lab via restriction of the plasmid with SpeI followed by self-ligation.
A shorter version of the construct, abbreviated "CB2-CD28-CAR", was generated via deletion of GFP reporter using primers SEQ ID NO: 52 and 54. Briefly, the complete CAR construct was amplified via polymerase chain reaction (PCR) using a reverse primer with an overhang to insert a 3' KpnI restriction site. Then, the amplicon and the original backbone were restricted using XbaI (restriction site upstream CB2 domain) and KpnI (restriction site downstream GFP) and ligated. The [C105S]CB2 mutant of this construct was generated via overlap extension PCR as described by Heckman et al.[32], leading to "[C105S]CB2-CD28-CAR". Sequences of the anti-GFP nanobody LaG-16 and its mutant [S108C]LaG-16 were ordered as synthetic gene fragments from IDT Inc. (Coralville, IA). The sequence was published by Fridy et al.[33] The gene fragments were inserted into the CD28-CAR backbone via NEBuilder® HiFi DNA Assembly (NEB, Ipswich, MA), leading to "LaG-16-CD28-CAR" and "[S108C]LaG-16-CD28-CAR".
Furthermore, a second-generation CAR construct comprising granulocyte-macrophage colony-stimulating factor (GM-CSF) SP (SEQ ID NO: 27) instead of CD8-SP, CD8 hinge (SEQ ID NO: 19) instead of IgG1-Fc hinge, CD8-TM (SEQ ID NO: 21) instead of CD28-TM, and 4-1BB-CD (SEQ ID NO:24) instead of CD28-CD was generated. The original plasmid was provided by the Seitz lab, university of Tübingen, with anti-human CD19 single-chain variable fragment FMC63 as extracellular antibody fragment (abbreviated "CD19-CAR"). To clone the construct "CB2-41BB-CAR", FMC63 was replaced by CB2 via NEBuilder® HiFi DNA Assembly.
The FMC63-CAR cysteine mutants N92C, D156C, S190C, G228C, and S229C were ordered as codon-optimized synthetic DNA fragments (IDT, Coralville, IA, USA) and inserted into the pLV:CD19-CAR backbone via restriction and ligation cloning using EcoRV and NdeI (NEB, Ipswich, MA, USA) according to the manufacturer's instructions.

Lentiviral constructs used in this study are summarized in Figure 8.

Lentiviral packaging

HEK293T cells were transfected with pLV (carrying the respective CAR construct), psPAX2 (plasmid was a gift from Didier Trono, Addgene plasmid # 12260; http://n2t.net/addgene: 12260; RRID:Addgene_12260), and pMD2.G (plasmid was a gift from Didier Trono, Addgene plasmid # 12259; http://n2t.net/addgene:12259; RRID:Addgene_12259) in a T75 flask with 6.3 $\mu$g DNA per plasmid and 30 $\mu$l TransIT 293T transfection reagent (Mirus Bio LLC, Madison, WI). Cells were incubated at 37°C for 48 h. Afterwards, viral supernatant was centrifuged at 1500 g for 10 min and filtered through a 0.45 $\mu$m filter. For concentration, supernatant was layered on a sucrose buffer (50 mM Tris-HCl, 100 mM NaCl, 0.5 mM EDTA, 10% w/v sucrose, pH 7.4) at 4:1 v/v ratio in a 50 ml tube and centrifuged at 10 000 g/4°C for 4 h. Afterwards, the supernatant was removed, and the viral pellet was resuspended in 500 $\mu$l sterile PBS. For storage, virus suspensions were aliquoted and frozen directly at -80°C. Lentivirus concentration protocol was adapted from Jiang et al.[34]

CAR-T cell generation

Human whole blood samples were obtained from the German Red Cross (DRK-Blutspendedienst Nord-Ost, Institut Berlin). PBMCs were isolated using SepMate™-50 tubes (Stemcell Technologies, Vancouver, Canada). Afterwards, T cells were isolated using Pan T Cell Isolation Kit (Miltenyi Biotec, Bergisch Gladbach, Germany) and seeded at 1.0 million cells/ml in T cell medium (RPMI 1640, 10% fetal calf serum (FCS), 2 mM L-glutamine, 1 mM pyruvate, 100 U/ml Penicillin-Streptomycin) supplemented with 30 U/ml human Interleukin-2 (IL-2; Peprotech Inc., Cranbury, NJ). 25 $\mu$l Dynabeads™ Human T-Activator CD3/CD28 beads (ThermoFisher Scientific, Waltham, MA) per million of T cells were added. One day later, concentrated lentivirus was added in a 1:15 dilution alongside with 5 $\mu$g/ml polybrene. On day 2, medium was replaced with fresh T cell medium + IL-2. Cells were monitored daily and maintained between 0.5 and 1.0 million cells/ml. T cells transduced with CB2-CD28-CAR_GFP and GFP control were enriched via fluorescence-activated cell sorting (see respective section) on day 6. All other constructs were not sorted but transduction efficiencies were determined via flow cytometry. Briefly, all nanobody-based CAR-T cells were incubated with MonoRab™ Rabbit Anti-Camelid VHH Cocktail iFluor 647 (1:500; GenScript, Piscataway, NJ) and CD19-CAR-T cells were incubated with Recombinant Human CD19 Fc Chimera Alexa Fluor® 647 (1:500; Bio-Techne, Minneapolis, MN) at room temperature (RT) for 30 min. Samples were measured on a FACSCanto™ II Flow Cytometry System (BD, Franklin Lakes, NJ). 24 h before experiments, T cells were transferred into medium without IL-2. For long-term storage, CAR-T cells were cryopreserved in liquid nitrogen with 90% FCS, 10% dimethyl sulfoxide as freezing medium.

Fluorescence-activated cell sorting (FACS)

T cells transduced with CB2-CD28-CAR_GFP were stained with MonoRab™ Rabbit Anti-Camelid VHH Cocktail iFluor 647 at 4°C for 30 min. GFP control T cells were resuspended in PBS. Subsequently, cells were sorted for nanobody-positive or GFP-positive cells respectively, using a FACSMelody™ Cell Sorter (BD, Franklin Lakes, NJ). Sorting efficiency was determined afterwards using the same staining pattern (Figure 9).

Immunophenotyping of CAR-T cells (CD4/CD8 ratio)

The CD4/CD8 ratio of non-transduced and CB2-CAR-T cells was determined via flow cytometry. T cells were stained with MonoRab™ Rabbit Anti-Camelid VHH Cocktail iFluor 647, PE anti-human CD4 Antibody (1:100; BioLegend, San Diego, CA), and FITC anti-human CD8 Antibody (1:100; BioLegend, San Diego, CA) at RT for 30 min. Each condition was prepared in triplicates. Samples were measured on a FACSCanto™ II and events were gated for single cells and, in the case of CB2-CAR-T cells, also for CAR-positive cells.

Apoptosis assays

Non-transduced, sorted GFP control and sorted CB2-CAR-T cells were co-cultivated with SC-1 cells in different Effector to Target (E:T) ratios (1:1, 3:1, 5:1 and 9:1). Each condition was prepared in triplicates. SC-1 cell numbers were constant ($5\times10^3$ cells/well). Co-cultivation took place in a 96-well plate for 72 h at 37°C. For analysis, cells were stained with anti-CD3-APC antibody (1:200; BioLegend, San Diego, CA) to distinguish B cell lymphoma (BCL) and T cells, Annexin V-PE (1:100; BioLegend, San Diego, CA) to stain early apoptotic cells and 7-AAD Viability Staining Solution (1:100; ThermoFisher Scientific, Waltham, MA) to stain late apoptotic cells. Incubation took place in Annexin V Binding Buffer (BioLegend, San Diego, CA) at RT for 15 min. Cells from each well were analyzed separately on a FACSCanto™ II. Target cells were gated for single cells and SC-1 cells (CD3-/GFP- events; Figure 10). Average frequencies of early and late apoptotic SC-1 cells were compared between the different conditions and E:T ratios.

Apoptosis assays with healthy human PBMCs were performed as described above, but PBMCs were stained with CellTrace™ Far Red Cell Proliferation Kit (ThermoFisher Scientific, Waltham, MA) prior to co-culture to distinguish them from CAR-T cells and replace the use of anti-CD3 antibody. Therefore, healthy PBMCs were gated for CellTrace™+/GFP- events.

Significance of differences was assessed via 2-way ANOVA followed by Tukey's post-hoc test.

Cytokine secretion assays

Cytokine levels were measured via enzyme-linked immunosorbent assay (ELISA). Supernatants (9:1 ratio) of the co-culture assays described above were collected and levels of IFN-$\gamma$ and TNF-o were determined in triplicates using Human IFN-$\gamma$ and Human TNF-o Mini TMB ELISA Kits (Peprotech Inc., Cranbury, NJ). Samples of T cells only were included to determine baseline cytokine secretion. Significance of differences was assessed via 2-way ANOVA followed by Tukey's post-hoc test.

Analysis of T cell activation marker expression

T cells were co-cultivated with SC-1 cells in an E:T ratio of 9:1 in triplicates as described above. Subsequently, each replicate was split into three equal fractions and stained with one of the antibodies for marker detection (PE/Cyanine7 anti-human CD25, PE/Cyanine7 anti-human CD107a (LAMP-1) or PE/Cyanine7 anti-human CD69; all BioLegend, San Diego, CA). Anti-CD3-APC antibody (BioLegend, San Diego, CA) was included in all samples to gate for T cells. Baselines of marker expression were determined with T cells only samples. Marker expression was measured on a FACSCanto™ II. Expression levels of T cells co-cultivated with SC-1 cells were subtracted with expression levels of T cells only. Significance of differences for each marker was assessed via 1-way ANOVA followed by Tukey's post-hoc test.

Cell culture

SC-1 and HEK293T cell lines were purchased from DSMZ, Braunschweig, Germany. JeKo-1 and its knockouts were kindly provided by the Seitz lab, university of Tübingen. All cell lines were maintained in a humidified incubator at 37°C and 5% $CO_2$. HEK293T cells were cultured in DMEM medium supplemented with 10% FCS, 2 mM L-glutamine, and 100 U/ml Penicillin-Streptomycin. All BCL lines were cultured in RPMI 1640 medium supplemented with 10% FCS, 2 mM L-glutamine, 1 mM pyruvate and 100 U/ml Penicillin-Streptomycin. For culture conditions of CAR-T cells, see respective section. All cell culture media and supplements were purchased from PAN-Biotech GmbH, Aidenbach, Germany. Cell cultures were tested negative for mycoplasma via PCR monthly.

JeKo-1 lymphoma knockout cell lines

JeKo-1 cells and the knockout lines JeKo-1 CD19-KO and JeKo-1 CD20-KO, all expressing firefly luciferase, were generated by the Seitz lab, university of Tübingen, as described elsewhere.[20] Knockout efficiencies were determined via flow cytometry (Figure 11) incubating with PE anti-human CD19 Antibody (1:100) and APC anti-human CD20 Antibody (1:100; both BioLegend, San Diego, CA) at RT for 1 h.

Generation of luciferase-expressing SC-1 cells

SC-1 cells expressing firefly luciferase and GFP were generated in-lab via lentiviral transduction. Lentiviral particles were produced as described above with SIN40C.SFFV.Luciferase.IRES.GFP as transfer plasmid (plasmid was a gift from Jan-Henning Klusmann, Addgene plasmid # 169308; http://n2t.net/addgene:169308; RRID:Addgene_169308).[35] One million SC-1 cells were transduced with 120 $\mu$l concentrated lentivirus and 8 $\mu$g/ml polybrene. On day 8, cells were single-cell sorted for GFP-positive cells using a FACSMelody™. Expanding clones were screened on day 23 for uniform GFP expression. One clone was selected, further expanded and cryo-preserved. The SC-1 origin was confirmed via flow cytometry with PE anti-human CD19 Antibody (1:100; BioLegend, San Diego, CA), confirming uniform CD19 and GFP expression of the clone (Figure 12).

Luciferase-based cytotoxicity assays

Non-transduced and CAR-T cells were co-cultivated with BCL in different E:T ratios (1:1, 3:1, 5:1 and 9:1). Each condition was prepared in triplicates. BCL cell numbers were constant ($5 \times 10^3$ cells/well). BCL only samples were included for lysis control. Differences in CAR-T cell transduction efficiencies were considered by adjusting T cell numbers to reach the same amounts of CAR-positive cells among all constructs. Co-cultivation took place in a 96-well plate at 37°C for 24 h (SC-1) or 48 h (all other BCL). For analysis, 100 μg/ml D-luciferin potassium salt (Abcam, Cambridge, UK) was added to each well. For lysis control wells, 1% NP-40 was added. Cells were incubated at 37°C for 1 h. Afterwards, relative light units (RLU) were measured in a CLARIOstar Plus plate reader (BMG LABTECH GmbH, Ortenberg, Germany) with an exposure time of 10 seconds per well. Normalized specific lysis (Normalized spec. lysis) was calculated by normalizing test RLUs to target cells co-cultured with non-transduced T cells (NT) at the respective E:T ratio (background cytotoxicity) and lysis control wells (maximal killing) using the following equation:

$$\text{Normalized spec. lysis } [\%] = 100 \frac{RLU_{NT} - RLU_{test}}{RLU_{NT} - RLU_{lysis\ control}}$$

If indicated in the figure legend, data from independent experiments was pooled by calculating the grand mean (mean of means) and pooled standard error ($SEM_p$) via the following equation:

$$SEM_p = \sqrt{\frac{\sum_{i=1}^{k}(n_i - 1)s_i^2}{\sum_{i=1}^{k}(n_i - 1)} \sum_{i=1}^{k} \frac{1}{n_i}}$$

Where $n_i$ is the sample size of the i-th independent experiment; $s_i^2$ is the variance of the i-th independent experiment; and k is the number of independent experiments. Significance of differences was assessed via 2-way ANOVA followed by Tukey's post-hoc test.

Recombinant expression of nanobodies in *Escherichia coli*

Histidine-tagged LaG-16 expression plasmid "pET21-pelB-LaG-16" was a gift from Michael Rout (Addgene plasmid #172746; http://n2t.net/addgene:172746; RRID:Addgene_172746).[33] The S108C mutation was introduced by overlap-extension PCR as described elsewhere.[32] Both plasmids were transformed into ArcticExpress (DE3) *Escherichia coli* cells (Agilent Technologies, Santa Clara, CA), and protein expression was induced in lysogenic broth medium with 1 mM isopropyl-β-D-1-thiogalactopyranoside at 12°C overnight. Bacteria were lysed with 5 freeze-thaw cycles, alternating between a 37°C water bath and dry ice. Nanobody was then purified in two steps, first by Nickel-NTA agarose resin (ThermoFisher Scientific, Waltham, MA) followed by size exclusion chromatography on an ÄKTApurifier using an S200 column (Cytiva, Marlborough, MA). All steps and purity of the final product were validated by sodium dodecyl sulfate poly-acrylamide gel electrophoresis (SDS-PAGE, Figure 13). CB2 and [C1055]CB2 were expressed and purified as described elsewhere.[25]

Flow cytometry nanobody binding assays

One million SC-1 cells per sample were incubated with 24 μM nanobody solution or PBS (negative control) at RT for 1 h. Afterwards, all samples were incubated with MonoRab™ Rabbit Anti-Camelid VHH Cocktail iFluor 647 at RT for 1 h. Samples were measured on a FACSCanto™ II.

*In vivo* experiments

Immunodeficient mice (n=3 per group) were xenografted with human lymphoma cell lines JeKo-1 and JeKo-1 CD19-KO in a 1:1 mixture at $0.5 \times 10^6$ cells each per mouse on day -6. Both cell lines are expressing GFP and firefly luciferase. On day 0, CAR-T cells were injected. Groups tested were tumor only, mock T cells, CD19-CAR, and [S229C]CD19-CAR. Tumor burden was measured via the luciferase signal on days -1, 1, 4, 6, 8, 10, 13, 15, 17, 21, 23, and 25.

**Table of Sequences**

| SEQ ID NO | Type | Description | Sequence |
|---|---|---|---|
| 1 | AA | anti-CD19 scFv FMC63 | DIQMTQTTSSLSASLGDRVTISCRASQDISKYLNWYQQKPDGTV KLLIYHTSRLHSGVPSRFSGSGSGTDYSLTISNLEQEDIATYFCQQ GNTLPYTFGGGTKLEITGSTSGSGKPGSGEGSTKGEVKLQESGPG LVAPSQSLSVTCTVSGVSLPDYGVSWIRQPPRKGLEWLGVIWGS ETTYYNSALKSRLTIIKDNSKSQVFLKMNSLQTDDTAIYYCAKHY YYGGSYAMDYWGQGTSVTVSS |
| 2 | AA | anti-CD19 scFv FMC63 S229C | DIQMTQTTSSLSASLGDRVTISCRASQDISKYLNWYQQKPDGTV KLLIYHTSRLHSGVPSRFSGSGSGTDYSLTISNLEQEDIATYFCQQ GNTLPYTFGGGTKLEITGSTSGSGKPGSGEGSTKGEVKLQESGPG LVAPSQSLSVTCTVSGVSLPDYGVSWIRQPPRKGLEWLGVIWGS ETTYYNSALKSRLTIIKDNSKSQVFLKMNSLQTDDTAIYYCAKHY YYGGCYAMDYWGQGTSVTVSS |
| 3 | AA | anti-CD19 scFv FMC63 G228C | DIQMTQTTSSLSASLGDRVTISCRASQDISKYLNWYQQKPDGTV KLLIYHTSRLHSGVPSRFSGSGSGTDYSLTISNLEQEDIATYFCQQ GNTLPYTFGGGTKLEITGSTSGSGKPGSGEGSTKGEVKLQESGPG LVAPSQSLSVTCTVSGVSLPDYGVSWIRQPPRKGLEWLGVIWGS ETTYYNSALKSRLTIIKDNSKSQVFLKMNSLQTDDTAIYYCAKHY YYGCSYAMDYWGQGTSVTVSS |
| 4 | AA | anti-CD19 scFv FMC63 N92C | DIQMTQTTSSLSASLGDRVTISCRASQDISKYLNWYQQKPDGTV KLLIYHTSRLHSGVPSRFSGSGSGTDYSLTISNLEQEDIATYFCQQ GCTLPYTFGGGTKLEITGSTSGSGKPGSGEGSTKGEVKLQESGPG LVAPSQSLSVTCTVSGVSLPDYGVSWIRQPPRKGLEWLGVIWGS ETTYYNSALKSRLTIIKDNSKSQVFLKMNSLQTDDTAIYYCAKHY YYGGSYAMDYWGQGTSVTVSS |
| 5 | AA | CB2 | GHVQLVESGGGLVQPGGSLRLSCAASGFTFSNYRMYWIRQAPGK GLEWVSTISSDGVATYYADSVKGRFTISRDNAKNTLYLQMNRLK LEDTAVYSCAALNRYCGNEYEYDYRGQGTQVTVSS |
| 6 | AA | LAG16 WT | QVQLVESGGRLVQAGDSLRLSCAASGRTFSTSAMAWFRQAPGR EREFVAAITWTVGNTILGDSVKGRFTISRDRAKNTVDLQMDNLE PEDTAVYYCSARSRGYVLSVLRSVDSYDYWGQGTQVTVSS |
| 7 | AA | CD8 hinge | TTTPAPRPPTPAPTIASQPLSLRPEACRPAAGGAVHTRGLDFACD |

(continued)

| SEQ ID NO | Type | Description | Sequence |
|---|---|---|---|
| 8 | AA | IgG Fc hinge | EPKSCDKTHTCPPCPAPELLGGPSVFLFPPKPKDTLMISRTPEVTC VVVDVSHEDPEVKFNWYVDGVEVHNAKTKPREEQYNSTYRVVS VLTVLHQDWLNGKEYKCKVSNKALPAPIEKTISKAKGQPREPQVY TLPPSRDELTKNQVSLTCLVKGFYPSDIAVEWESNGQPENNYKTT PPVLDSDGSFFLYSKLTVDKSRWQQGNVFSCSVMHEALHNHYT QKSLSLSPVD |
| 9 | AA | CD8 TM | IYIWAPLAGTCGVLLLSLVITLYC |
| 10 | AA | CD28 TM | FWVLVVVGGVLACYSLLVTVAFIIFWV |
| 11 | AA | CD28 CD | GSRSKRSRLLHSDYMNMTPRRPGPTRKHYQPYAPPRDFAAYRS |
| 12 | AA | 4-1BB CD | KRGRKKLLYIFKQPFMRPVQTTQEEDGCSCRFPEEEEGGCEL |
| 13 | AA | CD3 ζ AD | RVKFSRSADAPAYQQGQNQLYNELNLGRREEYDVLDKRRGRDP EMGGKPRRKNPQEGLYNELQKDKMAEAYSEIGMKGERRRGKGH DGLYQGLSTATKDTYDALHMQALPPR |
| 14 | AA | CD8 SP | MALPVTALLLPLALLLHAARP |
| 15 | AA | GM-CSF | MLLLVTSLLLCELPHPAFLLIP |
| 16 | DNA | anti-CD19 scFv FMC63 | gacatccagatgacccagaccacaagcagcctgtctgccagcctgggcgataga gtgaccatcagctgtagagccagccaggacatcagcaagtacctgaactggtatc agcaaaagcccgacggcaccgtgaagctgctgatctaccacaccagcagactgc acagcggcgtgccaagcagattttctggcagcggctctggcaccgactacagcct gacaatcagcaacctggaacaagaggatatcgctacctacttctgccagcaaggc aacaccctgccttacacctttggcggaggcaccaagctggaaatcaccggctctac aagcggcagcggcaaacctggatctggcgagggatctaccaagggcgaagtga aactgcaagagtctggccctggactggtggccccatctcagtctctgagcgtgacc tgtacagtcagcggagtgtccctgcctgattacggcgtgtcctggatcagacagcct cctcggaaaggcctggaatggctgggagtgatctggggcagcgagacaacctac tacaacagcgccctgaagtcccggctgaccatcatcaaggacaactccaagagcc aggtgttcctgaagatgaacagcctgcagaccgacgacaccgccatctactattgc gccaagcactactactacggcggcagctacgccatggattattggggccagggca ccagcgtgacagtttcttca |

(continued)

| | SEQ ID NO | Type | Description | Sequence |
|---|---|---|---|---|
| | 17 | DNA | CB2 | GGCCACGTGCAGCTGGTGGAGAGCGGCGGCGGCCTGGTGCAGCCCGGCGGCAGCCTGCGCCTGAGCTGCGCCGCCAGCGGCTTCACCTTCAGCAACTACCGCATGTACTGGATACGCCAGGCCCCCGGCAAGGGCCTGGAGTGGGTGAGCACCATCAGCAGCGACGGCGTGGCCACCTACTACGCCGACAGCGTGAAGGGCCGCTTCACCATCAGCCGCGACAACGCCAAGAACACCCTGTACCTGCAGATGAACCGCCTGAAGCTGGAGGACACCGCCGTGTACAGCTGCGCCGCCCTGAACCGCTACTGCGGCAACGAGTACGAGTACGACTACCGCGGCCAGGGCACCCAGGTGACCGTGAGCAGC |
| | 18 | DNA | LAG16 | CAAGTTCAACTGGTGGAAAGTGGCGGGAGACTTGTGCAGGCCGGCGATAGCCTGCGGCTCTCATGTGCAGCCTCTGGCAGAACTTTCTCCACGTCAGCCATGGCCTGGTTTAGACAAGCTCCAGGAAGGGAGCGGGAATTTGTGGCCGCGATTACCTGGACTGTCGGGAACACTATCCTGGGAGATTCAGTTAAGGGCCGCTTTACCATATCCCGGGATCGGGCTAAGAATACCGTGGACCTCCAGATGGACAACCTTGAGCCTGAAGACACCGCAGTCTACTACTGTGCTGTCCGTGCTGCGGAGCCGCGGCTACGTACTTTGCGTGCTGCGCTCTGTGGACAGCTACGATTACTGGGGACAGGGCACACAAGTGACAGTCAGCTCT |
| | 19 | DNA | CD8 hinge | acaacaacaccagctcctcggcctccaactcctgctcctacaattgcctctcagcctctgtctctgaggcccgaagcttgtagacctgctgctggcggagctgtgcacaccagaggactggatttcgcctgcgac |

(continued)

| SEQ ID NO | Type | Description | Sequence |
|---|---|---|---|
| 20 | DNA | IgG Fc hinge | GAGCCCAAATCTTGTGACAAAACTCACACATGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATCCCGGGATGAGCTGACCAAGAACCAGGTCAGCCTGACCTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACACAGAAGAGCCTCTCCCTGTCTCCGGTCGAC |
| 21 | DNA | CD8 TM | atctacatttgggcccctctggctggaacatgcggagttctgctgctgagcctggtcatcaccctgtactgc |
| 22 | DNA | CD28 TM | TTTTGGGTGCTGGTGGTGGTTGGTGGAGTCCTGGCTTGCTATAGCTTGCTAGTAACAGTGGCCTTTATTATTTTCTGGGTG |
| 23 | DNA | CD28 CD | GGATCCAGGAGTAAGAGGAGCAGGCTCCTGCACAGTGACTACATGAACATGACTCCCCGCCGCCCCGGGCCCACCCGCAAGCATTACCAGCCCTATGCCCCACCACGCGACTTCGCAGCCTATCGCTCC |
| 24 | DNA | 4-1BB CD | aagcggggcagaaagaagctgctgtacatcttcaagcagcccttcatgcggcccgtgcagaccacacaagaggaagatggctgctcctgcagattccccgaggaagaagaaggcggctgcgagctg |
| 25 | DNA | CD3 ζ AD | agagtgaagttctccagatctgccgacgctcctgcctaccagcagggccagaatcagctgtacaacgagctgaacctggggagaagagaagagtacgacgtgctggataagcggagaggcagagatcctgagatgggcggcaagcccagacggaagaatcctcaagagggcctgtataatgagctgcagaaagacaagatggccgaggcctacagcgagatcggaatgaagggcgagcgcagaagaggcaagggccacgatggactgtatcagggcctgagcacagccaccaaggatacctatgatgccctgcacatgcagGCCCTGCCTCCAAGA |

**EP 4 706 675 A1**

(continued)

| SEQ ID NO | Type | Description | Sequence |
|---|---|---|---|
| 26 | DNA | CD8 SP | atggccttaccagtgaccgccttgctcctgccgctggccttgctgctccacgccgcc aggccg |
| 27 | DNA | GM-CSF | atgctgctgctggtcacatctctgctgctgtgcgagctgccccatcctgcctttctgct gatcccc |
| 28 | RNA | anti-CD19 scFv FMC63 WT | gacatccagatgacccagaccacaagcagcctgtctgccagcctgggcgataga gtgaccatcagctgtagagccagccaggacatcagcaagtacctgaactggtatc agcaaaagcccgacggcaccgtgaagctgctgatctaccacaccagcagactgc acagcggcgtgccaagcagattttctggcagcggctctggcaccgactacagcct gacaatcagcaacctggaacaagaggatatcgctacctacttctgccagcaaggc aacaccctgccttacacctttggcggaggcaccaagctggaaatcaccggctctac aagcggcagcggcaaacctggatctggcgagggatctaccaagggcgaagtga aactgcaagagtctggccctggactggtggccccatctcagtctctgagcgtgacc tgtacagtcagcggagtgtccctgcctgattacggcgtgtcctggatcagacagcct cctcggaaaggcctggaatggctgggagtgatctggggcagcgagacaacctac tacaacagcgccctgaagtcccggctgaccatcatcaaggacaactccaagagcc aggtgttcctgaagatgaacagcctgcagaccgacgacaccgccatctactattgc gccaagcactactactacggcggcagctacgccatggattattggggccagggca ccagcgtgacagtttcttca |
| 29 | RNA | CB2 | GGCCACGTGCAGCTGGTGGAGAGCGGCGGCGGCCTGGTGCAG CCCGGCGGCAGCCTGCGCCTGAGCTGCGCCGCCAGCGGCTTC ACCTTCAGCAACTACCGCATGTACTGGATACGCCAGGCCCCCG GCAAGGGCCTGGAGTGGGTGAGCACCATCAGCAGCGACGGCG TGGCCACCTACTACGCCGACAGCGTGAAGGGCCGCTTCACCAT CAGCCGCGACAACGCCAAGAACACCCTGTACCTGCAGATGAAC CGCCTGAAGCTGGAGGACACCGCCGTGTACAGCTGCGCCGCCC TGAACCGCTACTGCGGCAACGAGTACGAGTACGACTACCGCGG CCAGGGCACCCAGGTGACCGTGAGCAGC |
| 30 | RNA | LAG16 WT | CAAGTTCAACTGGTGGAAAGTGGCGGGAGACTTGTGCAGGCC GGCGATAGCCTGCGGCTCTCATGTGCAGCCTCTGGCAGAACTT TCTCCACGTCAGCCATGGCCTGGTTTAGACAAGCTCCAGGAAG GGAGCGGGAATTTGTGGCCGCGATTACCTGGACTGTCGGGAA |

42

(continued)

| SEQ ID NO | Type | Description | Sequence |
|---|---|---|---|
| | | | CACTATCCTGGGAGATTCAGTTAAGGGCCGCTTTACCATATCC CGGGATCGGGCTAAGAATACCGTGGACCTCCAGATGGACAACC TTGAGCCTGAAGACACCGCAGTCTACTACTGTTCTGCTCGGAG CCGCGGCTACGTACTTAGTGTGCTGCGCTCTGTGGACAGCTAC GATTACTGGGGACAGGGCACACAAGTGACAGTCAGCTCT |
| 31 | RNA | CD8 hinge | ACAACAACACCAGCTCCTCGGCCTCCAACTCCTGCTCCTACAAT TGCCTCTCAGCCTCTGTCTCTGAGGCCCGAAGCTTGTAGACCT GCTGCTGGCGGAGCTGTGCACACCAGAGGACTGGATTTCGCCT GCGAC |
| 32 | RNA | IgG Fc hinge | GAGCCCAAATCTTGTGACAAAACTCACACATGCCCACCGTGCC CAGCACCTGAACTCCTGGGGGGACCGTCAGTCTTCCTCTTCCC CCCAAAACCCAAGGACACCCTCATGATCTCCCGGACCCCTGAG GTCACATGCGTGGTGGTGGACGTGAGCCACGAAGACCCTGAG GTCAAGTTCAACTGGTACGTGGACGGCGTGGAGGTGCATAATG CCAAGACAAAGCCGCGGGAGGAGCAGTACAACAGCACGTACCG TGTGGTCAGCGTCCTCACCGTCCTGCACCAGGACTGGCTGAAT GGCAAGGAGTACAAGTGCAAGGTCTCCAACAAAGCCCTCCCAG CCCCCATCGAGAAAACCATCTCCAAAGCCAAAGGGCAGCCCCG AGAACCACAGGTGTACACCCTGCCCCCATCCCGGGATGAGCTG ACCAAGAACCAGGTCAGCCTGACCTGCCTGGTCAAAGGCTTCT ATCCCAGCGACATCGCCGTGGAGTGGGAGAGCAATGGGCAGC CGGAGAACAACTACAAGACCACGCCTCCCGTGCTGGACTCCGA CGGCTCCTTCTTCCTCTACAGCAAGCTCACCGTGGACAAGAGC AGGTGGCAGCAGGGGAACGTCTTCTCATGCTCCGTGATGCATG AGGCTCTGCACAACCACTACACACAGAAGAGCCTCTCCCTGTC TCCGGTCGAC |
| 33 | RNA | CD8 TM | ATCTACATTTGGGCCCCTCTGGCTGGAACATGCGGAGTTCTGC TGCTGAGCCTGGTCATCACCCTGTACTGC |
| 34 | RNA | CD28 TM | TTTTGGGTGCTGGTGGTGGTTGGTGGAGTCCTGGCTTGCTAT AGCTTGCTAGTAACAGTGGCCTTTATTATTTTCTGGGTG |
| 35 | RNA | CD28 CD | GGATCCAGGAGTAAGAGGAGCAGGCTCCTGCACAGTGACTACA TGAACATGACTCCCCGCCGCCCCGGGCCCACCCGCAAGCATTA CCAGCCCTATGCCCCACCACGCGACTTCGCAGCCTATCGCTCC |

(continued)

| SEQ ID NO | Type | Description | Sequence |
|---|---|---|---|
| 36 | RNA | 4-1BB CD | AAGCGGGGCAGAAAGAAGCTGCTGTACATCTTCAAGCAGCCCTTCATGCGGCCCGTGCAGACCACACAAGAGGAAGATGGCTGCTCCTGCAGATTCCCCGAGGAAGAAGAAGGCGGCTGCGAGCTG |
| 37 | RNA | CD3 ζ AD | AGAGTGAAGTTCTCCAGATCTGCCGACGCTCCTGCCTACCAGCAGGGCCAGAATCAGCTGTACAACGAGCTGAACCTGGGGAGAAGAGAAGAGTACGACGTGCTGGATAAGCGGAGAGGCAGAGATCCTGAGATGGGCGGCAAGCCCAGACGGAAGAATCCTCAAGAGGGCCTGTATAATGAGCTGCAGAAAGACAAGATGGCCGAGGCCTACAGCGAGATCGGAATGAAGGGCGAGCGCAGAAGAGGCAAGGGCCACGATGGACTGTATCAGGGCCTGAGCACAGCCACCAAGGATACCTATGATGCCCTGCACATGCAGGCCCTGCCTCCAAGA |
| 38 | RNA | CD8 SP | ATGGCCTTACCAGTGACCGCCTTGCTCCTGCCGCTGGCCTTGCTGCTCCACGCCGCCAGGCCG |
| 39 | RNA | GM-CSF SP | ATGCTGCTGCTGGTCACATCTCTGCTGCTGTGCGAGCTGCCCCATCCTGCCTTTCTGCTGATCCCC |
| 40 | AA | anti-GD2 scFv 14G2a | EVQLLQSGPELEKPGASVMISCKASGSSFTGYNMNWVRQNIGKSLEWIGAIDPYYGGTSYNQKFKGRATLTVDKSSSTAYMHLKSLTSEDSAVYYCVSGMEYWGQGTSVTVSSGGGGSGGGGSGGGGSEIVMTQSPATLSVSPGERATLSCRSSQSLVHRNGNTYLHWYLQKPGQSPKLLIHKVSNRFSGVPDRFSGSGSGTDFTLKISRVEAEDLGVYFCSQSTHVPPLTFGAGTKLELKR |
| 41 | AA | anti-GD2 scFv 14G2a M100C | EVQLLQSGPELEKPGASVMISCKASGSSFTGYNMNWVRQNIGKSLEWIGAIDPYYGGTSYNQKFKGRATLTVDKSSSTAYMHLKSLTSEDSAVYYCVSGCEYWGQGTSVTVSSGGGGSGGGGSGGGGSEIVMTQSPATLSVSPGERATLSCRSSQSLVHRNGNTYLHWYLQKPGQSPKLLIHKVSNRFSGVPDRFSGSGSGTDFTLKISRVEAEDLGVYFCSQSTHVPPLTFGAGTKLELKR |
| 42 | AA | anti-GD2 scFv 14G2a H226C | EVQLLQSGPELEKPGASVMISCKASGSSFTGYNMNWVRQNIGKSLEWIGAIDPYYGGTSYNQKFKGRATLTVDKSSSTAYMHLKSLTSEDSAVYYCVSGMEYWGQGTSVTVSSGGGGSGGGGSGGGGSEIVMTQSPATLSVSPGERATLSCRSSQSLVHRNGNTYLHWYLQKPGQSPKLLIHKVSNRFSGVPDRFSGSGSGTDFTLKISRVEAEDLGVYFCSQSTCVPPLTFGAGTKLELKR |

(continued)

| SEQ ID NO | Type | Description | Sequence |
|---|---|---|---|
| 43 | AA | anti-GD2 scFv 14G2a V227C | EVQLLQSGPELEKPGASVMISCKASGSSFTGYNMNWVRQNIGKS LEWIGAIDPYYGGTSYNQKFKGRATLTVDKSSSTAYMHLKSLTSE DSAVYYCVSGMEYWGQGTSVTVSSGGGGSGGGGSGGGGSEIV MTQSPATLSVSPGERATLSCRSSQSLVHRNGNTYLHWYLQKPG QSPKLLIHKVSNRFSGVPDRFSGSGSGTDFTLKISRVEAEDLGVY FCSQSTHCPPLTFGAGTKLELKR |
| 44 | DNA | anti-GD2 scFv 14G2a | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACGTGCCACC TCTGACCTTTGGCGCTGGCACAAAGCTGGAACTGAAGCGG |

(continued)

| SEQ ID NO | Type | Description | Sequence |
|---|---|---|---|
| 45 | DNA | anti-GD2 scFv 14G2a M100C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCTGTG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACGTGCCACC TCTGACCTTTGGCGCTGGCACAAAGCTGGAACTGAAGCGG |
| 46 | DNA | anti-GD2 scFv 14G2a H226C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACATGTGTGCCACC TCTGACCTTTGGCGCTGGCACAAAGCTGGAACTGAAGCGG |

(continued)

| SEQ ID NO | Type | Description | Sequence |
|---|---|---|---|
| 47 | DNA | anti-GD2 scFv 14G2a V227C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACTGTCCACC TCTGACCTTTGGCGCTGGCACAAAGCTGGAACTGAAGCGG |
| 48 | RNA | anti-GD2 scFv 14G2a | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACTGTCCACC TCTGACCTTTGGCGCTGGCACAAAGCTGGAACTGAAGCGG |

(continued)

| SEQ ID NO | Type | Description | Sequence |
|---|---|---|---|
| 49 | RNA | anti-GD2 scFv 14G2a M100C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCTGTG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACGTGCCACC TCTGACCTTTGGCGCTGGCACAAAGCTGGAACTGAAGCGG |
| 50 | RNA | anti-GD2 scFv 14G2a H226C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACATGTGTGCCACC TCTGACCTTTGGCGCTGGCACAAAGCTGGAACTGAAGCGG |

(continued)

| SEQ ID NO | Type | Description | Sequence |
|---|---|---|---|
| 51 | RNA | anti-GD2 scFv 14G2a V227C | GCTGCAGTCTGGCCCTGAACTGGAAAAACCTGGCGCCTCCGTGATGATCAGCTGTAAAGCCAGCGGCAGCAGCTTCACCGGCTACAACATGAACTGGGTCCGACAGAACATCGGCAAGAGCCTGGAATGGATCGGCGCCATCGATCCTTACTACGGCGGCACCAGCTACAACCAGAAGTTCAAGGGCAGAGCCACACTGACCGTGGACAAGAGCAGCAGCACAGCCTACATGCACCTGAAGTCCCTGACAAGCGAGGACAGCGCCGTGTACTACTGTGTGTCCGGCATGGAATATTGGGGCCAGGGCACAAGCGTGACAGTTTCTTCTGGTGGCGGAGGATCTGGCGGAGGTGGAAGCGGCGGAGGCGGATCTGAAATCGTGATGACACAGAGCCCCGCCACTCTGAGTGTGTCTCCAGGCGAAAGAGCTACCCTGAGCTGTAGAAGCAGCCAGAGCCTGGTGCACAGAAACGGCAATACCTACCTGCACTGGTATCTGCAGAAGCCCGGCCAGTCTCCTAAGCTGCTGATCCACAAGGTGTCCAACAGATTCAGCGGCGTGCCCGATAGATTTTCTGGCTCTGGCAGCGGCACCGACTTCACCCTGAAGATTAGCAGAGTGGAAGCCGAGGACCTGGGCGTGTACTTCTGTAGCCAGTCTACACACTGTCCACCTCTGACCTTTGGCGCTGGCACAAAGCTGGAACTGAAGCGG |
| 52 | DNA | EQ_CAR_FW, primer for sequencing and cloning of CAR constructs | TTCAAAGTTTTTTTCTTCCATTTCAGG |
| 53 | DNA | SEQ CAR_RV, primer for sequencing and cloning of CAR constructs | ACAAATTTTGTAATCCAGAGGTTGATT |
| 54 | DNA | AMP_CAR_KpnI_RV, for cloning of shorter CAR construct (GFP deletion) | TTATTAGGTACCGCGGGGAGGC |
| 55 | DNA | C105S_FW, for cloning of CB2 mutant C105S | CGCTACAGCGGCAAC |
| 56 | DNA | C105S_RV, for cloning of CB2 mutant C105S | GTTGCCGCTGTAGCG |
| 57 | DNA | 5' LTR (truncated) | GGGTCTCTCTGGTTAGACCAGATCTGAGCCTGGGAGCTCTCTGGCTAACTAGGGAACCCACTGCTTAAGCCTCAATAAAGCTTGCCTTGAGTGCTTCAAGTAGTGTGTGCCCGTCTGTTGTGTGACTCTGGTAACTAGAGATCCCTCAGACCCTTTTAGTCAGTGTGGAAAATCTCTAGCA |

(continued)

| SEQ ID NO | Type | Description | Sequence |
|---|---|---|---|
| 58 | DNA | 3' LTR (ΔU3) | TGGAAGGGCTAATTCACTCCCAACGAAGACAAGATCTGCTTTT TGCTTGTACTGGGTCTCTCTGGTTAGACCAGATCTGAGCCTGG GAGCTCTCTGGCTAACTAGGGAACCCACTGCTTAAGCCTCAAT AAAGCTTGCCTTGAGTGCTTCAAGTAGTGTGTGCCCGTCTGTT GTGTGACTCTGGTAACTAGAGATCCCTCAGACCCTTTTAGTCA GTGTGGAAAATCTCTAGCA |
| 59 | DNA | EF-1o promoter | GGCTCCGGTGCCCGTCAGTGGGCAGAGCGCACATCGCCCACA GTCCCCGAGAAGTTGGGGGGAGGGGTCGGCAATTGAACCGGT GCCTAGAGAAGGTGGCGCGGGGTAAACTGGGAAAGTGATGTC GTGTACTGGCTCCGCCTTTTTCCCGAGGGTGGGGGAGAACCGT ATATAAGTGCAGTAGTCGCCGTGAACGTTCTTTTTCGCAACGG GTTTGCCGCCAGAACACAGGTAAGTGCCGTGTGTGGTTCCCGC GGGCCTGGCCTCTTTACGGGTTATGGCCCTTGCGTGCCTTGAA TTACTTCCACCTGGCTGCAGTACGTGATTCTTGATCCCGAGCT TCGGGTTGGAAGTGGGTGGGAGAGTTCGAGGCCTTGCGCTTA AGGAGCCCCTTCGCCTCGTGCTTGAGTTGAGGCCTGGCCTGG GCGCTGGGGCCGCCGCGTGCGAATCTGGTGGCACCTTCGCGC CTGTCTCGCTGCTTTCGATAAGTCTCTAGCCATTTAAAATTTTT GATGACCTGCTGCGACGCTTTTTTTCTGGCAAGATAGTCTTGT AAATGCGGGCCAAGATCTGCACACTGGTATTTCGGTTTTTTGGG GCCGCGGGCGGCGACGGGGCCCGTGCGTCCCAGCGCACATGT TCGGCGAGGCGGGGCCTGCGAGCGCGGCCACCGAGAATCGGA CGGGGGTAGTCTCAAGCTGGCCGGCCTGCTCTGGTGCCTGGT CTCGCGCCGCCGTGTATCGCCCCGCCCTGGGCGGCAAGGCTG GCCCGGTCGGCACCAGTTGCGTGAGCGGAAAGATGGCCGCTT CCCGGCCCTGCTGCAGGGAGCTCAAAATGGAGGACGCGGCGC TCGGGAGAGCGGGCGGGTGAGTCACCCACACAAAGGAAAAGG GCCTTTCCGTCCTCAGCCGTCGCTTCATGTGACTCCACGGAGT ACCGGGCGCCGTCCAGGCACCTCGATTAGTTCTCGAGCTTTTG GAGTACGTCGTCTTTAGGTTGGGGGGAGGGGTTTTATGCGAT GGAGTTTCCCCACACTGAGTGGGTGGAGACTGAAGTTAGGCCA GCTTGGCACTTGATGTAATTCTCCTTGGAATTTGCCCTTTTTGA GTTTGGATCTTGGTTCATTCTCAAGCCTCAGACAGTGGTTCAA AGTTTTTTTTCTTCCATTTCAGGTGTCGTGA |

(continued)

| SEQ ID NO | Type | Description | Sequence |
|---|---|---|---|
| 60 | DNA | CMV promoter | GTGATGCGGTTTTGGCAGTACATCAATGGGCGTGGATAGCGG TTTGACTCACGGGGATTTCCAAGTCTCCACCCCATTGACGTCA ATGGGAGTTTGTTTTGGCACCAAAATCAACGGGACTTTCCAAA ATGTCGTAACAACTCCGCCCCATTGACGCAAATGGGCGGTAGG CGTGTACGGTGGGAGGTCTATATAAGCAGAGCT |
| 61 | DNA | WPRE | AATCAACCTCTGGATTACAAAATTTGTGAAAGATTGACTGGTAT TCTTAACTATGTTGCTCCTTTTACGCTATGTGGATACGCTGCTT TAATGCCTTTGTATCATGCTATTGCTTCCCGTATGGCTTTCATT TTCTCCTCCTTGTATAAATCCTGGTTGCTGTCTCTTTATGAGGA GTTGTGGCCCGTTGTCAGGCAACGTGGCGTGGTGTGCACTGT GTTTGCTGACGCAACCCCCACTGGTTGGGGCATTGCCACCACC TGTCAGCTCCTTTCCGGGACTTTCGCTTTCCCCCCTCCCTATTGC CACGGCGGAACTCATCGCCGCCTGCCTTGCCCGCTGCTGGACA GGGGCTCGGCTGTTGGGCACTGACAATTCCGTGGTGTTGTCG GGGAAGCTGACGTCCTTTCCATGGCTGCTCGCCTGTGTTGCCA CCTGGATTCTGCGCGGGACGTCCTTCTGCTACGTCCCTTCGGC CCTCAATCCAGCGGACCTTCCTTCCCGCGGCCTGCTGCCGGCT CTGCGGCCTCTTCCGCGTCTTCGCCTTCGCCCTCAGACGAGTC GGATCTCCCTTTGGGCCGCCTCCCCGC |

The sequences in the Table of Sequences are presented according to WIPO Standard ST.26, which lists RNA sequences with the nucleotide T instead of U. However, these RNA sequences should be interpreted and utilized as containing the U nucleotide at T positions.

**References**

1. Wu L, Wei Q, Brzostek J, Gascoigne NRJ. Signaling from T cell receptors (TCRs) and chimeric antigen receptors (CARs) on T cells. Cell Mol Immunol. 2020;17(6):600-612.

2. O'Leary MC, Lu X, Huang Y, et al. FDA Approval summary: Tisagenlecleucel for treatment of patients with relapsed or refractory b-cell precursor acute lymphoblastic leukemia. Clinical Cancer Research. 2019;25(4):1142-1146.

3. Bouchkouj N, Kasamon YL, Claro RA de, et al. FDA approval summary: Axicabtagene Ciloleucel for Relapsed or Refractory Large B-cell Lymphoma. Clinical Cancer Research. 2019;25(6):1702-1708.

4. St-Pierre F, Gordon LI. Lisocabtagene maraleucel in the treatment of relapsed/refractory large B-cell lymphoma. Future Oncology. 2023;19(1):19-28.

5. Anderson LD. Idecabtagene vicleucel (ide-cel) CAR T-cell therapy for relapsed and refractory multiple myeloma. Future Oncology. 2022;18(3):277-289.

6. Chekol Abebe E, Yibeltal Shiferaw M, Tadele Admasu F, Asmamaw Dejenie T. Ciltacabtagene autoleucel: The second anti-BCMA CAR T-cell therapeutic armamentarium of relapsed or refractory multiple myeloma. Front Immunol. 2022;13:991092.

7. Frey N V. Approval of brexucabtagene autoleucel for adults with relapsed and refractory acute lymphocytic leukemia. Blood. 2022;140(1):11-15.

8. Sadelain M. CAR therapy: The CD19 paradigm. Journal of Clinical Investigation. 2015;125(9):3392-3400.

9. Abreu TR, Fonseca NA, Gonçalves N, Moreira JN. Current challenges and emerging opportunities of CAR-T cell therapies. Journal of Controlled Release. 2020;319:246-261.

10. Frey N, Porter D. Cytokine Release Syndrome with Chimeric Antigen Receptor T Cell Therapy. Biology of Blood and Marrow Transplantation. 2019;25(4):e123-e127.

11. Gust J, Hay KA, Hanafi LA, et al. Endothelial activation and blood-brain barrier disruption in neurotoxicity after adoptive immunotherapy with CD19 CAR-T cells. Cancer Discov. 2017;7(12):1404-1419.

12. Orlando EJ, Han X, Tribouley C, et al. Genetic mechanisms of target antigen loss in CAR19 therapy of acute lymphoblastic leukemia. Nat Med. 2018;24(10):1504-1506.

13. Sotillo E, Barrett DM, Black KL, et al. Convergence of acquired mutations and alternative splicing of CD19 enables resistance to CART-19 immunotherapy. Cancer Discov. 2015;5(12):1282-1295.

14. Majzner RG, Mackall CL. Tumor Antigen Escape from CAR T-cell Therapy. Cancer Discov. 2018;8(10):1219-26.

15. Ghilardi G, Lee YG, Porazzi P, et al. Overcoming CD19-Negative Relapses in Patients with B-Cell Lymphomas Treated with Tisagenlecleucel. Blood. 2022;140(Supplement 1):7371-7373.

16. Dourthe M-E, Rabian F, Yakouben K, et al. Determinants of CD19-positive vs CD19-negative relapse after tisagenlecleucel for B-cell acute lymphoblastic leukemia. Leukemia. 2021;35(12):3383-3393.

17. Gardner R, Wu D, Cherian S, et al. Acquisition of a CD19-negative myeloid phenotype allows immune escape of MLL-rearranged B-ALL from CD19 CAR-T-cell therapy. Blood 2016;127(20):2406-2410.

18. Shah NN, Johnson BD, Schneider D, et al. Bispecific anti-CD20, anti-CD19 CAR T cells for relapsed B cell malignancies: a phase 1 dose escalation and expansion trial. Nat Med. 2020;26(10):1569-1575.

19. Zah E, Lin MY, Anne SB, Jensen MC, Chen YY. T cells expressing CD19/CD20 bispecific chimeric antigen receptors prevent antigen escape by malignant B cells. Cancer Immunol Res. 2016;4(6):498-508.

20. Seitz CM, Mittelstaet J, Atar D, et al. Novel adapter CAR-T cell technology for precisely controllable multiplex cancer targeting. Oncoimmunology. 2021;10(1):e2003532-2-16.

21. Rodriguez-Garcia A, Palazon A, Noguera-Ortega E, Powell DJ, Guedan S. CAR-T Cells Hit the Tumor Microenvironment: Strategies to Overcome Tumor Escape. Front Immunol. 2020;11(1109):1-17.

22. Brassart-Pasco S, Brézillon S, Brassart B, et al. Tumor Microenvironment: Extracellular Matrix Alterations Influence Tumor Progression. Front Oncol. 2020;10(397):1-13.

23. Xu S, Sankar S, Neamati N. Protein disulfide isomerase: a promising target for cancer therapy. Drug Discov Today. 2014;19(3):222-240.

24. Arnér ESJ, Holmgren A. The thioredoxin system in cancer. Semin Cancer Biol. 2006;16(6):420-426.

25. Goerdeler F, Reuber EE, Lühle J, et al. Thiol-Mediated Uptake of a Cysteine-Containing Nanobody for Anticancer Drug Delivery. ACS Cent Sci. 2023;9(6):1111-1118.

26. Popielarski M, Ponamarczuk H, Stasiak M, Wataka C, Świątkowska M. Modifications of disulfide bonds in breast cancer cell migration and invasiveness. *Am J Cancer Res.* 2019;9(8):1554-1582.

27. Ceccarelli J, Delfino L, Zappia E, et al. The redox state of the lung cancer microenvironment depends on the levels of thioredoxin expressed by tumor cells and affects tumor progression and response to prooxidants. Int J Cancer. 2008;123(8):1770-1778.

28. Tufo G, Jones AWE, Wang Z, et al. The protein disulfide isomerases PDIA4 and PDIA6 mediate resistance to cisplatin-induced cell death in lung adenocarcinoma. Cell Death Differ. 2014;21(5):685-695.

29. Kuo TF, Chen TY, Jiang ST, et al. Protein disulfide isomerase a4 acts as a novel regulator of cancer growth through the procaspase pathway. Oncogene. 2017;36(39): 5484-5496.

30. Robinson RM, Reyes L, Duncan RM, et al. Inhibitors of the protein disulfide isomerase family for the treatment of multiple myeloma. Leukemia. 2019;33(4):1011-1022.

31. Slezak AJ, Mansurov A, Raczy MM, et al. Tumor Cell-Surface Binding of Immune Stimulating Polymeric Glyco-Adjuvant via Cysteine-Reactive Pyridyl Disulfide Promotes Antitumor Immunity. ACS Cent Sci. 2022;8(10):1435-1446.

32. Heckman KL, Pease LR. Gene splicing and mutagenesis by PCR-driven overlap extension. Nat Protoc. 2007;2(4):924-932.

33. Fridy PC, Li Y, Keegan S, et al. A robust pipeline for rapid production of versatile nanobody repertoires. Nat Methods. 2014;11(12):1253-1260.

34. Jiang W, Hua R, Wei M, et al. An optimized method for high-titer lentivirus preparations without ultracentrifugation. Sci Rep. 2015;5:13875.

35. Alejo-Valle O, Weigert K, Bhayadia R, et al. The megakaryocytic transcription factor ARID3A suppresses leukemia pathogenesis. Blood 2022;139(5):651-665.

36. Xue Q, Bettini E, Paczkowski P, et al. Single-cell multiplexed cytokine profiling of CD19 CAR-T cells reveals a diverse landscape of polyfunctional antigen-specific response. J Immunother Cancer. 2017;5(1):85.

37. Wang H, Tsao ST, Gu M, et al. A simple and effective method to purify and activate T cells for successful generation of chimeric antigen receptor T (CAR-T) cells from patients with high monocyte count. J Transl Med. 2022;20(1):1-15.

38. Erber R, Kailayangiri S, Huebner H, Ruebner M, Hartmann A, Häberle L, Meyer J, Völkl S, Mackensen A, Landgraf L, Geppert CI, Schulz-Wendtland R, Beckmann MW, Fasching PA, Farwick N, Rossig C, Gass P. Variable Expression of the Disialoganglioside GD2 in Breast Cancer Molecular Subtypes. Cancers (Basel). 2021 Nov 8;13(21):5577.

39. Seitz CM, Schroeder S, Knopf P, Krahl AC, Hau J, Schleicher S, Martella M, Quintanilla-Martinez L, Kneilling M, Pichler B, Lang P, Atar D, Schilbach K, Handgretinger R, Schlegel P. GD2-targeted chimeric antigen receptor T cells prevent metastasis formation by elimination of breast cancer stem-like cells. Oncoimmunology. 2019 Nov 7;9(1):1683345.

40. CHIHIRO HIGASHI, KANAKO SAITO, YUJI KOZUKA, HIROTO YUASA, KAHO NAKAMURA, MAKOTO ISHITOBI, MIKIYA ISHIHARA, TOSHIRO MIZUNO, ISAO TAWARA, HIROSHI FUJIWARA, TOMOKO OGAWA. Ganglioside GD2 Expression Is Associated With Unfavorable Prognosis in Early Triple-negative Breast Cancer Anticancer Research Sep 2023, 43 (9) 4045-4053.

## Claims

1. A chimeric antigen receptor (CAR) comprising

- an antigen-binding domain comprising a heavy chain variable region (VH), and optionally a light chain variable region (VL);
- a hinge;
- a transmembrane domain;
- a co-stimulatory domain; and
- an activating domain;

wherein the CAR is capable of binding to an antigen,

**characterised in that** the VH, and optionally the VL, comprise three complementarity-determining regions (CDRs), wherein at least one CDR comprises at least one amino acid residue other than cysteine, which has a solvent-accessible surface area from 0.00 to 0.75 and is located at a distance to the antigen from 3 to 14 Å, and wherein said at least one amino acid residue is substituted to a thiol group containing amino acid.

2. The CAR of claim 1, wherein the solvent-accessible surface area is from 0.00 to 0.70, more preferably area is from 0.00 to 0.65, even more preferably is from 0.00 to 0.60, even more preferably is from 0.00 to 0.55, and most preferably is from 0.00 to 0.50; and/or
wherein the residue for cysteine substitution is located at the distance to the antigen of 3-13 Å, more preferably of 3.5-12 Å, even more preferably of 4-11 Å, even more preferably of 4-10 Å, even more preferably of 4-9 Å, and most preferably of 4-8 Å.

3. The CAR of any one of the preceding claims, wherein the thiol group containing amino acid selected from cysteine, cysteamine, 3-mercapto propionic acid, cysteine sulfinic acid, S-sulfocysteine, N-acetylcysteine (NAC), N, N'-Diacetyl-L-cystine, N, N'-Diacetyl-L-cystine Dimethylester (DACDM), S-Propylcysteine, S-Allylcysteine, S-propargyl-cysteine (SPRC) and S-Phenylcysteine, preferably wherein the thiol group containing amino acid is cysteine.

4. The CAR of any one of the preceding claims, wherein the antigen-binding domain of the CAR binds the antigen with at least 1 mM $K_d$, preferably wherein the $K_d$ is determinable by surface plasmon resonance (SPR), enzyme-linked immunosorbent assay (ELISA), Isothermal Titration Calorimetry (ITC), MicroScale Thermophoresis (MST), preferably SPR.

5. The CAR of any one of the preceding claims, wherein the antigen binding domain is a Fab fragment, a single chain variable fragment (scFV), single domain antibody fragment, antigen binding domain fragment, a diabody or a combination thereof.

6. The CAR of any one of the preceding claims, wherein the antigen-binding domain binds any one antigen selected from CD19, GD2, CD20, CD22, CD33, CD123, CD38, BMCA, HER2, PMSA, mesothelin, NY-ESO-1 and EGFRvIII.

7. The CAR of any one of the preceding claims, wherein the CAR binds to a target cell in an antigen-dependent manner, preferably wherein the CAR additionally binds to the target in an antigen-independent manner, more preferably wherein the antigen-independent binding comprises formation of at least one bond between at least one thiol group containing amino acid residue within at least one CDR of the CAR and at least one cysteine residue present in at least one surface protein of a cell.

8. A polynucleotide encoding the CAR of any one of the preceding claims, preferably wherein the polynucleotide is a DNA or RNA, more preferably an mRNA.

9. A viral vector containing the polynucleotide encoding the CAR according to claim 8, preferably wherein the viral vector is a lentiviral vector.

10. An engineered immune cell expressing at the surface membrane a CAR according to any one of the claims 1 to 7, preferably wherein the engineered immune cell is derived from a T-lymphocyte, a NK cell, a NKT cell, or a macrophage.

11. The engineered immune cell according to claim 10 for use in a method of treating a cancer, preferably wherein the cancer is a haematological cancer or a solid tumour, preferably wherein the solid tumor is breast cancer, colon cancer, lung cancer, skin cancer, brain cancer, head and neck cancer, soft tissue cancer, gastrointestinal cancer, genitourinary and gynecologic cancer, musculoskeletal cancer or endocrine cancer; or preferably wherein the haematological cancer is a lymphoma or a leukemia, more preferably wherein the lymphoma is Non-Hodgkin Lymphoma, Hodgkin lymphoma, Burkitt Lymphoma, multiple myeloma or follicular lymphoma.

12. The engineered immune cell for use in the method of treatment according to claim 11, wherein the cancer comprises a subset of cancer cells that is **characterised in** a reduced expression of the CAR antigen or in a sub-population that does not express the antigen, in particular wherein the cancer cells have undergone an antigen escape.

13. The engineered immune cell according to any one of claims for use in the method of treatment according to claim 11 or 12, wherein:
the cancer cell targeted in said method of treatment has at least a 2-fold increase in the expression level of at least one enzyme that catalyzes the formation and/or breakage of disulfide bonds between cysteine residues, compared to the expression level of said enzyme in a non-cancerous cell of the same cell type.

14. A method of engineering an immune cell comprising the steps of:

a) providing the immune cell; preferably wherein the immune cell is a T-lymphocyte;
b) introducing to said cell at least one polynucleotide as defined in claim 8;
c) expressing said polynucleotide in said cell thus expressing a CAR according to any of the claims 1-7 at the surface membrane of said immune cell.

15. The method of claim 14, wherein the method additionally comprises the step of:
d) testing the cytotoxicity of the generated immune cell expressing the CAR on its surface in parallel using:

i) a cell line expressing the antigen, and
ii) a cell line that does not express the antigen,
wherein the cytotoxicity is measurable using luciferase-based cytotoxicity assays, and wherein the cytotoxicity is greater than 50% at an effector-to-target (E:T) ratio of 9:1 in both the antigen-expressing cell line and the antigen KO cell line,
preferably wherein ii) the cell line that that does not express the antigen is either a cell line wherein the antigen is knocked out (KO) or wherein the antigen which inherently does not express the antigen.

## Fig. 1A

CB2-CAR-T Cell       B Cell Lymphoma

## Fig. 1B

## Fig. 1C

**Fig. 1D**

**Fig. 2A**

**Fig. 2B**

**Fig. 2C**

**Fig. 2D**

**Fig. 3A**

# Fig. 3B

# Fig. 3C

## Fig. 4A

## Fig. 4B

## Fig. 4C

**Fig. 5A**

**Fig. 5B**

**Fig. 5C**

## Fig. 5D

## Fig. 6A

Solvent-accessible surface area (SASA)

# Fig. 6B

# Fig. 7A

tumor cell injection on day -6

**Fig. 7B**

**Fig. 7C**

# Fig. 8 A-D

**A**

| 5' LTR | EF-1α | SP | CB2 | IgG1-Fc | CD28-TM | CD28 | CD3ζ | WPRE | CMV | GFP | 3' LTR |

**B**

| 5' LTR | CMV | GFP | 3' LTR |

**C**

| 5' LTR | EF-1α | SP | CB2 | IgG1-Fc | CD28-TM | CD28 | CD3ζ | WPRE | 3' LTR |

| 5' LTR | EF-1α | SP | C105S CB2 | IgG1-Fc | CD28-TM | CD28 | CD3ζ | WPRE | 3' LTR |

| 5' LTR | EF-1α | SP | LaG-16 | IgG1-Fc | CD28-TM | CD28 | CD3ζ | WPRE | 3' LTR |

| 5' LTR | EF-1α | SP | S108C LaG-16 | IgG1-Fc | CD28-TM | CD28 | CD3ζ | WPRE | 3' LTR |

**D**

| 5' LTR | EF-1α | SP | CB2 | CD8-h | CD8-TM | 4-1BB | CD3ζ | WPRE | 3' LTR |

| 5' LTR | EF-1α | SP | FMC63 | CD8-h | CD8-TM | 4-1BB | CD3ζ | WPRE | 3' LTR |

# Fig. 9

## Fig. 10 A

## Fig. 10 B

Non-transduced (9:1)    GFP (9:1)    CB2-CAR (9:1)

## Fig. 11

# Fig. 12

# Fig. 13 A-B

Europäisches
Patentamt

European
Patent Office

Office européen
des brevets

# EUROPEAN SEARCH REPORT

**Application Number**

EP 24 19 9448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG YUEDI ET AL: "Chimeric antigen receptor clustering via cysteines enhances T-cell efficacy against tumor", CANCER IMMUNOLOGY IMMUNOTHERAPY, vol. 71, no. 11, 1 November 2022 (2022-11-01), pages 2801-2814, XP093244660, Berlin/Heidelberg ISSN: 0340-7004, DOI: 10.1007/s00262-022-03195-4 Retrieved from the Internet: URL:https://link.springer.com/content/pdf/ 10.1007/s00262-022-03195-4.pdf> * page 2805 - page 2807; figures 1A, 2J * | 1,3-10, 14,15 | INV. A61K40/11 A61K40/31 A61K40/42 C07K16/28 C07K16/30 |
| Y | WO 2011/156328 A1 (GENENTECH INC [US]; BHAKTA SUNIL [US]; JUNUTULA JAGATH R [US]) 15 December 2011 (2011-12-15) * paragraph [0146] - paragraph [0153]; figure 1 * | 1-15 | |
| Y | WO 2015/157595 A1 (MEDIMMUNE LLC [US]) 15 October 2015 (2015-10-15) * paragraphs [0005], [0248]; table 6 * | 1-15 | **TECHNICAL FIELDS SEARCHED (IPC)** A61K C07K |
| Y | WO 2009/052249 A1 (GENENTECH INC [US]; MAO WEIGUANG [US] ET AL.) 23 April 2009 (2009-04-23) * page 45 - page 48 * | 1-15 | |

-/--

The present search report has been drawn up for all claims

| Place of search | Date of completion of the search | Examiner |
|---|---|---|
| The Hague | 6 March 2025 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 19 9448

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | GOERDELER FELIX ET AL: "Thiol-Mediated Uptake of a Cysteine-Containing Nanobody for Anticancer Drug Delivery", ACS CENTRAL SCIENCE, vol. 9, no. 6, 28 June 2023 (2023-06-28), pages 1111-1118, XP093244483, ISSN: 2374-7943, DOI: 10.1021/acscentsci.3c00177 Retrieved from the Internet: URL:https://pubs.acs.org/doi/pdf/10.1021/acscentsci.3c00177> * page 1117; figure S2A * ----- | 1-15 | |
| Y | WO 2014/124316 A2 (IRM LLC [US]; GEIERSTANGER BERNHARD HUBERT [US] ET AL.) 14 August 2014 (2014-08-14) * pages 114, 121; figure 1; examples 1-3; tables 6, 20 * ----- | 1-15 | |

**TECHNICAL FIELDS SEARCHED (IPC)**

The present search report has been drawn up for all claims

| Place of search | Date of completion of the search | Examiner |
|---|---|---|
| The Hague | 6 March 2025 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                      

& : member of the same patent family, corresponding document

## ANNEX TO THE EUROPEAN SEARCH REPORT
## ON EUROPEAN PATENT APPLICATION NO.

EP 24 19 9448

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2025

| Patent document<br>cited in search report | | Publication<br>date | Patent family<br>member(s) | | Publication<br>date |
|---|---|---|---|---|---|
| WO 2011156328 | A1 | 15-12-2011 | AU | 2011265054 A1 | 15-11-2012 |
| | | | AU | 2016256788 A1 | 01-12-2016 |
| | | | BR | 112012030311 A2 | 24-01-2017 |
| | | | CA | 2799540 A1 | 15-12-2011 |
| | | | CA | 3220104 A1 | 15-12-2011 |
| | | | CN | 103068406 A | 24-04-2013 |
| | | | CN | 107335062 A | 10-11-2017 |
| | | | CN | 114246952 A | 29-03-2022 |
| | | | CN | 119552249 A | 04-03-2025 |
| | | | EP | 2579897 A1 | 17-04-2013 |
| | | | JP | 2013534520 A | 05-09-2013 |
| | | | JP | 2018027932 A | 22-02-2018 |
| | | | KR | 20130087382 A | 06-08-2013 |
| | | | MX | 336540 B | 22-01-2016 |
| | | | RU | 2012156248 A | 20-07-2014 |
| | | | RU | 2017124859 A | 31-01-2019 |
| | | | SG | 185428 A1 | 28-12-2012 |
| | | | SG | 10201600791T A | 30-03-2016 |
| | | | US | 2011301334 A1 | 08-12-2011 |
| | | | US | 2014288280 A1 | 25-09-2014 |
| | | | US | 2017260253 A1 | 14-09-2017 |
| | | | US | 2020339664 A1 | 29-10-2020 |
| | | | US | 2024218050 A1 | 04-07-2024 |
| | | | WO | 2011156328 A1 | 15-12-2011 |
| WO 2015157595 | A1 | 15-10-2015 | AU | 2015243380 A1 | 10-11-2016 |
| | | | BR | 112016023436 A2 | 17-10-2017 |
| | | | CA | 2944784 A1 | 15-10-2015 |
| | | | CN | 106459205 A | 22-02-2017 |
| | | | CY | 1122155 T1 | 25-11-2020 |
| | | | DK | 3129406 T3 | 01-04-2019 |
| | | | EP | 3129406 A1 | 15-02-2017 |
| | | | ES | 2719584 T3 | 11-07-2019 |
| | | | HR | P20190486 T1 | 31-05-2019 |
| | | | HU | E041976 T2 | 28-06-2019 |
| | | | JP | 6685924 B2 | 22-04-2020 |
| | | | JP | 2017512486 A | 25-05-2017 |
| | | | LT | 3129406 T | 10-04-2019 |
| | | | PL | 3129406 T3 | 31-07-2019 |
| | | | PT | 3129406 T | 24-04-2019 |
| | | | SI | 3129406 T1 | 30-04-2019 |
| | | | SM | T201900160 T1 | 10-05-2019 |
| | | | TR | 201903526 T4 | 21-03-2019 |
| | | | US | 2018169255 A1 | 21-06-2018 |
| | | | US | 2021069341 A1 | 11-03-2021 |
| | | | WO | 2015157595 A1 | 15-10-2015 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 24 19 9448

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2025

| Patent document cited in search report | Publication date | Patent family member(s) | Publication date |
|---|---|---|---|
| WO 2009052249 A1 | 23-04-2009 | AR 068941 A1 | 16-12-2009 |
| | | AU 2008312457 A1 | 23-04-2009 |
| | | AU 2014203779 A1 | 31-07-2014 |
| | | BR PI0818780 A2 | 22-04-2015 |
| | | CA 2698541 A1 | 23-04-2009 |
| | | CL 2008003074 A1 | 28-12-2009 |
| | | CN 101835803 A | 15-09-2010 |
| | | CO 6390071 A2 | 29-02-2012 |
| | | EP 2209808 A1 | 28-07-2010 |
| | | ES 2450755 T3 | 25-03-2014 |
| | | HK 1143379 A1 | 31-12-2010 |
| | | IL 204159 A | 29-02-2016 |
| | | JP 5606916 B2 | 15-10-2014 |
| | | JP 2011504460 A | 10-02-2011 |
| | | JP 2013173776 A | 05-09-2013 |
| | | KR 20100090267 A | 13-08-2010 |
| | | MY 188455 A | 09-12-2021 |
| | | NZ 584514 A | 27-07-2012 |
| | | PE 20091112 A1 | 25-07-2009 |
| | | PE 20140833 A1 | 14-07-2014 |
| | | RU 2010119937 A | 27-11-2011 |
| | | TW 200927172 A | 01-07-2009 |
| | | US 2009117100 A1 | 07-05-2009 |
| | | US 2015158952 A1 | 11-06-2015 |
| | | US 2017166635 A1 | 15-06-2017 |
| | | WO 2009052249 A1 | 23-04-2009 |
| | | ZA 201001383 B | 28-04-2011 |
| WO 2014124316 A2 | 14-08-2014 | AU 2014214751 A1 | 20-08-2015 |
| | | AU 2017219059 A1 | 14-09-2017 |
| | | BR 112015018899 A2 | 06-08-2015 |
| | | CA 2900755 A1 | 14-08-2014 |
| | | CN 105143271 A | 09-12-2015 |
| | | CN 115925957 A | 07-04-2023 |
| | | DK 2953976 T3 | 21-06-2021 |
| | | EA 201591465 A1 | 30-12-2015 |
| | | EP 2953976 A2 | 16-12-2015 |
| | | EP 3929217 A2 | 29-12-2021 |
| | | ES 2874493 T3 | 05-11-2021 |
| | | HK 1213580 A1 | 08-07-2016 |
| | | HU E054443 T2 | 28-09-2021 |
| | | JP 6609477 B2 | 20-11-2019 |
| | | JP 2016511637 A | 21-04-2016 |
| | | KR 20150115000 A | 13-10-2015 |
| | | KR 20210125593 A | 18-10-2021 |

For more details about this annex : see Official Journal of the European Patent Office, No. 12/82

page 2 of 3

## ANNEX TO THE EUROPEAN SEARCH REPORT
## ON EUROPEAN PATENT APPLICATION NO.

EP 24 19 9448

This annex lists the patent family members relating to the patent documents cited in  the above-mentioned European search report.
The members are as contained in the European Patent Office EDP file on
The European Patent Office is in no way liable for these particulars which are merely given for the purpose of information.

06-03-2025

| Patent document cited in search report | Publication date | Patent family member(s) | | Publication date |
|---|---|---|---|---|
| | | KR | 20220133313 A | 04-10-2022 |
| | | PL | 2953976 T3 | 02-11-2021 |
| | | PT | 2953976 T | 23-06-2021 |
| | | SG | 10201706468R A | 28-09-2017 |
| | | SG | 11201506025R A | 28-08-2015 |
| | | SI | 2953976 T1 | 31-08-2021 |
| | | US | 2016067351 A1 | 10-03-2016 |
| | | US | 2020338207 A1 | 29-10-2020 |
| | | US | 2023270876 A1 | 31-08-2023 |
| | | WO | 2014124316 A2 | 14-08-2014 |

For more details about this annex : see Official Journal of the European Patent Office, No. 12/82

page 3 of 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WU L** ; **WEI Q** ; **BRZOSTEK J** ; **GASCOIGNE NRJ**. Signaling from T cell receptors (TCRs) and chimeric antigen receptors (CARs) on T cells. *Cell Mol Immunol.*, 2020, vol. 17 (6), 600-612
- **O'LEARY MC** ; **LU X** ; **HUANG Y et al.** FDA Approval summary: Tisagenlecleucel for treatment of patients with relapsed or refractory b-cell precursor acute lymphoblastic leukemia. *Clinical Cancer Research*, 2019, vol. 25 (4), 1142-1146
- **BOUCHKOUJ N** ; **KASAMON YL** ; **CLARO RA et al.** FDA approval summary: Axicabtagene Ciloleucel for Relapsed or Refractory Large B-cell Lymphoma. *Clinical Cancer Research*, 2019, vol. 25 (6), 1702-1708
- **ST-PIERRE F** ; **GORDON LI**. Lisocabtagene maraleucel in the treatment of relapsed/refractory large B-cell lymphoma. *Future Oncology*, 2023, vol. 19 (1), 19-28
- **ANDERSON LD**. Idecabtagene vicleucel (ide-cel) CAR T-cell therapy for relapsed and refractory multiple myeloma. *Future Oncology*, 2022, vol. 18 (3), 277-289
- **CHEKOL ABEBE E** ; **YIBELTAL SHIFERAW M** ; **TADELE ADMASU F** ; **ASMAMAW DEJENIE T**. Ciltacabtagene autoleucel: The second anti-BCMA CAR T-cell therapeutic armamentarium of relapsed or refractory multiple myeloma. *Front Immunol.*, 2022, vol. 13, 991092
- **FREY N V.** Approval of brexucabtagene autoleucel for adults with relapsed and refractory acute lymphocytic leukemia. *Blood.*, 2022, vol. 140 (1), 11-15
- **SADELAIN M**. CAR therapy: The CD19 paradigm. *Journal of Clinical Investigation*, 2015, vol. 125 (9), 3392-3400
- **ABREU TR** ; **FONSECA NA** ; **GONÇALVES N** ; **MOREIRA JN**. Current challenges and emerging opportunities of CAR-T cell therapies. *Journal of Controlled Release*, 2020, vol. 319, 246-261
- **FREY N** ; **PORTER D**. Cytokine Release Syndrome with Chimeric Antigen Receptor T Cell Therapy. *Biology of Blood and Marrow Transplantation*, 2019, vol. 25 (4), e123-e127
- **GUST J** ; **HAY KA** ; **HANAFI LA et al.** Endothelial activation and blood-brain barrier disruption in neurotoxicity after adoptive immunotherapy with CD19 CAR-T cells. *Cancer Discov.*, 2017, vol. 7 (12), 1404-1419

- **ORLANDO EJ** ; **HAN X** ; **TRIBOULEY C et al.** Genetic mechanisms of target antigen loss in CAR19 therapy of acute lymphoblastic leukemia. *Nat Med.*, 2018, vol. 24 (10), 1504-1506
- **SOTILLO E** ; **BARRETT DM** ; **BLACK KL et al.** Convergence of acquired mutations and alternative splicing of CD19 enables resistance to CART-19 immunotherapy. *Cancer Discov.*, 2015, vol. 5 (12), 1282-1295
- **MAJZNER RG** ; **MACKALL CL**. Tumor Antigen Escape from CAR T-cell Therapy. *Cancer Discov.*, 2018, vol. 8 (10), 1219-26
- **GHILARDI G** ; **LEE YG** ; **PORAZZI P et al.** Overcoming CD19-Negative Relapses in Patients with B-Cell Lymphomas Treated with Tisagenlecleucel. *Blood.*, 2022, vol. 140 (1), 7371-7373
- **DOURTHE M-E** ; **RABIAN F** ; **YAKOUBEN K et al.** Determinants of CD19-positive vs CD19-negative relapse after tisagenlecleucel for B-cell acute lymphoblastic leukemia. *Leukemia*, 2021, vol. 35 (12), 3383-3393
- **GARDNER R** ; **WU D** ; **CHERIAN S et al.** Acquisition of a CD19-negative myeloid phenotype allows immune escape of MLL-rearranged B-ALL from CD19 CAR-T-cell therapy. *Blood*, 2016, vol. 127 (20), 2406-2410
- **SHAH NN** ; **JOHNSON BD** ; **SCHNEIDER D et al.** Bispecific anti-CD20, anti-CD19 CAR T cells for relapsed B cell malignancies: a phase 1 dose escalation and expansion trial. *Nat Med.*, 2020, vol. 26 (10), 1569-1575
- **ZAH E** ; **LIN MY** ; **ANNE SB** ; **JENSEN MC** ; **CHEN YY**. T cells expressing CD19/CD20 bispecific chimeric antigen receptors prevent antigen escape by malignant B cells. *Cancer Immunol Res.*, 2016, vol. 4 (6), 498-508
- **SEITZ CM** ; **MITTELSTAET J** ; **ATAR D et al.** Novel adapter CAR-T cell technology for precisely controllable multiplex cancer targeting. *Oncoimmunology*, 2021, vol. 10 (1), e2003532-2, 16
- **RODRIGUEZ-GARCIA A** ; **PALAZON A** ; **NOGUERA-ORTEGA E** ; **POWELL DJ** ; **GUEDAN S**. CAR-T Cells Hit the Tumor Microenvironment: Strategies to Overcome Tumor Escape. *Front Immunol.*, 2020, vol. 11 (1109), 1-17
- **BRASSART-PASCO S** ; **BRÉZILLON S** ; **BRASSART B et al.** Tumor Microenvironment: Extracellular Matrix Alterations Influence Tumor Progression. *Front Oncol.*, 2020, vol. 10 (397), 1-13

- **XU S** ; **SANKAR S** ; **NEAMATI N**. Protein disulfide isomerase: a promising target for cancer therapy. *Drug Discov Today.*, 2014, vol. 19 (3), 222-240
- **ARNÉR ESJ** ; **HOLMGREN A**. The thioredoxin system in cancer. *Semin Cancer Biol.*, 2006, vol. 16 (6), 420-426
- **GOERDELER F** ; **REUBER EE** ; **LÜHLE J et al.** Thiol-Mediated Uptake of a Cysteine-Containing Nanobody for Anticancer Drug Delivery. *ACS Cent Sci.*, 2023, vol. 9 (6), 1111-1118
- **CECCARELLI J** ; **DELFINO L** ; **ZAPPIA E et al.** The redox state of the lung cancer microenvironment depends on the levels of thioredoxin expressed by tumor cells and affects tumor progression and response to prooxidants. *Int J Cancer.*, 2008, vol. 123 (8), 1770-1778
- **TUFO G** ; **JONES AWE** ; **WANG Z et al.** The protein disulfide isomerases PDIA4 and PDIA6 mediate resistance to cisplatin-induced cell death in lung adenocarcinoma. *Cell Death Differ.*, 2014, vol. 21 (5), 685-695
- **KUO TF** ; **CHEN TY** ; **JIANG ST et al.** Protein disulfide isomerase a4 acts as a novel regulator of cancer growth through the procaspase pathway. *Oncogene*, 2017, vol. 36 (39), 5484-5496
- **ROBINSON RM** ; **REYES L** ; **DUNCAN RM et al.** Inhibitors of the protein disulfide isomerase family for the treatment of multiple myeloma. *Leukemia.*, 2019, vol. 33 (4), 1011-1022
- **SLEZAK AJ** ; **MANSUROV A** ; **RACZY MM et al.** Tumor Cell-Surface Binding of Immune Stimulating Polymeric Glyco-Adjuvant via Cysteine-Reactive Pyridyl Disulfide Promotes Antitumor Immunity. *ACS Cent Sci.*, 2022, vol. 8 (10), 1435-1446
- **HECKMAN KL** ; **PEASE LR**. Gene splicing and mutagenesis by PCR-driven overlap extension. *Nat Protoc.*, 2007, vol. 2 (4), 924-932
- **FRIDY PC** ; **LI Y** ; **KEEGAN S et al.** A robust pipeline for rapid production of versatile nanobody repertoires. *Nat Methods.*, 2014, vol. 11 (12), 1253-1260
- **JIANG W** ; **HUA R** ; **WEI M et al.** An optimized method for high-titer lentivirus preparations without ultracentrifugation. *Sci Rep.*, 2015, vol. 5, 13875
- **ALEJO-VALLE O** ; **WEIGERT K** ; **BHAYADIA R et al.** The megakaryocytic transcription factor ARID3A suppresses leukemia pathogenesis. *Blood*, 2022, vol. 139 (5), 651-665
- **XUE Q** ; **BETTINI E** ; **PACZKOWSKI P et al.** Single-cell multiplexed cytokine profiling of CD19 CAR-T cells reveals a diverse landscape of polyfunctional antigen-specific response. *J Immunother Cancer.*, 2017, vol. 5 (1), 85
- **WANG H** ; **TSAO ST** ; **GU M et al.** A simple and effective method to purify and activate T cells for successful generation of chimeric antigen receptor T (CAR-T) cells from patients with high monocyte count. *J Transl Med.*, 2022, vol. 20 (1), 1-15
- **ERBER R** ; **KAILAYANGIRI S** ; **HUEBNER H** ; **RUEBNER M** ; **HARTMANN A** ; **HÄBERLE L** ; **MEYER J** ; **VÖLKL S** ; **MACKENSEN A** ; **LANDGRAF L**. Variable Expression of the Disialoganglioside GD2 in Breast Cancer Molecular Subtypes. *Cancers (Basel).*, 08 November 2021, vol. 13 (21), 5577
- **SEITZ CM** ; **SCHROEDER S** ; **KNOPF P** ; **KRAHL AC** ; **HAU J** ; **SCHLEICHER S** ; **MARTELLA M** ; **QUINTANILLA-MARTINEZ L** ; **KNEILLING M** ; **PICHLER B**. GD2-targeted chimeric antigen receptor T cells prevent metastasis formation by elimination of breast cancer stem-like cells. *Oncoimmunology*, 07 November 2019, vol. 9 (1), 1683345
- **CHIHIRO HIGASHI** ; **KANAKO SAITO** ; **YUJI KOZUKA** ; **HIROTO YUASA** ; **KAHO NAKAMURA** ; **MAKOTO ISHITOBI** ; **MIKIYA ISHIHARA** ; **TOSHIRO MIZUNO** ; **ISAO TAWARA** ; **HIROSHI FUJIWARA**. *Ganglioside GD2 Expression Is Associated With Unfavorable Prognosis in Early Triple-negative Breast Cancer Anticancer Research*, September 2023, vol. 43 (9), 4045-4053